# EUROPEAN PATENT APPLICATION

(11) **EP 2 650 284 A1**
(43) Date of publication of application: **16.10.2013**
(21) Application number: 12163604.7
(22) Date of filing: 10.04.2012
(51) Int. Cl.: C07D 215/24, C07D 471/04, C07D 491/04, C07D 497/04, C07D 498/04, A61K 31/436, A61K 31/4365, A61K 31/4375, A61K 31/47, A61P 25/00, A61P 35/00, A61P 31/00

(54) **Heterocyclic derivatives as metabotropic glutamate receptor modulators**

(71) Applicant: Merz Pharma GmbH & Co. KGaA, 60318 Frankfurt (DE)
(72) Inventor: The designation of the inventor has not yet been filed
(74) Representative: Goddard, Christopher Robert

(57) **Abstract**

The invention relates to heterocyclic derivatives of formula I as well as their pharmaceutically acceptable salts. The invention further relates to a process for the preparation of such compounds. The compounds of the invention are mGluR5 modulators and are therefore useful for the control and prevention of acute and/or chronic neurological disorders.

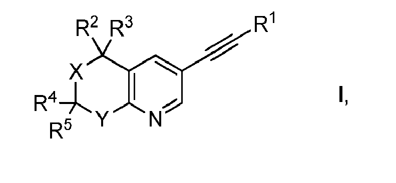

## Description

### FIELD OF THE INVENTION

The present invention relates to heterocyclic derivatives, which may act as novel metabotropic glutamate receptor (mGluR) modulators, methods for their synthesis and the treatment and/or prevention of various diseases and disorders, including neurological disorders, by administration of such derivatives.

### BACKGROUND OF THE INVENTION

Neuronal stimuli are transmitted by the central nervous system (CNS) through the interaction of a neurotransmitter released by a neuron, which neurotransmitter has a specific effect on a neuroreceptor of another neuron. L-Glutamic acid is considered to be a major excitatory neurotransmitter in the mammalian CNS, consequently playing a critical role in a large number of physiological processes. Glutamate-dependent stimulus receptors are divided into two main groups. The first group comprises ligand-controlled ion channels whereas the other comprises metabotropic glutamate receptors (mGluR). Metabotropic glutamate receptors are a subfamily of G-protein-coupled receptors (GPCR). There is increasing evidence for a peripheral role of both ionotropic and metabotropic glutamate receptors outside the CNS e.g, in chronic pain states.

At present, eight different members of these mGluRs are known. On the basis of structural parameters such as sequence homology, the second messenger system utilized by these receptors and their different affinity to low-molecular weight compounds, these eight receptors may be divided into three groups. MGluR1 and mGluR5 belong to Group I which are positively coupled to phospholipase C and their activation leads to a mobilization of intracellular calcium ions. MGluR2 and mGluR3 belong to Group II and mGluR4, mGluR6, mGluR7 and mGluR8 belong to Group III, both of which are negatively coupled to adenylyl cyclase, i.e., their activation causes a reduction in second messenger cAMP and thus a dampening of neuronal activity.

In contrast to class A GPCRs (rhodopsin like receptors) where orthosteric ligands bind to a site within the transmembrane helix bundle, for class C GPCRs (the class to which mGluR5 belongs) the orthosteric ligand (e.g. glutamate) binds to a distinct module at the N-terminus of the receptor, the venus fly trap module (Bockaert, et al., The EMBO Journal, 1999, 18, 1723-1729). Moreover, within the helix bundle of mGluR5 a binding site for allosteric ligands, exemplified by MPEP, exists.

In analogy to ligands to the orthosteric site of class A receptors, where full agonists, partial agonists, neutral antagonists, and inverse agonists can be described (Seifert, et al., Naunyn Schmiedebergs Arch Pharmacol., 2002, 366, 381-416), a spectrum of ligands to the allosteric site (i.e., negative allosteric modulators, positive allosteric modulators, and silent allosteric modulators) may be observed. The first example of a silent allosteric modulator for mGluR5 (5-MPEP) was reported by Rodriguez, et al. (Molecular Pharmacology, 2005, 68, 1793-1802).

The mGluR5 modulators have been shown to modulate the effects of the presynaptically released neurotransmitter glutamate via postsynaptic mechanisms (receptors). Moreover, as these modulators may be both positive and/or negative mGluR5 modulators, such modulators may increase or inhibit the effects mediated through these metabotropic glutamate receptors.

Modulators which are negative mGluR5 modulators decrease the effects mediated through metabotropic glutamate receptors. Since a variety of patho-physiological processes and disease states affecting the CNS are thought to be related to abnormal glutamate neurotransmission, and mGluR5 receptors are shown to be expressed in many areas of the CNS and in PNS (peripheral nervous system), modulators of these receptors could be therapeutically beneficial in the treatment of diseases involving CNS and PNS.

Therefore, mGluR5 positive or negative modulators may be administered to provide neuroprotection and/or disease modification in the following acute or chronic pathological conditions or to provide a symptomatological effect on the following conditions: Alzheimer's disease, Creutzfeld-Jakob's syndrome/disease, bovine spongiform encephalopathy (BSE), prion related infections, diseases involving mitochondrial dysfunction, diseases involving β-amyloid and/or tauopathy, Down's syndrome, hepatic encephalopathy, Huntington's disease, motor neuron diseases, amyotrophic lateral sclerosis (ALS), multiple system atrophy, olivoponto-cerebellar atrophy, synucleinopathies, alpha-synucleinopathies, Lewy body dementia, neurodegeneration with Brain Iron Accumulation, Parkinson-plus syndrome, Pick's disease, progressive supranuclear palsy (PSP), frontotemporal dementia and parkinsonism linked to chromosome 17 (FTDP-17), neurodegenerative diseases, post-operative cognitive deficit (POCD), systemic lupus erythematosus, systemic sclerosis, Sjogren's syndrome, neuronal ceroid lipofuscinosis, neurodegenerative cerebellar ataxias, Parkinson's disease, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, eye injuries, eye diseases, eye disorders, glaucoma, retinopathy, macular degeneration, head or brain or spinal cord injuries, head or brain or spinal cord trauma, trauma, hypoglycaemia, hypoxia, perinatal hypoxia, ischaemia, ischaemia resulting from cardiac arrest or stroke or bypass operations or transplants, convulsions, epileptic convulsions, epilepsy, temporal lobe epilepsy, myoclonic epilepsy, inner ear insult, inner ear insult in tinnitus, tinnitus, sound- or drug-induced inner ear insult, sound- or drug-induced tinnitus, hyperacusis, L-DOPA-induced dyskinesias, L-DOPA-induced dyskinesias in Parkinson's disease therapy, dyskinesias, dyskinesia in Huntington's disease, drug induced dyskinesias, neuroleptic-induced dyskinesias, haloperidol-induced dyskinesias, dopaminomimetic-induced dyskinesias, chorea, Huntington's chorea, athetosis, dystonia, stereotypy, ballism, tardive dyskinesias, neuroleptics-induced dyskinesia, tic disorder, torticollis spasmodicus, blepharospasm, focal and generalized dystonia, nystagmus, hereditary cerebellar ataxias, corticobasal degeneration, tremor, essential tremor, abuse, addiction, nicotine addiction, nicotine abuse, alcohol addiction, alcohol abuse, opiate addiction, opiate abuse, cocaine addiction, cocaine abuse, amphetamine addiction, amphetamine abuse, anxiety disorders, panic disorders, anxiety and panic disorders, social anxiety disorder (SAD), attention deficit hyperactivity disorder (ADHD), attention deficit syndrome (ADS), restless leg syndrome (RLS), hyperactivity in children, autism, dementia, dementia in Alzheimer's disease, dementia in Korsakoff syndrome, Korsakoff syndrome, vascular dementia, dementia related to HIV infections, HIV-1 encephalopathy, AIDS encephalopathy, AIDS dementia complex, AIDS-related dementia, major depressive disorder, major depression, depression, depression resulting from Borna virus infection, major depression resulting from Borna virus infection, bipolar manic-depressive disorder, drug tolerance, drug tolerance to opioids, movement disorders, fragile-X syndrome, irritable bowel syndrome (IBS), migraine, multiple sclerosis (MS), muscle spasms, pain, chronic pain, acute pain, inflammatory pain, neuropathic pain, diabetic neuropathic pain (DNP), pain related to rheumatic arthritis, allodynia, hyperalgesia, nociceptive pain, cancer pain, posttraumatic stress disorder (PTSD), schizophrenia, positive or negative symptoms of schizophrenia, cognitive deficits of schizophrenia, spasticity, Tourette's syndrome, urinary incontinence, vomiting, pruritic conditions, pruritis, sleep disorders, micturition disorders, neuromuscular disorder in the lower urinary tract, gastroesophageal reflux disease (GERD), gastrointestinal dysfunction, lower esophageal sphincter (LES) disease, functional gastrointestinal disorders, dyspepsia, regurgitation, respiratory tract infection, bulimia nervosa, chronic laryngitis, asthma, reflux-related asthma, lung disease, eating disorders, obesity, obesity-related disorders, obesity abuse, food addiction, binge eating disorders, agoraphobia, generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, or delirium, diabetes, hyperammonemia and liver failure and sleep disturbances.

mGluR5 negative or positive modulators may also be administered to provide inhibition of tumour cell growth, migration, invasion, adhesion and toxicity in the peripheral tissues, peripheral nervous system and CNS. mGluR5 modulators may be administered to provide therapeutic intervention in neoplasia, hyperplasia, dysplasia, cancer, carcinoma, sarcoma, oral cancer, squamous cell carcinoma (SCC), oral squamous cell carcinoma (SCC), lung cancer, lung adenocarcinoma, breast cancer, prostate cancer, gastric cancer, liver cancer, colon cancer, colorectal carcinoma, rhabdomyosarcoma, brain tumour, tumour of a nerve tissue, glioma, malignant glioma, astroglioma, neuroglioma, neuroblastoma, glioblastoma, medulloblastoma, cancer of skin cells, melanoma, malignant melanoma, epithelial neoplasm, lymphoma, myeloma, Hodgkin's disease, Burkitt's lymphoma, leukemia, thymoma, and other tumours.

mGluR5 positive or negative modulators may also be administered to provide disease modification and/or to provide a symptomatological effect on the following conditions: diabetes, hyperammonemia and liver failure.

Further indications for mGluR5 negative or positive modulators include those indications wherein a particular condition does not necessarily exist but wherein a particular physiological parameter may be improved through administration of the instant compounds, for example cognitive enhancement, learning impairment and/or neuroprotection.

Positive modulators may be particularly useful in the treatment of positive and negative symptoms and cognitive deficits in schizophrenia as well as cognitive deficits in various forms of dementia and mild cognitive impairment.

Silent modulators may be useful for attenuation of disease states emerging from disregulation of a natural ligand for the allosteric site.

Moreover, mGluR modulators may have activity when administered in combination with other substances exhibiting neurological effects via different mechanisms.

Simultaneous administration of Group I mGluR modulators and NMDA receptor antagonists has also been shown to provide neuroprotection in animal models (Zieminska et al. Acta Neurobiol. Exp., 2006, 66, 301-309; Zieminska et al. Neurochemistry International, 2003, 43, 481-492; and Zieminska et al. Neurochemistry International, 2006, 48, 491-497).

Simultaneous administration of Group I mGluR modulators and compounds such as L-DOPA, dopaminomimetics, and/or neuroleptics may be useful in treating various conditions including drug induced dyskinesias, neuroleptic-induced dyskinesias, haloperidol-induced dyskinesias, dopaminomimetic-induced dyskinesias.

In the literature, several types of modulators of mGluR5 have already been described.

### THE PRESENT INVENTION

It now has been found that certain heterocyclic derivatives are potent mGluR5 modulators. Therefore, these substances may be therapeutically beneficial in the treatment of conditions which involve abnormal glutamate neurotransmission or in which modulation of mGluR5 receptors results in therapeutic benefit. These substances may be administered in the form of a pharmaceutical composition, wherein they are present together with one or more pharmaceutically acceptable diluents, carriers, or excipients.

### OBJECTS OF THE INVENTION

It is an object of the present invention to provide novel pharmaceutical compounds which are mGluR5 modulators and pharmaceutical compositions thereof. It is a further object of the invention to provide a novel method of treating, eliminating, alleviating, palliating, modifying, or ameliorating undesirable CNS disorders which involve abnormal glutamate neurotransmission, and/or to provide symptomological effects, by employing a compound of the invention or a pharmaceutical composition containing the same.

An additional object of the invention is the provision of processes for producing the heterocyclic derivatives.

### SUMMARY OF THE INVENTION

What we therefore believe to be comprised by our invention may be summarized inter alia in the following words:
A compound selected from those of Formula I 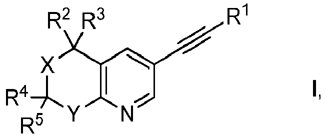 wherein
R¹ represents aryl, heteroaryl, cycloC₃₋₁₂alkyl, cycloC₅₋₁₂alkenyl, or heterocyclyl;
R² represents H, F, OH, C₁₋₆alkyl, C₁₋₆alkoxy, heterocyclyl, or NR⁶R⁷;
R³ represents H, F, or C₁₋₆alkyl;
or R² and R³ together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R⁴ represents H, F, or C₁₋₆alkyl;
or R² and R⁴ together with the carbon atoms to which they are attached form a 4-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R⁵ represents H, F, or C₁₋₆alkyl;
or R⁴ and R⁵ together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R⁶ represents H, C₁₋₆alkyl, or acyl;
R⁷ represents H or C₁₋₆alkyl;
X represents CR⁸R⁹, NR¹⁰, S, S=O, or SO₂;
Y represents a bond, CR¹¹R¹², -CR¹³R¹⁴-CH₂-, O, -CR¹⁵R¹⁶-O-, NR¹⁷, or -CR¹⁸R¹⁹-NR²⁰-;
R⁸ represents H, C₁₋₆alkyl, acyl, heterocyclyl, heterocyclylsulfinyl;
or R² and R⁸ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoroethyl, trifluoropropyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R² and R⁸ together with the carbon atoms to which they are attached form a 5-6 membered aromatic or heteroaromatic ring, wherein the ring may be optionally substituted by one or more substituents selected from halogen, fluoromethyl, difluoromethyl, trifluoromethyl, trifluoroethyl, trifluoropropyl, trifluoromethoxy, C₁₋₆alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl, in which case the R³ and R⁹ substituents are absent;
or R⁴ and R⁸ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R⁹ represents hydrogen or C₁₋₆alkyl;
or R⁸ and R⁹ together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹⁰ represents H, C₁₋₆alkyl, acyl, -C(O)-C(O)-C₁₋₆alkoxy, C₁₋₆alkylsulfinyl, C₁₋₆alkylsulfonyl, cycloC₃₋₇alkylsulfonyl, heterocyclylsulfonyl, arylsulfonyl, di-(C₁₋₆alkyl)aminosulfonyl, aryl, or heteroaryl;
or R² and R¹⁰ together with the atoms to which they are attached form a 3-7 membered ring which may be saturated or unsaturated, wherein the ring may optionally contain one or two additional heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R⁴ and R¹⁰ together with the atoms to which they are attached form a 3-7 membered ring which may be saturated or unsaturated, wherein the ring may optionally contain one or two additional heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹¹ represents H, C₁₋₆alkyl, acyl or heterocyclyl;
or R⁴ and R¹¹ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R² and R¹¹ together with the carbon atoms to which they are attached form a 5-7 membered ring which may be saturated or unsaturated, wherein the ring may optionally contain one or two heteroatoms selected from sulfur, oxygen, and
nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R⁸ and R¹¹ together with the carbon atoms to which they are attached form a 4-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R¹⁰ and R¹¹ together with the atoms to which they are attached form a 4-7 membered ring which may be saturated or unsaturated, wherein the ring may optionally contain one or two additional heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹² represents H or C₁₋₆alkyl;
or R¹¹ and R¹² together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹³ represents H, C₁₋₆alkyl, acyl or heterocyclyl;
or R⁴ and R¹³ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹⁴ represents H or C₁₋₆alkyl;
or R¹³ and R¹⁴ together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹⁵ represents H, C₁₋₆alkyl, acyl or heterocyclyl;
or R⁴ and R¹⁵ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹⁶ represents H or C₁₋₆alkyl;
or R¹⁵ and R¹⁶ together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹⁷ represents H, C₁₋₆alkyl, acyl, aryl, or heteroaryl;
or R² and R¹⁷ together with the atoms to which they are attached form a 5-7 membered ring which may be saturated or unsaturated, wherein the ring may optionally contain one or two additional heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R⁴ and R¹⁷ together with the atoms to which they are attached form a 3-7 membered ring which may be saturated or unsaturated, wherein the ring may optionally contain one or two additional heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R⁸ and R¹⁷ together with the atoms to which they are attached form a 4-7 membered ring which may be saturated or unsaturated, wherein the ring may optionally contain one or two additional heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹⁸ represents H, C₁₋₆alkyl, acyl or heterocyclyl;
or R⁴ and R¹⁸ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹⁹ represents H or C₁₋₆alkyl;
R²⁰ represents H, C₁₋₆alkyl, acyl, aryl, or heteroaryl;
and optical isomers, prodrugs, pharmaceutically acceptable salts, hydrates, solvates, and polymorphs thereof.

A further aspect of the invention relates to a compound of Formula I, wherein R¹ represents aryl or heteroaryl.

Such a compound of Formula **I**, wherein R² represents H, OH, C₁₋₆alkyl, or C₁₋₆alkoxy and R³ represents H or C₁₋₆alkyl or R² and R³ together with the carbon atom to which they are attached form a 5-membered ring containing one or two heteroatoms selected from sulfur, oxygen, and nitrogen, wherein the ring may be optionally substituted by one or more substituents selected from C₁₋₆alkyl, oxo, phenyl, and cycloC₃₋₇alkyl.

Such a compound of Formula **I**, wherein R² represents H, OH, methyl, or methoxy and R³ represents H or methyl or R² and R³ together with the carbon atom to which they are attached form an oxazolidine ring substituted by an oxo group and a methyl, propyl, phenyl or cyclopropyl group.

Such a compound of Formula **I,** wherein R⁴ and R⁵ each independently represent H or C₁₋₆alkyl.

Such a compound of Formula **I,** wherein R⁴ and R⁵ each independently represent H or methyl.

Such a compound of Formula **I,** wherein X represents CR⁸R⁹, NR¹⁰, or SO₂.

Such a compound of Formula **I,** wherein X represents NR¹⁰ and R¹⁰ represents C₁₋₆alkylcarbonyl, cycloC₃₋₁₂alkylcarbonyl, C₁₋₆alkoxycarbonyl, aryloxycarbonyl, di-(C₁₋₆alkyl)aminocarbonyl (wherein the alkyl moieties may be optionally substituted by one or more halogen atoms), -C(O)-C(O)-C₁₋₆alkoxy, C₁₋₆alkylsulfinyl, C₁₋₆alkylsulfonyl, cycloC₃₋₇alkylsulfonyl, arylsulfonyl, heterocyclylsulfonyl, or di-(C₁₋₆alkyl)aminosulfonyl.

Such a compound of Formula **I,** wherein X represents NR¹⁰ and R⁴ and R¹⁰ together with the atoms to which they are attached form a 6-membered ring optionally containing one or two additional heteroatoms selected from sulfur, oxygen, and nitrogen, wherein the ring may be optionally substituted by one or more substituents selected from oxo and C₁₋₆alkyl.

Such a compound of Formula **I,** wherein R⁴ and R¹⁰ together with the atoms to which they are attached form a tetrahydropyrimidine ring optionally substituted by one or more substituents selected from oxo and C₁₋₆alkyl.

Such a compound of Formula **I,** wherein X represents CR⁸R⁹ and R² and R⁸ together with the carbon atoms to which they are attached form a 5 or 6 membered ring optionally containing one or two heteroatoms selected from sulfur, oxygen, and nitrogen, wherein the ring may be optionally substituted by one or more substituents selected from C₁₋₆alkyl, trifluoroethyl, trifluoropropyl, C₁₋₆alkoxycarbonyl, and di-(C₁₋₆alkyl)aminocarbonyl or R² and R⁸ together with the carbon atoms to which they are attached form a 5-membered heteroaromatic ring optionally substituted by one or more C₁₋₆alkyl groups.

Such a compound of Formula **I,** wherein X represents CR⁸R⁹ and R² and R⁸ together with the carbon atoms to which they are attached form an oxazine ring optionally substituted by one or more substituents selected from C₁₋₆alkyl, trifluoroethyl, trifluoropropyl, C₁₋₆alkoxycarbonyl, and di-(C₁₋₆alkyl)aminocarbonyl; R² and R⁸ together with the carbon atoms to which they are attached form a pyrrolidine ring optionally substituted by one or more C₁₋₆alkoxycarbonyl groups; or R² and R⁸ together with the carbon atoms to which they are attached form a pyrazole ring optionally substituted by one or more C₁₋₆alkyl groups.

Such a compound of Formula **I,** wherein X represents CR⁸R⁹ and R⁴ and R⁸ together with the carbon atoms to which they are attached form a 5 membered ring optionally containing one or two heteroatoms selected from sulfur, oxygen, and nitrogen, wherein the ring may be optionally substituted by one or more C₁₋₆alkoxycarbonyl groups.

Such a compound of Formula **I**, wherein Y represents -CR¹¹R¹², O, NR¹⁷, or a bond.

Such a compound of Formula **I,** wherein Y represents NR¹⁷ and R¹⁷ represents C₁₋₆alkyl; or Y represents -CR¹¹R¹² and R¹¹ and R¹² each represent H or R⁴ and R¹¹ together with the carbon atoms to which they are attached form a 5 or 6 membered ring optionally containing one or two heteroatoms selected from sulfur, oxygen, and nitrogen, wherein the ring may be optionally substituted by one or more C₁₋₆alkoxycarbonyl groups.

Such a compound of Formula **I,** wherein R⁴ and R¹¹ together with the carbon atoms to which they are attached form a pyrrolidine ring optionally substituted by C₁₋₆alkoxycarbonyl, a cyclopentane ring, or a cyclohexane ring.

Such a compound of Formula **I,** wherein R¹ represents phenyl optionally substituted by one or more substituents selected from F, C₁₋₆alkyl, and cyano; pyridyl optionally substituted by one or more amino groups; or imidazopyridyl.

Such a compound of Formula **I,** wherein R¹ represents phenyl substituted by one or more fluorine atoms.

Such a compound of Formula **I,** wherein R¹ represents phenyl substituted by one or more C₁₋₆alkyl (e.g., methyl) groups.

Such a compound of Formula **I,** wherein R¹ represents phenyl substituted by one or more cyano groups.

Such a compound of Formula **I,** wherein R¹ represents pyridyl substituted by one or more amino groups.

Such a compound of Formula **I**, wherein R¹ represents imidazopyridyl.

A further aspect of the invention relates to a compound of Formula **I**, which is selected from those of Formula **IA** 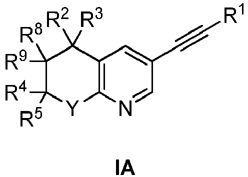 wherein
R¹-R⁵, R⁸-R⁹, and Y are as defined above for Formula I,
and optical isomers, prodrugs, pharmaceutically acceptable salts, hydrates, solvates, and polymorphs thereof.

A further aspect of the invention relates to a compound of Formula **IA**, wherein R¹ represents aryl or heteroaryl.

A further aspect of the invention relates to a compound of Formula **IA**, wherein R¹ represents aryl or heteroaryl; R³-R⁵ and R⁹ each represent hydrogen; Y represents CH₂; and R² and R⁸ together with the carbon atoms to which they are attached form a 5 or 6 membered ring optionally containing one or two heteroatoms selected from sulfur, oxygen, and nitrogen, wherein the ring may be optionally substituted by one or more substituents selected from C₁₋₆alkyl, trifluoroethyl, trifluoropropyl, C₁₋₆alkoxycarbonyl, and di-(C₁₋₆alkyl)aminocarbonyl.

Such a compound of Formula **IA**, wherein R² and R⁸ together with the carbon atoms to which they are attached form an oxazine ring optionally substituted by one or more substituents selected from C₁₋₆alkyl, trifluoroethyl, trifluoropropyl, C₁₋₆alkoxycarbonyl, and di-(C₁₋₆alkyl)aminocarbonyl; or R² and R⁸ together with the carbon atoms to which they are attached form a pyrrolidine ring optionally substituted by one or more C₁₋₆alkoxycarbonyl groups.

Such a compound of Formula **IA**, wherein wherein R² and R⁸ together with the carbon atoms to which they are attached form an oxazine ring substituted by one or more C₁₋₆alkyl (e.g., methyl, ethyl, propyl, isopropyl) groups.

Such a compound of Formula **IA,** wherein R² and R⁸ together with the carbon atoms to which they are attached form an oxazine ring substituted by one or more trifluoroethyl groups.

Such a compound of Formula **IA,** wherein R² and R⁸ together with the carbon atoms to which they are attached form an oxazine ring substituted by one or more trifluoropropyl groups.

Such a compound of Formula **IA,** wherein R² and R⁸ together with the carbon atoms to which they are attached form an oxazine ring substituted by one or more C₁₋₆alkoxycarbonyl (e.g., methoxycarbonyl) groups.

Such a compound of Formula **IA,** wherein R² and R⁸ together with the carbon atoms to which they are attached form an oxazine ring substituted by one or more di-(C₁₋₆alkyl)aminocarbonyl (e.g., dimethylaminocarbonyl) groups.

Such a compound of Formula **IA,** wherein R² and R⁸ together with the carbon atoms to which they are attached form a pyrrolidine ring substituted by one or more C₁₋₆alkoxycarbonyl (e.g., methoxycarbonyl) groups.

Such a compound of Formula **IA,** wherein R¹ represents phenyl optionally substituted by one or more substituents selected from F, C₁₋₆alkyl, and cyano; pyridyl optionally substituted by one or more amino groups; or imidazopyridyl.

Such a compound of Formula **IA,** wherein R¹ represents phenyl substituted by one or more fluorine atoms.

Such a compound of Formula **IA,** wherein R¹ represents phenyl substituted by one or more C₁₋₆alkyl (e.g., methyl) groups.

Such a compound of Formula **IA,** wherein R¹ represents phenyl substituted by one or more cyano groups.

Such a compound of Formula **IA**, wherein R¹ represents pyridyl substituted by one or more amino groups.

Such a compound of Formula **IA**, wherein R¹ represents imidazopyridyl.

A further aspect of the invention relates to a compound of Formula **IA,** wherein R¹ represents aryl or heteroaryl; R², R³, R⁵ and R⁹ each represent hydrogen; Y represents a bond; and R⁴ and R⁸ together with the carbon atoms to which they are attached form a 5 membered ring optionally containing one or two heteroatoms selected from sulfur, oxygen, and nitrogen, wherein the ring may be optionally substituted by one or more C₁₋₆alkoxycarbonyl groups.

Such a compound of Formula **IA,** wherein R⁴ and R⁸ together with the carbon atoms to which they are attached form a pyrrolidine ring optionally substituted by one or more C₁₋₆alkoxycarbonyl (e.g., methoxycarbonyl) groups.

Such a compound of Formula **IA**, wherein R¹ represents phenyl optionally substituted by one or more substituents selected from F, C₁₋₆alkyl, and cyano; pyridyl optionally substituted by one or more amino groups; or imidazopyridyl.

Such a compound of Formula **IA,** wherein R¹ represents phenyl substituted by one or more fluorine atoms.

Such a compound of Formula **IA,** wherein R¹ represents phenyl substituted by one or more C₁₋₆alkyl (e.g., methyl) groups.

Such a compound of Formula **IA,** wherein R¹ represents phenyl substituted by one or more cyano groups.

Such a compound of Formula **IA,** wherein R¹ represents pyridyl substituted by one or more amino groups.

Such a compound of Formula **IA,** wherein R¹ represents imidazopyridyl.

A further aspect of the invention relates to a compound of Formula I, which is selected from those of Formula **IB** 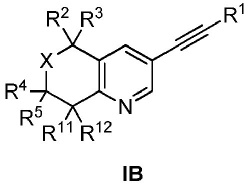 wherein
R¹-R⁵, R¹¹-R¹², and X are as defined above for Formula **I**,
and optical isomers, prodrugs, pharmaceutically acceptable salts, hydrates, solvates, and polymorphs thereof.

A further aspect of the invention relates to a compound of Formula **IB,** wherein R¹ represents aryl or heteroaryl.

A further aspect of the invention relates to a compound of Formula **IB,** wherein X represents CR⁸R⁹ or NR¹⁰, wherein R⁸-R¹⁰ are as defined above for Formula **I,** and R⁴ and R¹¹ together with the carbon atoms to which they are attached form a 5 or 6 membered ring optionally containing one or two heteroatoms selected from sulfur, oxygen, and nitrogen, wherein the ring may be optionally substituted by one or more C₁₋₆alkoxycarbonyl groups.

Such a compound of Formula **IB,** wherein X represents CR⁸R⁹, wherein R⁸ and R⁹ each represent H or X represents NR¹⁰ wherein R¹⁰ represents C₁₋₆alkoxycarbonyl (e.g., methoxycarbonyl).

Such a compound of Formula **IB,** wherein R⁴ and R¹¹ together with the carbon atoms to which they are attached form a pyrrolidine ring optionally substituted by one or more C₁₋₆alkoxycarbonyl (e.g., methoxycarbonyl) groups, a cyclopentane ring, or a cyclohexane ring.

Such a compound of Formula **IB,** wherein R¹ represents phenyl optionally substituted by one or more substituents selected from F, C₁₋₆alkyl, and cyano; pyridyl optionally substituted by one or more amino groups; or imidazopyridyl.

Such a compound of Formula **IB,** wherein R¹ represents phenyl substituted by one or more fluorine atoms.

Such a compound of Formula **IB,** wherein R¹ represents phenyl substituted by one or more C₁₋₆alkyl (e.g., methyl) groups.

Such a compound of Formula **IB,** wherein R¹ represents phenyl substituted by one or more cyano groups.

Such a compound of Formula **IB,** wherein R¹ represents pyridyl substituted by one or more amino groups.

Such a compound of Formula **IB,** wherein R¹ represents imidazopyridyl.

A further aspect of the invention relates to a compound of Formula **I,** which is selected from those of Formula **IC** 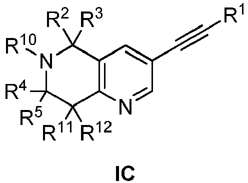 wherein
R¹-R⁵ and R¹⁰-R¹² are as defined above for Formula I,
and optical isomers, prodrugs, pharmaceutically acceptable salts, hydrates, solvates, and polymorphs thereof.

A further aspect of the invention relates to a compound of Formula **IC,** wherein R¹ represents aryl or heteroaryl.

A further aspect of the invention relates to a compound of Formula **IC,** wherein R¹ represents aryl or heteroaryl and R¹⁰ represents C₁₋₆alkylcarbonyl, cycloC₃₋₁₂alkylcarbonyl, C₁₋₆alkoxycarbonyl, aryloxycarbonyl, di-(C₁₋₆alkyl)aminocarbonyl (wherein the alkyl moieties may be optionally substituted by one or more halogen atoms), -C(O)-C(O)-C₁₋₆alkoxy, C₁₋₆alkylsulfinyl, C₁₋₆alkylsulfonyl, cycloC₃₋₇alkylsulfonyl, arylsulfonyl, heterocyclylsulfonyl, or di-(C₁₋₆alkyl)aminosulfonyl.

A further aspect of the invention relates to a compound of Formula **IC**, wherein R¹ represents aryl or heteroaryl and R⁴ and R¹⁰ together with the atoms to which they are attached form a 6-membered ring optionally containing one or two additional heteroatoms selected from sulfur, oxygen, and nitrogen, wherein the ring may be optionally substituted by one or more substituents selected from oxo and C₁₋₆alkyl.

Such a compound of Formula **IC,** wherein R⁴ and R¹⁰ together with the atoms to which they are attached form a tetrahydropyrimidine ring optionally substituted by one or more substituents selected from oxo and C₁₋₆alkyl (e.g., methyl).

Such a compound of Formula **IC,** wherein R⁴ and R¹⁰ together with the atoms to which they are attached form a tetrahydropyrimidine ring substituted by two or more substituents selected from oxo and C₁₋₆alkyl (e.g., methyl).

Such a compound of Formula **IC,** wherein R¹ represents phenyl optionally substituted by one or more substituents selected from F, C₁₋₆alkyl, and cyano; pyridyl optionally substituted by one or more amino groups; or imidazopyridyl.

Such a compound of Formula **IC**, wherein R¹ represents phenyl substituted by one or more fluorine atoms.

Such a compound of Formula **IC**, wherein R¹ represents phenyl substituted by one or more C₁₋₆alkyl (e.g., methyl) groups.

Such a compound of Formula **IC**, wherein R¹ represents phenyl substituted by one or more cyano groups.

Such a compound of Formula **IC,** wherein R¹ represents pyridyl substituted by one or more amino groups.

Such a compound of Formula **IC**, wherein R¹ represents imidazopyridyl.

A further aspect of the invention relates to a compound of Formula I, which is selected from those of Formula **ID**, 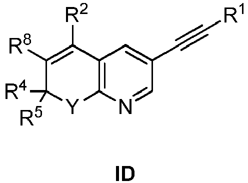 wherein
R², R⁴-R⁵, R⁸, and Y are as defined above for Formula **I**,
and optical isomers, prodrugs, pharmaceutically acceptable salts, hydrates, solvates, and polymorphs thereof.

A further aspect of the invention relates to a compound of Formula **ID**, wherein R¹ represents aryl or heteroaryl.

A further aspect of the invention relates to a compound of Formula **ID,** wherein R² and R⁸ together with the carbon atoms to which they are attached form a 5-membered heteroaromatic ring optionally substituted by C₁₋₆alkyl.

Such a compound of Formula **ID,** wherein R² and R⁸ together with the carbon atoms to which they are attached form a pyrazole ring optionally substituted by one or more C₁₋₆alkyl (e.g., methyl) groups.

Such a compound of Formula **ID,** wherein R¹ represents phenyl optionally substituted by one or more substituents selected from F, C₁₋₆alkyl, and cyano; pyridyl optionally substituted by one or more amino groups; or imidazopyridyl.

Such a compound of Formula **ID,** wherein R¹ represents phenyl substituted by one or more fluorine atoms.

Such a compound of Formula **ID,** wherein R¹ represents phenyl substituted by one or more C₁₋₆alkyl (e.g., methyl) groups.

Such a compound of Formula **ID,** wherein R¹ represents phenyl substituted by one or more cyano groups.

Such a compound of Formula **ID,** wherein R¹ represents pyridyl substituted by one or more amino groups.

Such a compound of Formula **ID,** wherein R¹ represents imidazopyridyl.

Specific compounds of **Formula** I within the present invention include, but are not limited to, the following compounds:
(*rac*)-5-Methoxy-3-(phenylethynyl)-5,6,7,8-tetrahydroquinoline,
5-Methoxy-3-(phenylethynyl)-5,6,7,8-tetrahydroquinoline,
(*rac*)-6-((5-Methoxy-5,6,7,8-tetrahydroquinolin-3-yl)ethynyl)pyridin-2-amine,
(*rac*)-5-Methoxy-7,7-dimethyl-3-(phenylethynyl)-5,6,7,8-tetrahydroquinoline,
(*rac*)-6-((5-Methoxy-7,7-dimethyl-5,6,7,8-tetrahydroquinolin-3-yl)ethynyl)pyridin-2-amine,
(*rac*)-3-(Imidazo[1,2-a]pyridin-2-ylethynyl)-5-methoxy-7,7-dimethyl-5,6,7,8-tetrahydroquinoline,
(*rac*)-6-((4-Methoxy-2,2-dimethyl-3,4-dihydro-2*H*-pyrano[2,3-b]pyridin-6-yl)ethynyl)pyridin-2-am ine,
(*rac*)- 6-(Imidazo[1,2-a]pyridin-2-ylethynyl)-4-methoxy-2,2-dimethyl-3,4-dihydro-2*H-*pyrano[2,3-*b*]pyridine,
4-Methoxy-1-methyl-6-(phenylethynyl)-1,2,3,4-tetrahydro-1,8-naphthyridine,
(*rac*)-3'-(Phenylethynyl)-7',8'-dihydro-6'*H*-spiro[oxazolidine-5,5'-quinolin]-2-one,
(*rac*)-3-Methyl-3'-(phenylethynyl)-7',8'-dihydro-6'*H*-spiro[oxazolidine-5,5'-quinolin]-2-one,
(*rac*)-3-Phenyl-3'-(phenylethynyl)-7',8'-dihydro-6'*H*-spiro[oxazolidine-5,5'-quinolin]-2-one,
(*rac*)-*cis*-4-Methyl-9-(phenylethynyl)-3,4,4a,5,6,1 0b-hexahydro-2*H*-[1,4]oxazino[2,3-*f*]quinoline,
(*rac*)-*cis*-4-Ethyl-9-(phenylethynyl)-3,4,4a,5,6,10b-hexahydro-2*H*-[1,4]oxazino[2,3-*f*]quinoline,
(*rac*)-*cis*-9-(Phenylethynyl)-4-propyl-3,4,4a,5,6,10b-hexahydro-2*H*-[1,4]oxazino[2,3-*f*]quinoline,
*cis*-9-(Phenylethynyl)-4-propyl-3,4,4a,5,6,10b-hexahydro-2*H*-[1,4]oxazino[2,3-*f*]quinoline,
*cis*-9-(Phenylethynyl)-4-propyl-3,4,4a,5,6,10b-hexahydro-2*H*-[1,4]oxazino[2,3-*f*]quinoline,
(*rac*)-cis-9-((2-Fluorophenyl)ethynyl)-4-propyl-3,4,4a,5,6,10b-hexahydro-2*H-*[1,4]oxazino[2,3-f]quinoline,
(*rac*)-*cis*-9-((3-Fluorophenyl)ethynyl)-4-propyl-3,4,4a,5,6,10b-hexahydro-2*H-*[1,4]oxazino[2,3-*f*]quinoline,
(*rac*)-*cis*-9-((4-Fluorophenyl)ethynyl)-4-propyl-3,4,4a,5,6,10b-hexahydro-2*H-*[1,4]oxazino[2,3-*f*]quinoline,
(*rac*)-*cis*-4-Propyl-9-(m-tolylethynyl)-3,4,4a,5,6,10b-hexahydro-2*H*-[1,4]oxazino[2,3-*f*]quinoline,
(*rac*)-*trans*-9-(Phenylethynyl)-4-propyl-3,4,4a,5,6,10b-hexahydro-2*H*-[1,4]oxazino[2,3-*f*]quinoline,
(*rac*)-*cis*-4-Isobutyl-9-(phenylethynyl)-3,4,4a,5,6,10b-hexahydro-2*H*-[1,4]oxazino[2,3-*f*]quinoline,
(*rac*)-*cis*-9-(Phenylethynyl)-4-(2,2,2-trifluoroethyl)-3,4,4a,5,6,10b-hexahydro-2*H-*[1,4]oxazino[2,3-*f*]quinoline,
(*rac*)-*cis*-9-(Phenylethynyl)-4-(3,3,3-trifluoropropyl)-3,4,4a,5,6,10b-hexahydro-2*H-*[1,4]oxazino[2,3-*f*]quinoline,
(*rac*)-cis-Methyl 9-(phenylethynyl)-4a,5,6,10b-tetrahydro-2*H*-[1,4]oxazino[2,3-f]quinoline-4(3*H*)-carboxylate,
(*rac*)-*cis*-*N*,*N*-Dimethyl-9-(phenylethynyl)-4a,5,6,10b-tetrahydro-2*H*-[1,4]oxazino[2,3-*f*]quinoline-4(3*H*)-carboxamide,
1-Methyl-8-(phenylethynyl)-4,5-dihydro-1*H*-pyrazolo[3,4-f]quinoline,
(*rac*)-*cis*-Methyl 8-(phenylethynyl)-2,3,3a,4,5,9b-hexahydro-1*H*-pyrrolo[2,3-*f*]quinoline-1-carboxylate,
Methyl 8-(phenylethynyl)-3,3a,4,5-tetrahydro-1*H*-pyrrolo[3,4-*f*]quinoline-2(9b*H*)-carboxylate,
Methyl 8-((3-fluorophenyl)ethynyl)-3,3a,4,5-tetrahydro-1*H*-pyrrolo[3,4-*f*]quinoline-2(9b*H*)-carboxylate,
(*rac*)-*cis*-Methyl 3-((3-fluorophenyl)ethynyl)-6a,7,9,9a-tetrahydro-5*H*-pyrrolo[3,4-h]quinoline-8(6*H*)-carboxylate,
2-Methyl-1-(3-(phenylethynyl)-7,8-dihydro-1,6-naphthyridin-6(5*H*)-yl)propan-1-one,
2,2-Dimethyl-1-(3-(phenylethynyl)-7,8-dihydro-1,6-naphthyridin-6(5*H*)-yl)propan-1-one,
1-(3-((6-Aminopyridin-2-yl)ethynyl)-7,8-dihydro-1,6-naphthyridin-6(5*H*)-yl)-2,2-dimethylpropan-1-one,
(3-Methyloxetan-3-yl)(3-(phenylethynyl)-7,8-dihydro-1,6-naphthyridin-6(5*H*)-yl)methanone,
(*rac*)-6-(*tert*-Butylsulfinyl)-3-(phenylethynyl)-5,6,7,8-tetrahydro-1,6-naphthyridine,
6-(Cyclopropylsulfonyl)-3-(phenylethynyl)-5,6,7,8-tetrahydro-1,6-naphthyridine,
3-(Phenylethynyl)-6-(phenylsulfonyl)-5,6,7,8-tetrahydro-1,6-naphthyridine,
Ethyl 3-(phenylethynyl)-7,8-dihydro-1,6-naphthyridine-6(5*H*)-carboxylate,
Isobutyl 3-(phenylethynyl)-7,8-dihydro-1,6-naphthyridine-6(5*H*)-carboxylate,
*tert*-Butyl 3-(phenylethynyl)-7,8-dihydro-1,6-naphthyridine-6(5*H*)-carboxylate,
Phenyl 3-(phenylethynyl)-7,8-dihydro-1,6-naphthyridine-6(5*H*)-carboxylate,
N,N-Dimethyl-3-(phenylethynyl)-7,8-dihydro-1,6-naphthyridine-6(5*H*)-carboxamide,
3-((3-Fluorophenyl)ethynyl)-*N,N-*dimethyl-7,8-dihydro-1,6-naphthyridine-6(5*H*)-carboxam ide,
3-((3-Cyanophenyl)ethynyl)-*N,N*-dimethyl-7,8-dihydro-1,6-naphthyridine-6(5*H*)-carboxam ide,
3-((6-Aminopyridin-2-yl)ethynyl)-*N,N*-dimethyl-7,8-dihydro-1,6-naphthyridine-6(5*H*)-carboxam ide,
3-((3-Cyanophenyl)ethynyl)-*N,N*-diethyl-7,8-dihydro-1,6-naphthyridine-6(5*H*)-carboxam ide,
3-(Phenylethynyl)-*N,N*-bis(2,2,2-trifluoroethyl)-7,8-dihydro-1,6-naphthyridine-6(5*H*)-carboxam ide,
(3-(Phenylethynyl)-7,8-dihydro-1,6-naphthyridin-6(5*H*)-yl)(piperidin-1-yl)methanone, 3-((6-(Piperidine-1-carbonyl)-5,6,7,8-tetrahydro-1,6-naphthyridin-3-yl)ethynyl)benzonitrile,
*N,N*-Dimethyl-3-(phenylethynyl)-7,8-dihydro-1,6-naphthyridine-6(5*H*)-sulfonamide,
3-(Phenylethynyl)-6-(pyrrolidin-1-ylsulfonyl)-5,6,7,8-tetrahydro-1,6-naphthyridine,
3-(Phenylethynyl)-6-(piperidin-1-ylsulfonyl)-5,6,7,8-tetrahydro-1,6-naphthyridine,
4-((3-(Phenylethynyl)-7,8-dihydro-1,6-naphthyridin-6(5*H*)-yl)sulfonyl)morpholine,
6-(Azepan-1-ylsulfonyl)-3-(phenylethynyl)-5,6,7,8-tetrahydro-1,6-naphthyridine,
(*rac*)-8-Methyl-3-(phenylethynyl)-9,10,10a,11-tetrahydro-5*H*-pyrimido[1,6-*g*][1,6]naphthyridin-7(8H)-one,
(*rac*)-Methyl 3-(phenylethynyl)-6,7,8,9-tetrahydro-5*H*-5,8-epiminocyclohepta[b]pyridine-10-carboxylate,
(*rac*)-Methyl 3-((3-fluorophenyl)ethynyl)-6,7,8,9-tetrahydro-5*H*-5,8-epiminocyclohepta[b]pyridine-10-carboxylate,
(*rac*)-*cis*-Methyl 3-(phenylethynyl)-7,8,9,9a-tetrahydro-5*H-*cyclopenta[*h*][1,6]naphthyridine-6(6a*H*)-carboxylate,
(*rac*)-*cis*-Methyl 3-((3-fluorophenyl)ethynyl)-7,8,9,9a-tetrahydro-5*H-*cyclopenta[*h*][1,6]naphthyridine-6(6a*H*)-carboxylate,
(*rac*)-*cis*-Methyl 3-(phenylethynyl)-6a,7,8,9,10,10a-hexahydrobenzo[*h*][1,6]naphthyridine-6(5*H*)-carboxylate,
(*rac*)-*cis*-Methyl 3-((3-fluorophenyl)ethynyl)-6a,7,8,9,10,10a-hexahydrobenzo[*h*][1,6]naphthyridine-6(5*H*)-carboxylate,
(*rac*)-3-Propyl-3'-(m-tolylethynyl)-7',8'-dihydro-6'H-spiro[oxazolidine-5,5'-quinolin]-2-one,
(*rac*)-*cis*-Azetidin-1-yl-(9-(phenylethynyl)-5,6-dihydro-2*H*-[1,4]oxazino[2,3-*f*]quinolin-4(3*H*,4a*H*,10b*H*)-yl)methanone,
(*rac*)-3'-((3-Fluorophenyl)ethynyl)-3-propyl-7',8'-dihydro-6'*H*-spiro[oxazolidine-5,5'-quinolin]-2-one,
(*rac*)-3'-((3-Fluorophenyl)ethynyl)-3-phenyl-7',8'-dihydro-6'*H*-spiro[oxazolidine-5,5'-quinolin]-2-one,
3-(*m*-Tolylethynyl)-7,8-dihydro-5*H*-thiopyrano[4,3-*b*]pyridine 6,6-dioxide,
3-((3-Fluorophenyl)ethynyl)-7,8-dihydro-5*H*-thiopyrano[4,3-*b*]pyridine 6,6-dioxide,
3-((4-Fluorophenyl)ethynyl)-7,8-dihydro-5H-thiopyrano[4,3-*b*]pyridine 6,6-dioxide,
(6a*R*,9a*R*)-Methyl 3-(phenylethynyl)-6,6a,9,9a-tetrahydro-5*H*-pyrrolo[2,3-*h*]quinoline-7(8*H*)-carboxylate,
(6a*R*,9a*R*)-Methyl 3-((3-fluorophenyl)ethynyl)-6,6a,9,9a-tetrahydro-5*H*-pyrrolo[2,3-h]quinoline-7(8*H*)-carboxylate,
(*rac*)-*cis*-Methyl 3-((3-fluorophenyl)ethynyl)-5a,6,8,8a-tetrahydropyrrolo[3',4':4,5]cyclopenta[1,2-*b*]pyridine-7(5*H*)-carboxylate,
(*rac*)-3-Cyclopropyl-3'-(phenylethynyl)-7',8'-dihydro-6'*H*-spiro[oxazolidine-5,5'-quinolin]-2-one,
5,5-Dimethyl-3-(phenylethynyl)-7,8-dihydro-5*H*-thiopyrano[4,3-*b*]pyridine-6,6-dioxide, (*rac*)-3-Cyclopropyl-3'-((3-fluorophenyl)ethynyl)-7',8'-dihydro-6'*H*-spiro[oxazolidine-5,5'-quinolin]-2-one,
Ethyl 2-oxo-2-(3-(phenylethynyl)-7,8-dihydro-1,6-naphthyridin-6(5*H*)-yl)acetate, 6-((4-Methylpiperazin-1-yl)sulfonyl)-3-(phenylethynyl)-5,6,7,8-tetrahydro-1,6-naphthyridine,
(*rac*)-Methyl 3-(phenylethynyl)-5a,6,8,8a-tetrahydropyrrolo[3',4':4,5]cyclopenta[1,2-*b*]pyridine-7(5H)-carboxylate,
(*rac*)-*cis*-Methyl 7-(phenylethynyl)-2,3,3a,4,5,9b-hexahydro-1*H*-pyrrolo[3,2-h]quinoline-1-carboxylate,
6-(Isopropylsulfonyl)-3-(phenylethynyl)-5,6,7,8-tetrahydro-1,6-naphthyridine, (*rac*)-3'-(Phenylethynyl)-3-propyl-7',8'-dihydro-6'H-spiro[oxazolidine-5,5'-quinolin]-2-one
and optical isomers, prodrugs, pharmaceutically acceptable salts, hydrates, solvates, and polymorphs thereof.

In a further aspect, the invention relates to a compound of Formula I as defined above or an optical isomer, pharmaceutically acceptable salt, hydrate, solvate or polymorph thereof for use in therapy.

Moreover, the invention relates to a compound of Formula **I** as defined above or an optical isomer, pharmaceutically acceptable salt, hydrate, solvate or polymorph thereof for the treatment and/or prevention of a condition or disease associated with abnormal glutamate neurotransmission, including a condition or disease which is affected or facilitated by modulation of the mGluR5 receptor, including for the conditions or diseases selected from those described earlier in the description.

In a further aspect, the invention relates to a compound of Formula **I** as defined above or an optical isomer, pharmaceutically acceptable salt, hydrate, solvate or polymorph thereof for the treatment and/or prevention of a CNS disorder. Moreover, the invention relates to a compound of Formula I as defined above or an optical isomer, pharmaceutically acceptable salt, hydrate, solvate or polymorph thereof for the treatment and/or prevention of abnormal glutamate neurotransmission. The invention additionally relates to a compound of Formula **I** as defined above or an optical isomer, pharmaceutically acceptable salt, hydrate, solvate or polymorph thereof for modulation of the mGluR5 receptor. The invention furthermore relates to such a compound for treatment, prevention and or modulation of a condition or disease selected from those described earlier in the description. The invention still further relates to such a compound for treatment, prevention and or modulation of a a physiological parameter, such as cognitive disorder, whether or not a specific identifiable condition exists.

A further aspect of the invention relates to a compound of Formula **I** as defined above or an optical isomer, pharmaceutically acceptable salt, hydrate, solvate or polymorph thereof for the treatment and/or prevention of a condition associated with abnormal glutamate neurotransmission or in which modulation of mGluR5 receptors results in therapeutic benefit. The conditions which may be treated have already been described above. Such conditions and indications include:
a) For mGluR5 modulators: chronic pain, neuropathic pain, diabetic neuropathic pain (DNP), cancer pain, pain related to rheumathic arthritis, inflammatory pain, L-DOPA-induced dyskinesias, dopaminomimetic-induced dyskinesias, L-DOPA-induced dyskinesias in Parkinson's disease therapy, dopaminomimetic-induced dyskinesias in Parkinson's disease therapy, tardive dyskinesias, Parkinson's disease, anxiety disorders, panic disorders, anxiety and panic disorders, social anxiety disorder (SAD), generalized anxiety disorder, substance-induced anxiety disorder, eating disorders, obesity, binge eating disorders, Huntington's chorea, epilepsy, Alzheimer's disease, positive and negative symptoms of schizophrenia, cognitive deficits of schizophrenia, cognitive impairment, functional gastrointestinal disorders, gastroesophageal reflux disease (GERD), migraine, irritable bowel syndrome (IBS), or for cognitive enhancement and/or neuroprotection.
b) Negative modulation of mGluR5 may be particularly useful for: chronic pain, neuropathic pain, diabetic neuropathic pain (DNP), cancer pain, pain related to rheumathic arthritis, inflammatory pain, L-DOPA-induced dyskinesias, dopaminomimetic-induced dyskinesias, L-DOPA-induced dyskinesias in Parkinson's disease therapy, dopaminomimetic-induced dyskinesias in Parkinson's disease therapy, tardive dyskinesias, Parkinson's disease, anxiety disorders, panic disorders, anxiety and panic disorders, social anxiety disorder (SAD), generalized anxiety disorder, substance-induced anxiety disorder, eating disorders, obesity, binge eating disorders, migraine, irritable bowel syndrome (IBS), functional gastrointestinal disorders, gastroesophageal reflux disease (GERD), Huntington's chorea and/or epilepsy.
c) Positive modulation of mGluR5 may be particularly useful for: Alzheimer's disease, positive and/or negative symptoms of schizophrenia, cognitive deficits of schizophrenia, cognitive impairment, or for cognitive enhancement and/or neuroprotection.

A further aspect of the invention relates to a compound of Formula I as defined above or an optical isomer, pharmaceutically acceptable salt, hydrate, solvate or polymorph thereof for the treatment of binge eating disorders.

Further, the invention relates to the use of a compound of Formula I as defined above or an optical isomer, pharmaceutically acceptable salt, hydrate, solvate or polymorph thereof for the preparation of a medicament for treating or preventing a condition or disease associated with abnormal glutamate neurotransmission. Such a use includes the use of such a compound for the preparation of a medicament for the prevention and/or treatment of a condition or disease in an animal including a human being which condition or disease is affected or facilitated by modulation of the mGluR5 receptor.

Moreover, the invention relates to a method for treating or preventing a condition associated or disease associated with abnormal glutamate neurotransmission, including a condition or disease which is affected or facilitated by modulation of the mGluR5 receptor, including for the conditions or diseases selected from those described earlier in the description.

A further aspect of the invention relates to the use of isotopic derivatives of the compounds of Formula I as PET ligands.

Further, the invention relates to a pharmaceutical composition comprising as active ingredient at least one compound of Formula **I** as defined above or an optical isomer, pharmaceuctically acceptable salt, hydrate, solvate or polymorph thereof, together with one or more pharmaceutically acceptable excipients.

Moreover, the mGluR modulators as described above are expected to have a high activity when administered in combination with other substances exhibiting neurological effects via different mechanisms.

In a yet further aspect, the invention thus relates to a compound of Formula **I** as defined above or an optical isomer, pharmaceutically acceptable salt, hydrate, solvate or polymorph thereof for neuroprotection and/or for cognitive enhancement in combination with at least one NMDA receptor antagonist such as Memantine.

A further aspect of the invention relates to a pharmaceutical composition comprising at least two different active ingredients, selected from least one compound of Formula **I** as defined above, and, additionally, at least one NMDA-antagonist, together with one or more pharmaceutically acceptable excipients. These compositions may be used for the treatment of CNS-related diseases, cognitive enhancement and for neuro-protection. The invention thus additionally provides a composition comprising at least two different active ingredients, selected from least one compound of Formula I as defined above, and, additionally, at least one NMDA-antagonist for the treatment of any of the conditions indicated herein, including CNS-related diseases, cognitive enhancement and for neuro-protection.

This invention also relates to a pharmaceutical composition comprising a combination of a compound of Formula **I** as described above and an NMDA receptor antagonist, including compositions wherein the NMDA receptor antagonist is e.g. Memantine and pharmaceutically acceptable salts, polymorphs, hydrates and solvates thereof.

The invention also relates to a pharmaceutical composition comprising at least two different active ingredients, selected from at least one compound of Formula **I** as defined above, and, additionally, at least one active ingredient selected from L-DOPA, other dopaminomimetics (such as antiparkinsonian dopaminomimetics, including bromocriptine, cabergolin, ropinirole, pramiperole, pergolide, rotigotine), and neuroleptics (such as classical neuroleptics, including haloperidol, perphenazin, chlorpromazine, metoclopramide or atypicals such as clozapine, risperidone or olanzapine).

The invention also relates to a method of providing neuroprotection in a living animal, including a human, comprising the step of administering to a living animal, including a human, a therapeutically effective amount of a composition as described above.

Furthermore, the invention relates to the use of a composition as described above for the manufacture of a medicament to provide neuroprotection in an animal, including a human.

The invention also relates to a process for the synthesis or preparation of a compound of Formula **I** 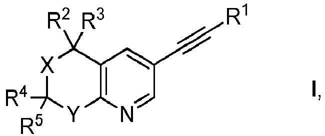 or an optical isomer, prodrug, pharmaceutically acceptable salt, hydrate, solvate, or polymorph thereof, wherein a compound of Formula **II** 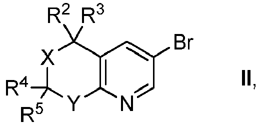 is treated with a substituted acetylene of Formula **III** 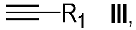 in the presence of a suitable catalyst, such as Pd[PPh₃]₂Cl₂, to yield a compound of Formula **I**, which may be converted to a prodrug, pharmaceutically acceptable salt, hydrate, solvate, or polymorph.

The invention also relates to a process for the synthesis or preparation of a compound of Formula **I** 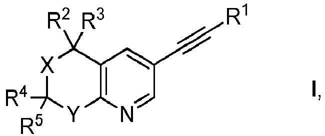 or an optical isomer, prodrug, pharmaceutically acceptable salt, hydrate, solvate, or polymorph thereof, wherein a compound of Formula **II** 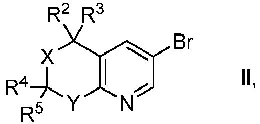 is treated with trimethylacetylene to yield, after removal of the TMS group under appropriate conditions, a compound of Formula **IV** 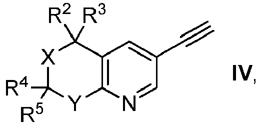 which is reacted with a compound of Formula **V**

R₁-X **V**

,
in the presence of a suitable catalyst, such as PdCl₂(PPh₃)₂, to yield a compound of Formula I, which may be converted to a prodrug, pharmaceutically acceptable salt, hydrate, solvate, or polymorph.

### DETAILED DESCRIPTION OF THE INVENTION

For the purpose of the present invention, in the compounds of Formula **I** the carbon atom content of various hydrocarbon-containing moieties is indicated by a prefix designating the minimum and maximum number of carbon atoms in the moiety, i.e., the prefix Cᵢ₋ⱼ indicates a moiety of the integer "i" to the integer "j" carbon atoms, inclusive. Thus, for example, (C₁₋₃)alkyl refers to alkyl of one to three carbon atoms (i.e. 1, 2 or 3 carbon atoms), inclusive, (i.e., methyl, ethyl, propyl, and isopropyl), straight and branched forms thereof, (C₁₋₆) for instance refers to a radical of one to six carbon atoms (i.e. 1, 2, 3, 4, 5 or 6 carbon atoms).

As used herein, the following definitions are applicable unless otherwise described, the term "C₁₋₆alkyl" represents straight or branched chain alkyl groups. Examples of such alkyl groups include methyl, ethyl, n-propyl, isopropyl, n-butyl, isobutyl, *sec*-butyl, and *tert*-butyl.

The term "C₂₋₆alkenyl" represents straight or branched chain alkenyl groups.

The term "C₁₋₆alkoxy" represents straight or branched chain -O-C₁₋₆alkyl groups. Examples of such alkoxy groups include methoxy, ethoxy, n-propoxy, and isopropoxy, sec-butoxy, *tert*-butoxy.

The term "acyl" represents C₁₋₆alkylcarbonyl, trifluoroacetyl, hydroxy-C₁₋₆alkylcarbonyl, C₁₋₆alkoxycarbonyl, *N*-C₁₋₆alkylaminocarbonyl, *N,N*-di-(C₁₋₆alkyl)aminocarbonyl (wherein the alkyl moieties may be optionally substituted by one or more halogen atoms), C₁₋₆alkoxy-C₁₋₆alkylcarbonyl, arylcarbonyl, heteroarylcarbonyl, cycloC₃₋₁₂alkylcarbonyl, aryloxycarbonyl, heteroaryloxycarbonyl, aryl-C₁₋₆alkylcarbonyl, heteroaryl-C₁₋₆alkylcarbonyl, arylamino-C₁₋₆alkylcarbonyl, heteroarylamino-C₁₋₆alkylcarbonyl, heterocyclylcarbonyl and heterocyclyl-C₁₋₆alkylcarbonyl.

The term "cycloC₃₋₁₂alkyl" represents monocyclic or bicyclic, or tricyclic alkyl groups, including cyclopropyl, cyclobutyl, cyclopentyl, cyclohexyl, bicyclo[2.2.1]heptyl and adamantanyl, which may be optionally substituted by one or more (e.g., 1, 2, 3, 4, or 5) substituents, which may be the same or different, selected independently from halogen, trifluoromethyl, trifluoromethoxy, C₁₋₆alkyl, C₂₋₆alkenyl, C₁₋₆alkoxy, amino, hydroxy, cyano, C₁₋₆alkoxycarbonyl, C₁₋₆alkylamino, and di-(C₁₋₆alkyl)amino, C₁₋₆alkylcarbonylamino, oxo, C₁₋₆alkoxyimino, *N*-C₁₋₆alkylaminocarbonyl, *N,N*-di-(C₁₋₆alkyl)aminocarbonyl, arylC₁₋₆alkoxycarbonyl, and C₁₋₆alkylenedioxy.

The term "cycloC₃₋₇alkyl" represents monocyclic alkyl groups, including cyclopropyl, cyclobutyl, cyclopentyl, and cyclohexyl, which may be optionally substituted by one or more (e.g., 1, 2, 3, 4, or 5) substituents, which may be the same or different, selected independently from halogen, trifluoromethyl, trifluoromethoxy, C₁₋₆alkyl, C₂₋₆alkenyl, C₁₋₆alkoxy, amino, hydroxy, cyano, C₁₋₆alkoxycarbonyl, C₁₋₆alkylcarbonyl, C₁₋₆alkylamino, and di-(C₁₋₆alkyl)amino, C₁₋₆alkylcarbonylamino, oxo, C₁₋₆alkoxyimino, *N*-C₁₋₆alkylaminocarbonyl, *N*,*N*-di-(C₁₋₆alkyl)aminocarbonyl, arylC₁₋₆alkoxycarbonyl, and C₁₋₆alkylenedioxy.

The term "cycloC₅₋₁₂alkenyl" represents monocyclic or bicyclic, or tricyclic alkenyl groups, including cyclopentenyl, cyclohexenyl, and bicyclo[2.2.1]heptenyl, which may be optionally substituted by one or more (e.g., 1, 2, 3, 4, or 5) substituents, which may be the same or different, selected independently from halogen, fluoromethyl, difluoromethyl, trifluoromethyl, trifluoromethoxy, C₁₋₆alkyl, C₂₋₆alkenyl, C₁₋₆alkoxy, amino, hydroxy, cyano, C₁₋₆alkoxycarbonyl, C₁₋₆alkylamino, and di-(C₁₋₆alkyl)amino, C₁₋₆alkylcarbonylamino, oxo, C₁₋₆alkoxyimino, C₁₋₆alkylaminocarbonyl, arylC₁₋₆alkoxycarbonyl, and C₁₋₆alkylenedioxy.

The term "aryl" represents phenyl or naphthyl, wherein the phenyl or naphthyl group is optionally substituted by one or more (e.g., 1, 2, 3, 4, or 5) substituents, which may be the same or different, selected independently from halogen, fluoromethyl, difluoromethyl, trifluoromethyl, fluoromethoxy, difluoromethoxy, trifluoromethoxy, C₁₋₆alkyl, hydroxyC₁₋₆alkyl, C₂₋₆alkenyl, C₁₋₆alkoxy, C₁₋₆alkylthio, C₁₋₆alkoxyC₁₋₆alkyl, amino, hydroxy, nitro, cyano, formyl, C₁₋₆alkylcarbonyl, C₁₋₆alkoxycarbonyl, C₁₋₆alkylcarbonyloxy, C₁₋₆alkylcarbonyloxyC₁₋₆alkyl, C₁₋₆alkylamino, di-(C₁₋₆alkyl)amino, cycloC₃₋₁₂alkylamino, C₁₋₆alkylcarbonylamino, phenylcarbonylamino, aminocarbonyl, *N*-C₁₋₆alkylaminocarbonyl, *N*,*N*-di-(C₁₋₆alkyl)aminocarbonyl, pyrrolidinyl, piperidinyl, morpholinyl, piperazinyl, cycloC₃₋₁₂alkyl, pyridyl, and C₁₋₆alkylenedioxy.

The term "heteroaryl" represents an aromatic 5-6 membered ring containing from one to four heteroatoms selected from oxygen, sulfur and nitrogen, or a bicyclic group comprising a 5-6 membered ring containing from one to four heteroatoms selected from oxygen, sulfur and nitrogen fused with a benzene ring or a 5-6 membered ring containing from one to four heteroatoms selected from oxygen, sulfur and nitrogen, wherein the heteroaryl group may be optionally substituted by one or more (e.g., 1, 2, 3, 4, or 5) substituents, which may be the same or different, selected independently from halogen, fluoromethyl, difluoromethyl, trifluoromethyl, fluoromethoxy, difluoromethoxy, trifluoromethoxy, C₁₋₆alkyl, hydroxyC₁₋₆alkyl, C₂₋₆alkenyl, C₁₋₆alkoxy, C₁₋₆alkylthio, amino, hydroxy, nitro, cyano, C₁₋₆alkylcarbonyl, C₁₋₆alkoxycarbonyl, C₁₋₆alkoxycarbonyloxy, C₁₋₆alkylamino, di-(C₁₋₆alkyl)amino, cycloC₃₋₁₂alkylamino, C₁₋₆alkylcarbonylamino, aminocarbonyl, *N*-C₁₋₆alkylaminocarbonyl, *N*,*N*-di-(C₁₋₆alkyl)aminocarbonyl, pyrrolidinyl, piperidinyl, morpholinyl, cycloC₃₋₁₂alkyl, C₁₋₆alkylenedioxy, aryl, and pyridyl. Representative heteroaryl groups include furyl, thienyl, pyrrolyl, oxazolyl, isoxazolyl, isothiazolyl, oxadiazolyl, pyrazolyl, triazolyl, thiadiazolyl, thiazolyl, imidazolyl, oxadiazolyl, tetrazolyl, pyridyl, pyrimidyl, pyridazinyl, pyrazinyl, triazinyl, purinyl, benzofuryl, benzothienyl, indolyl, indolizinyl, isoindolyl, indolinyl, indazolyl, benzimidazolyl, benzoxazolyl, benzothiazolyl, quinolinyl, quinazolinyl, quinoxalinyl, cinnolinyl, naphthyridinyl, isoquinolinyl, quinolizinyl, phthalazinyl, and pteridinyl.

The term "heterocyclyl" represents a saturated or unsaturated non-aromatic 3 to 12 membered ring comprising one to four heteroatoms selected from oxygen, sulfur and nitrogen, and a saturated or unsaturated non-aromatic bicyclic ring system having 3 to 12 members comprising one to six heteroatoms selected from oxygen, sulfur and nitrogen, wherein the heterocyclic ring or ring system may be optionally substituted by one or more (e.g., 1, 2, 3, 4, or 5) substituents, which may be the same or different, selected independently from halogen, trifluoromethyl, fluoromethoxy, difluoromethoxy, trifluoromethoxy, C₁₋₆alkyl, C₂₋₆alkenyl, C₁₋₆alkoxy, amino, hydroxy, nitro, cyano, C₁₋₆alkoxycarbonyl, C₁₋₆alkylcarbonyl, C₁₋₆alkylamino, and di-(C₁₋₆alkyl)amino, C₁₋₆alkylcarbonylamino, oxo, C₁₋₆alkoxyimino, *N*-C₁₋₆alkylaminocarbonyl, *N*,*N*-di-(C₁₋₆alkyl)aminocarbonyl, arylC₁₋₆alkoxycarbonyl, and C₁₋₆alkylenedioxy; examples of such heterocyclyl groups include oxetanyl, piperidinyl, morpholinyl, thiomorpholinyl, imidazolinyl, imidazolidinyl, pyrazolinyl, pyrazolidinyl, pyrrolinyl, pyrrolidinyl, or piperazinyl, wherein the heterocyclic ring or ring system is linked to the group to which it is attached optionally via nitrogen or a carbon atom.

The term "halogen" represents fluorine, chlorine, bromine and iodine.

The compounds of the present invention are usually named according to the IUPAC or CAS nomenclature system. Abbreviations which are well known to one of ordinary skill in the art may be used (e.g. "Ph" for phenyl, "Me" for methyl, "Et" for ethyl, "min" for minute or minutes, "h" for hour or hours, and "rt" for room temperature).

Memantine, also known as 1-amino-3,5-dimethyladamantane, is disclosed, U.S. Patent Nos. 4,122,193; 4,273,774; and 5,061,703, the subject matter of which patents is hereby incorporated by reference.

Memantine is a systemically-active noncompetitive NMDA receptor antagonists having moderate affinity for the receptor. It exhibits strong voltage dependent characteristics and fast blocking/unblocking kinetics (see e.g. Görtelmeyer et al., Arzneim-Forsch/Drug Res., 1992, 42:904-913; Winblad et al., Int. J. Geriat. Psychiatry, 1999, 14:135-146; Rogawski, Amino Acids, 2000, 19: 133-49; Danysz et al., Curr. Pharm. Des., 2002, 8:835-43; Jirgensons et. al. Eur. J. Med. Chem., 2000, 35: 555-565).

The term "analog" or "derivative" is used herein in the conventional pharmaceutical sense, to refer to a molecule that structurally resembles a reference molecule, but has been modified in a targeted and controlled manner to replace one or more specific substituents of the reference molecule with an alternate substituent, thereby generating a molecule which is structurally similar to the reference molecule. Synthesis and screening of analogs (e.g., using structural and/or biochemical analysis), to identify slightly modified versions of a known compound which may have improved or biased traits (such as higher potency and/or selectivity at a specific targeted receptor type, greater ability to penetrate blood-brain barriers, fewer side effects, etc.) is a drug design approach that is well known in pharmaceutical chemistry.

In addition, using methods known to those skilled in the art, analogs and derivatives of the compounds of the invention may be created which have improved therapeutic efficacy, i.e., higher potency and/or selectivity at a specific targeted receptor type, either greater or lower ability to penetrate mammalian blood-brain barriers (e.g., either higher or lower blood-brain barrier permeation rate), fewer side effects, etc.

The term "prodrug" is used herein in the conventional pharmaceutical sense, to refer to a molecule which undergoes a transformation *in vivo* (e.g., an enzymatic or chemical transformation) to release an active parent drug. Prodrugs of the compounds of Formula **I** of the present invention may be prepared by chemically modifying a functional group present in the compound of Formula **I** such that the chemically modified compound may undergo a transformation *in vivo* (e.g., enzymatic hydrolysis) to provide the compound of Formula **I**. Examples of functional groups present in the compounds of Formula **I** which may be modified to produce prodrugs include carboxy, hydroxy, amino, and thio groups. Prodrugs of the compounds of Formula **I** of the present invention may be prepared according to conventional techniques which have been described in the art (see, for example, Stella V., et al., Prodrugs: Challenges and Rewards, AAPS Press/Springer, New York, 2007).

The phrase "pharmaceutically acceptable", as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean approved by a regulatory agency of the Federal or a state government or listed in the U.S. Pharmacopeia or other generally recognized pharmacopeia for use in mammals, and more particularly in humans.

Compounds of the present invention may be in the form of pharmaceutically acceptable salts. "Pharmaceutically acceptable salts" refers to those salts which possess the biological effectiveness and properties of the parent compound and which are not biologically or otherwise undesirable. The nature of the salt is not critical, provided that it is non-toxic and does not substantially interfere with the desired pharmacological activity.

It will be appreciated by those skilled in the art that compounds of the invention having a chiral center may exist in and be isolated in optically active and racemic forms. Some compounds may exhibit polymorphism. It is to be understood that the present invention encompasses any racemic, optically active, polymorphic, tautomeric, or stereoisomeric form, or mixture thereof, of a compound of the invention, which possesses the useful properties described herein.

The following **Schemes 1-7** describe the preparation of compounds of Formula **I** of the present invention. All of the starting materials may be prepared by procedures described in these schemes, by procedures well known to one of ordinary skill in organic chemistry, or may be obtained commercially. All of the final compounds of the present invention may be prepared by procedures described in these charts or by procedures analogous thereto, which would be well known to one of ordinary skill in organic chemistry. All of the variables used in **Schemes 1-7** are as defined below or as in the claims. Compounds containing one or more chiral centers may be prepared as racemates or mixtures of various stereoisomers and then separated. However, they also may be prepared by a special enantioselective synthesis. For several of the chiral compounds, the enantiomers differ in pharmacological activity.

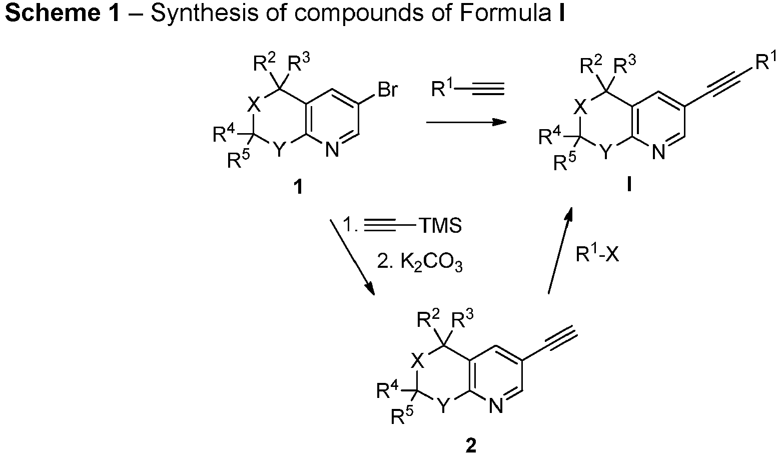

Compounds of general Formula **I** are prepared by Sonogashira coupling of fused bicyclic 3-bromopyridine derivatives **1** with substituted acetylenes in the presence of a suitable catalyst, such as Pd[PPh₃]₂Cl₂. Alternatively, fused bicyclic 3-bromopyridine derivatives **1** can be reacted with trimethylsilylacetylene in the presence of a suitable catalyst, such as Pd[PPh₃]₂Cl₂, to provide, after cleavage of TMS group, terminal acetylenes of general formula **2**. Terminal acetylenes **2** can be reacted with aryl or heteroaryl bromides, iodides or triflates in Sonogashira coupling reactions in the presence of a suitable catalyst, such as Pd[PPh₃]₂Cl₂, to provide compounds of general Formula **I**.

Fused bicyclic 3-bromopyridine derivatives **1** are either commercially available, prepared according to published procedures, or synthesized according to schemes depicted below.

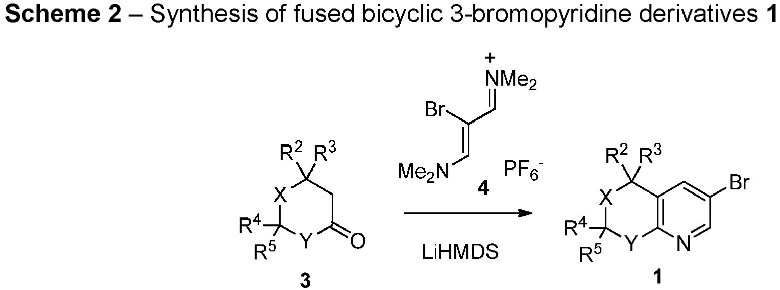

If not commercially available, fused bicyclic 3-bromopyridine derivatives **1** are conveniently prepared, e.g., by treament of ketones **3** with bromovinamidinium reagent **4** in the presence of an appropriate base, such as LiHMDS.

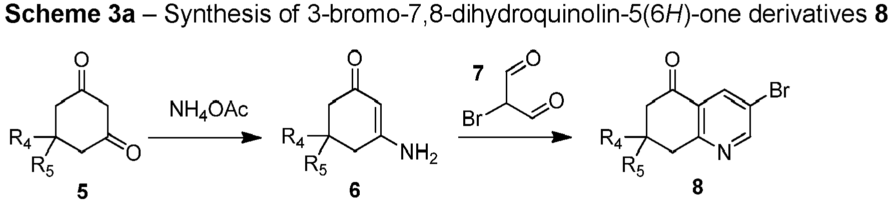

Substituted 7,8-dihydroquinolin-5(6*H*)-ones 8 are synthetically accessible from hexane-1,3-dione derivatives **5** which are reacted with ammonium acetate to form 3-aminocyclohex-2-enone **6.** This intermediate is converted to the desired 7,8-dihydroquinolin-5(6*H*)-one **8** using 2-bromomalonaldehyde **7**.

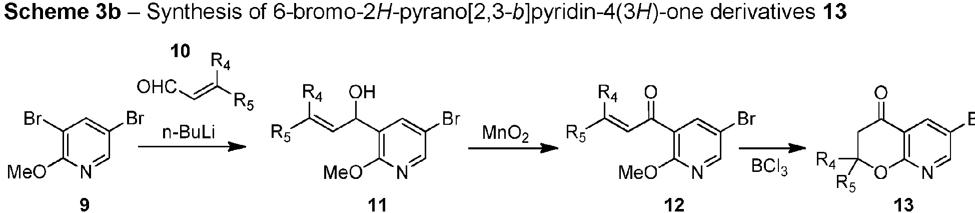

Substituted 2*H*-pyrano[2,3-*b*]pyridin-4(3H)-one **13** may be prepared from 3,5-dibromo-2-methoxypyridine in three steps. Reaction with acrolein derivative **10** leads to the formation of alcohol **11** which is oxidized to the corresponding ketone **12** by means of manganese(IV) oxide. In the last step boron trichloride is used to furnish the desired 2*H*-pyrano[2,3-*b*]pyridin-4(3*H*)-one derivative **13**.

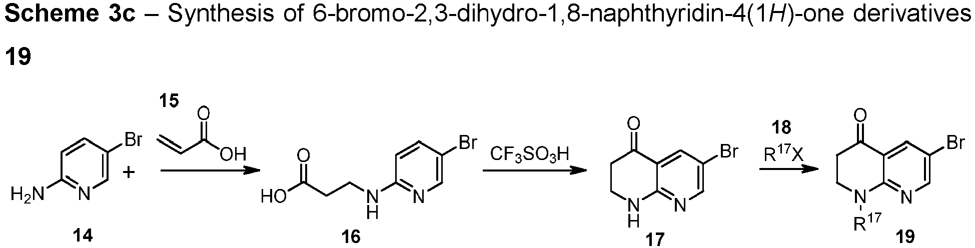

Respective bicyclic 1-aza analogs **19** are synthesized starting from *N-*alkylation of 2-amino-5-bromopyridine **14** which is treated with acrylic acid to form intermediate **16**. Ring formation is accomplished by means of trifluoromethylsulfonic acid which leads to naphthyridine derivative **17** that can be further modified in 1-position by reaction with alkyl halides, acyl halides, chloroformates, sulfonyl or carbamoyl chlorides (**18**) to provide compounds of formula **19**.

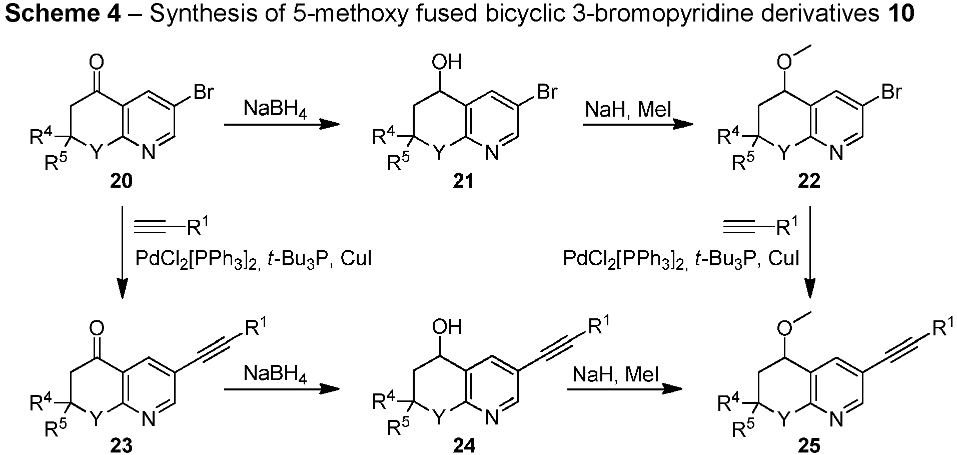

Fused bicyclic 3-bromopyridines **20** are treated with sodium borohydride to provide corresponding alcohols **21**, which are subsequently methylated by means of methyl iodide in the presence of a suitable base, such as sodium hydride. The final product **25** is formed in a Sonogashira coupling reaction using substituted acetylenes, a suitable palladium catalyst (e.g., Pd[PPh₃]₂Cl₂), copper iodide, and tri-*tert-*butylphosphine.

Alternatively, reduction of the carbonyl group and methylation of the alcohol moiety may be performed after Sonogashira coupling towards acetylenes **23** to provide compounds of general formula **25.**

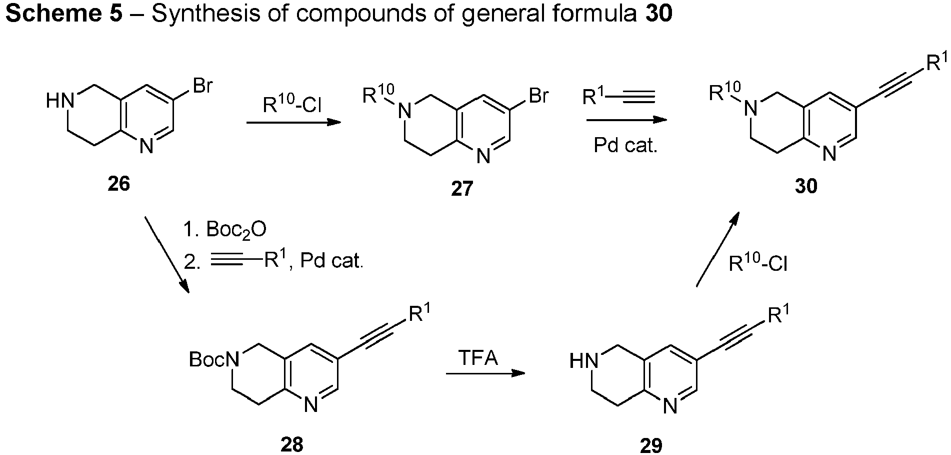

Commercially available 3-bromo-5,6,7,8-tetrahydro-1,6-naphthyridine **26** is acylated using acyl chlorides, chloroformates, sulfonyl or carbamoyl chlorides to provide derivatives **27** which are used for Sonogashira coupling with substituted acetylenes in the presence of a suitable catalyst, such as Pd[PPh₃]₂Cl₂, to yield compounds of general formula **30**.

Alternatively, 3-bromo-5,6,7,8-tetrahydro-1,6-naphthyridine **26** is protected with Boc group and coupled with substituted acetylenes in the presence of a suitable catalyst, such as Pd[PPh₃]₂Cl₂, to provide derivatives **28**. The Boc group is cleaved with trifluoroacetic acid (TFA), and the obtained amines **29** are acylated using acyl chlorides, chloroformates, sulfonyl, or carbamoyl chlorides to provide compounds of general formula **30**.

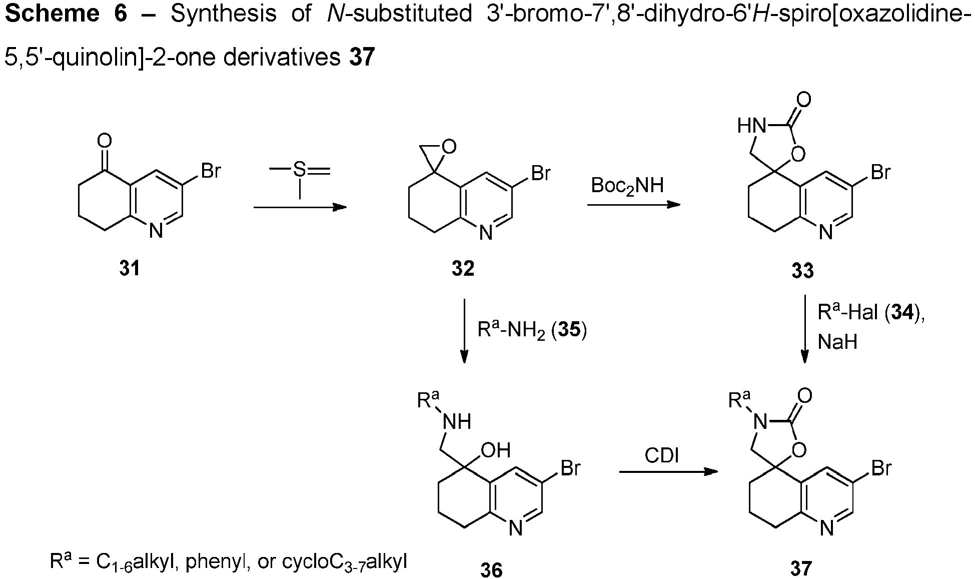

3-Bromo-7,8-dihydroquinolin-5(6*H*)-one **31** is treated with trimethylsulfonium ylide to provide 3'-bromo-7',8'-dihydro-6'*H*-spiro[oxirane-2,5'-quinoline] **32**, treatment of which with Boc₂O in the presence of a base provides 3'-bromo-7',8'-dihydro-6'*H*-spiro[oxazolidine-5,5'-quinolin]-2-one **33.** The free NH group in compound **33** can be alkylated with alkyl halides **34** in the presence of a base, such as NaH, to provide *N*-substituted derivatives **37**.

Alternatively, the epoxide in 3'-bromo-7',8'-dihydro-6'*H*-spiro[oxirane-2,5'-quinoline] **32** can be opened with primary amines **35** to give aminoalcohols **36**, which can be cyclized to *N*-substituted 3'-bromo-7',8'-dihydro-6'*H*-spiro[oxazolidine-5,5'-quinolin]-2-one derivatives **37** by treatment with carbonyldiimidazole (CDI).

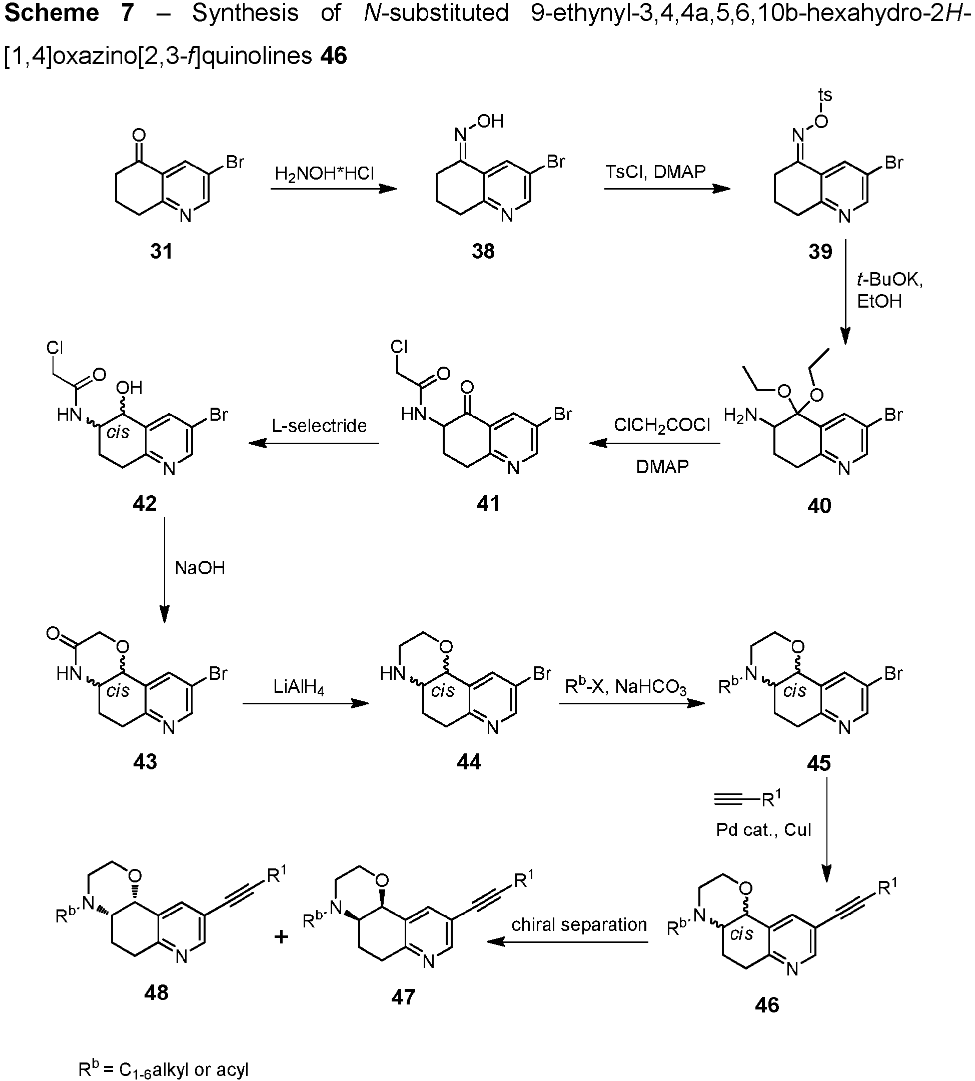

3-Bromo-7,8-dihydroquinolin-5(6*H*)-one **31** is treated with hydroxylamine hydrochloride to form oxime **38** which is subsequently reacted with tosyl chloride towards intermediate product **39**. The *O*-tosyl oxime moiety of **39** is rearranged to α-amino ketal **40** using a strong base, such as *tert*-butoxide. The primary amino group of compound **40** is acylated with chloroacetyl chloride (**41**), and ring formation is accomplished by means of sodium hydroxide after reduction of the carbonyl group using L-selectride to provide tricyclic intermediate **43**. The amide function of **43** is reduced by, e.g., lithium aluminium hydride to furnish [1,4]oxazino[2,3-*f*]quinoline derivative **44**, which can be *N*-derivatized to compounds of general structure **45** by means of alkylating or acylating reagents, such as alkyl iodides or acyl chlorides. In the last step compounds **45** are reacted with substituted acetylenes in a Sonogashira coupling reaction using a suitable catalyst, such as Pd[PPh₃]₂Cl₂, and copper iodide to yield final compounds **46** which may be chirally separated (e.g., by chiral HPLC) to isolate single enantiomers **47** and **48**.

Alternatively, aryl bromide **31** may be directly converted to the corresponding acetylene employing Sonogashira conditions followed by subsequent steps described in **Scheme 7** to provide racemic compounds **46**.

It will be appreciated that in the above transformations it may be necessary or desirable to protect any sensitive groups in the molecule of the compound in question in order to avoid undesirable side reactions.

Pure stereoisomeric forms (including optical isomers) of the compounds and the intermediates of this invention may be obtained by the application of art-known procedures. Diastereomers may be separated by physical separation methods such as selective crystallization and chromatographic techniques, e.g. liquid chromatography using chiral stationary phases. Enantiomers (optically active isomers) may be separated from each other by selective crystallization of their diastereomeric salts with optically active acids. Alternatively, enantiomers may be separated by chromatographic techniques using chiral stationary phases.

Pure stereoisomeric forms may also be derived from the corresponding pure stereoisomeric form of appropriate starting materials, provided that the reaction occur stereoselectively. Stereoisomeric forms of Formula **I** are included within the scope of this invention.

Compounds of Formula **I** which are marked by radioactive atoms may be obtained using art-known procedures. Typical compounds include those where one or more hydrogens are substituted by tritium, where one or more ¹²C are substituted by ¹⁴C where one or more fluor atoms are substituted by ¹⁸F or other isotopes. These may be used for the treatment of diseases (e.g. cancer) but also for diagnostic purposes. The radioactive atoms exchanged in the molecule are often isotopes of carbon, hydrogen, halogen, sulphur or phosphorus. Compounds of the Formula **I** which are marked by radioactive atoms are included within the scope of this invention.

### ADDITION SALTS

For therapeutic use, salts of the compounds of Formula **I** are those wherein the counterion is pharmaceutically acceptable. However, salts of acids and bases, which are non-pharmaceutically acceptable, may also find use, for example, in the preparation and purification of pharmaceutically acceptable compounds. All salts whether pharmaceutically acceptable or not are included within the ambit of the present invention. The pharmaceutically acceptable salts as mentioned above are meant to comprise the therapeutically active non-toxic salt forms, which the compounds of Formula **I** are able to form. The latter may conveniently be obtained by treating the base form with such appropriate acids as inorganic acids, e.g. hydrohalic acids such as hydrochloric, hydrobromic and the like; sulfuric acid; nitric acid; phosphoric acid and the like; or organic acids such as acetic, propanoic, hydroxyacetic, 2-hydroxypropanoic, oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexanesulfonic, 2-hydroxybenzoic, 4-amino-2-hydroxybenzoic and the like acids. Conversely, the salt form may be converted by treatment with alkali into the free base form.

### PHARMACEUTICAL COMPOSITIONS

The active ingredients of the compounds of the invention, together with one or more excipients such as adjuvants, carriers, or diluents, may be placed into the form of pharmaceutical compositions, unit dosages or dosage forms. The pharmaceutical compositions may be employed as solid dosage forms, such as powders, granules, pellets, coated or uncoated tablets or filled capsules, or liquid dosage forms, such as solutions, suspensions, emulsions, or capsules filled with the same, or semi solid dosage forms, such as gels, creams and ointments. The active ingredient(s) dissolution and release profiles of the pharmaceutical dosage forms may be varied from seconds to months.

The pharmaceutical compositions are designed for the use in animals and humans and may be applied via all application routes. Preferred application routes will be the oral route, the dermal route, the pulmonary route, the nasal route, the rectal route, the parenteral route. Such pharmaceutical compositions and unit dosage forms thereof may comprise conventional or new ingredients in conventional or special proportions, with or without additional active compounds or principles, and such unit dosage forms may contain any suitable effective amount of the active ingredient commensurate with the intended daily dosage range to be employed. Tablets containing one (1) to one hundred (100) milligrams of active ingredient or, more broadly, zero point five (0.5) to five hundred (500) milligrams per tablet, are accordingly suitable representative unit dosage forms.

The term "carrier" applied to pharmaceutical compositions of the invention refers to a diluent, excipient, or vehicle with which an active compound is administered. Such pharmaceutical carriers may be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. A.R. Gennaro, 20th Edition, describes suitable pharmaceutical carriers in "Remington: The Science and Practice of Pharmacy".

### METHOD OF TREATING

Due to their high degree of activity and their low toxicity, together presenting a most favorable therapeutic index, the active principles of the invention may be administered to a subject, e.g., a living animal (including a human) body, in need thereof, for the treatment, alleviation, or amelioration, palliation, or elimination of an indication or condition which is susceptible thereto, or representatively of an indication or condition set forth elsewhere in this application, preferably concurrently, simultaneously, or together with one or more pharmaceutically-acceptable excipients, carriers, or diluents, especially and preferably in the form of a pharmaceutical composition thereof, whether by oral, rectal, or parental (including intravenous and subcutaneous) or in some cases even topical route, in an effective amount. Suitable dosage ranges are 1-1000 mg daily, optionally 10-500 mg daily, and optionally 50-500 mg daily, depending as usual upon the exact mode of administration, form in which administered, the indication toward which the administration is directed, the subject involved and the body weight of the subject involved, and the preference and experience of the physician or veterinarian in charge.

The term "treat" is used herein to mean to relieve or alleviate at least one symptom of a disease in a subject. Within the meaning of the present invention, the term "treat" also denotes to arrest, delay the onset (i.e., the period prior to clinical manifestation of a disease) and/or reduce the risk of developing or worsening a disease.

The term "combination" is used herein to define a single pharmaceutical composition (formulation) comprising a compound of the present invention and a second active ingredient (e.g., an NMDA receptor antagonist, L-DOPA, a dopaminomimetic, or a neuroleptic), in a formulation known in the art, or two separate pharmaceutical compositions (formulations), one comprising a compound of the present invention as formulated above and one comprising a second active ingredient (e.g., an NMDA receptor antagonist, L-DOPA, a dopaminomimetic, or a neuroleptic) in a formulation known in the art, to be administered conjointly.

Within the meaning of the present invention, the term "conjoint administration" is used to refer to administration of a compound of the present invention and a second active ingredient (e.g., an NMDA receptor antagonist, L-DOPA, a dopaminomimetic, or a neuroleptic) in one composition, or simultaneously in different compositions, or sequentially. For the sequential administration to be considered "conjoint", however, the compound of the present invention and the NMDA receptor antagonist must be administered separated by a time interval that still permits the resultant beneficial effect in a mammal. For example, the compound of the present invention and the NMDA receptor antagonist must be administered on the same day (e.g., each - once or twice daily), including within an hour of each other, and including simultaneously.

The term "therapeutically effective" applied to dose or amount refers to that quantity of a compound or pharmaceutical composition that is sufficient to result in a desired activity upon administration to a living animal body in need thereof.

Compounds of the present invention may be administered orally, topically, parenterally, or mucosally (e.g., buccally, by inhalation, or rectally) in dosage unit formulations containing conventional non-toxic pharmaceutically acceptable carriers. It is usually desirable to use the oral route. The active agents may be administered orally in the form of a capsule, a tablet, or the like (see Remington: The Science and Practice of Pharmacy, 20th Edition). The orally administered pharmaceutical compositions may be administered in the form of a time-controlled release vehicle, including diffusion-controlled systems, osmotic devices, dissolution-controlled matrices, and erodible/degradable matrices.

For oral administration in the form of a tablet or capsule, the active drug component of Formula **I** may be combined with non-toxic, pharmaceutically acceptable excipients such as binding agents (e.g., pregelatinized maize starch, polyvinylpyrrolidone or hydroxypropyl methylcellulose); fillers (e.g., lactose, sucrose, glucose, mannitol, sorbitol and other reducing and non-reducing sugars, microcrystalline cellulose, calcium sulfate, or calcium hydrogen phosphate); lubricants (e.g., magnesium stearate, talc, or silica, steric acid, sodium stearyl fumarate, glyceryl behenate, calcium stearate, and the like); disintegrants (e.g., potato starch or sodium starch glycolate); and/or wetting agents (e.g., sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like. For oral administration in liquid form, the drug components may be combined with non-toxic, pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), non-aqueous vehicles (e.g., almond oil, oily esters, ethyl alcohol or fractionated vegetable oils), preservatives (e.g., methyl or propyl-p-hydroxybenzoates or sorbic acid), and the like. Stabilizing agents such as antioxidants (BHA, BHT, propyl gallate, sodium ascorbate, citric acid) may also be added to stabilize the dosage forms.

Tablets may be coated by methods well known in the art. Compositions of the invention containing as active compound a compound of Formula I may be also introduced in beads, microspheres or microcapsules, e.g., fabricated from polyglycolic acid/lactic acid (PGLA). Liquid preparations for oral administration may take the form of, for example, solutions, syrups, emulsions or suspensions, or they may be presented as a dry product for reconstitution with water or other suitable vehicle before use. Preparations for oral administration may be suitably formulated to give controlled or postponed release of the active compound.

Compounds of the present invention may also be administered in the form of liposome delivery systems, such as small unilamellar vesicles, large unilamellar vesicles and multilamellar vesicles. Liposomes may be formed from a variety of phospholipids, such as cholesterol, stearylamine or phosphatidylcholines, as is well known.

Compounds of the present invention may also be delivered by the use of monoclonal antibodies as individual carriers to which the compound molecules are coupled. Active drugs may also be coupled with soluble polymers as targetable drug carriers. Such polymers include polyvinyl-pyrrolidone, pyran copolymer, polyhydroxy-propyl methacrylamide-phenol, polyhydroxy-ethyl-aspartamide-phenol, or polyethyleneoxide-polylysine substituted with palmitoyl residues. Furthermore, active drug may be coupled to a class of biodegradable polymers useful in achieving controlled release of a drug, for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

For administration by inhalation, the therapeutics according to the present invention containing as active compound a compound of Formula I may be conveniently delivered in the form of an aerosol spray presentation from pressurized packs or a nebulizer, with the use of a suitable propellant, e.g., dichlorodifluoromethane, trichlorofluoromethane, dichlorotetra-fluoroethane, carbon dioxide, or other suitable gas. In the case of a pressurized aerosol, the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch.

Formulations comprising compounds of the present invention may be delivered parenterally, i.e., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection may be presented in unit dosage form, e.g., in ampoules or in multi-dose containers, with an added preservative. The compositions may take such forms as excipients, suspensions, solutions, or emulsions in oily or aqueous vehicles, and may contain formulatory agents such as suspending, stabilizing and/or dispersing agents. Alternatively, the active ingredient may be in powder form for reconstitution with a suitable vehicle, e.g., sterile pyrogen-free water, before use.

Compounds of the present invention may also be formulated for rectal administration, e.g., as suppositories or retention enemas (e.g., containing conventional suppository bases such as cocoa butter or other glycerides).

Compositions containing a compound of Formula **I** may, if desired, be presented in a pack or dispenser device, which may contain one or more unit dosage forms containing the active ingredient and/or may contain different dosage levels to facilitate dosage titration. The pack may, for example, comprise metal or plastic foil, such as a blister pack. The pack or dispenser device may be accompanied by instructions for administration. Compositions of the invention formulated in a compatible pharmaceutical carrier may also be prepared, placed in an appropriate container, and labeled for treatment of an indicated condition.

As disclosed herein, the dose of the components in the compositions of the present invention is determined to ensure that the dose administered continuously or intermittently will not exceed an amount determined after consideration of the results in test animals and the individual conditions of a patient. A specific dose naturally varies depending on the dosage procedure, the conditions of a patient or a subject animal such as age, body weight, sex, sensitivity, feed, dosage period, drugs used in combination, seriousness of the disease. The appropriate dose and dosage times under certain conditions may be determined by the test based on the above-described indices but may be refined and ultimately decided according to the judgment of the practitioner and each patient's circumstances (age, general condition, severity of symptoms, sex, etc.) according to standard clinical techniques.

Toxicity and therapeutic efficacy of the compositions of the invention may be determined by standard pharmaceutical procedures in experimental animals, e.g., by determining the LD₅₀ (the dose lethal to 50% of the population) and the ED₅₀ (the dose therapeutically effective in 50% of the population). The dose ratio between therapeutic and toxic effects is the therapeutic index and it may be expressed as the ratio LD₅₀/ED₅₀. Compositions that exhibit large therapeutic indices are preferred.

From the Examples described herein below, it is apparent that the present invention provides novel and valuable applications and uses of the compounds of the present invention, which compounds comprise the active principle according to the present invention, as well as novel pharmaceutical compositions thereof and methods of preparation thereof and of treating therewith.

The high order of activity of the active agent of the present invention and compositions thereof, as evidenced by the tests reported, is indicative of utility based on its valuable activity in human beings as well as in lower animals. Clinical evaluation in human beings has not been completed. It will be clearly understood that the distribution and marketing of any compound or composition falling within the scope of the present invention for use in human beings will of course have to be predicated upon prior approval by governmental agencies which are responsible for and authorized to pass judgment on such questions.

The instant compounds of Formula **I** represent a novel class of mGluR5 modulators. In view of their potency, they will be useful therapeutics in a wide range of disorders, in particular CNS disorders, which involve excessive glutamate induced excitation.

These compounds accordingly find application in the treatment of the disorders of a living animal body, especially a human, as listed earlier in the description.

These compounds also find application in the treatment of indications in a living animal body, especially a human, wherein a particular condition does not necessarily exist but wherein a particular physiological parameter may be improved through administration of the instant compounds, including cognitive enhancement.

Neuroprotection as well as cognitive enhancement may also be achieved by administration of the instant compounds in combination with a NMDA receptor antagonist like Memantine.

The method-of-treating a living animal body with a compound of the invention, for the inhibition of progression or alleviation of the selected ailment therein, is as previously stated by any normally-accepted pharmaceutical route, employing the selected dosage which is effective in the alleviation of the particular ailment desired to be alleviated. Use of the compounds of the present invention in the manufacture of a medicament for the treatment of a living animal for inhibition of progression or alleviation of selected ailments or conditions, particularly ailments or conditions susceptible to treatment with a Group I mGluR modulator is carried out in the usual manner comprising the step of admixing an effective amount of a compound of the invention with a pharmaceutically-acceptable diluent, excipient, or carrier, and the method-of-treating, pharmaceutical compositions, and use of a compound of the present invention in the manufacture of a medicament.

Representative pharmaceutical compositions prepared by admixing the active ingredient with a suitable pharmaceutically-acceptable excipient, diluent, or carrier, include tablets, capsules, solutions for injection, liquid oral formulations, aerosol formulations, TDS formulations, and nanoparticle formulations, thus to produce medicaments for oral, injectable, or dermal use, also in accord with the foregoing.

### EXPERIMENTAL PART

The compounds and their preparation of the present invention will be better understood in connection with the following examples, which are intended as an illustration of and not a limitation upon the scope of the invention.

Hereinafter, "MeCN" is defined as acetonitrile, "CDI" as 1,1'-carbonyldiimidazole, "DBU" as 1,8-diazabicyclo[5.4.0]undec-7-ene, "DCM" as dichloromethane, "DME" as 1,2-dimethoxyethane, "DMF" as *N,N*-dimethylformamide, "EtOAc" as ethyl acetate, "LiHMDS" as lithium bis(trimethylsilyl)amide, "MeOH" as methanol, "TEA" as triethylamine, "TFA" as trifluoroacetic acid, and "THF" as tetrahydrofuran.

### Preparation 1

### 3-Bromo-7,8-dihydroquinolin-5(6H)-one

A mixture of cyclohexane-1,3-dione (30.00 g, 267.6 mmol), ammonium acetate (44.49 g, 535.1 mmol), glacial acetic acid (one drop), and benzene (150 mL) is stirred at reflux under Dean-Stark conditions for 7 h, cooled down to rt, and concentrated at reduced pressure. To the obtained residue EtOH and NaHC0₃ (2 eq.) are added. The formed precipitate is collected by filtration. The filtrate is concentrated, treated with dioxane and filtered. The solid on filter is washed with dioxane, and the combined filtrates are diluted with EtOAc. The formed solid is collected by filtration and dried to yield 3-aminocyclohex-2-enone (16.04 g, 54%) as a white solid.

A solution of 3-aminocyclohex-2-enone (798 mg, 7.18 mmol) and 49% aqueous HBr solution (0.79 mL, 7.17 mmol) in EtOH (17 mL) is concentrated, the obtained residue is washed with MeCN and dried to give 3-aminocyclohex-2-enone hydrobromide (582 mg, 42%) as a brownish solid.

A mixture of 3-aminocyclohex-2-enone hydrobromide (5.87 g, 30.5 mmol) and 2-bromomalonaldehyde (6.04 g, 40.0 mmol) in EtOH (100 mL) is stirred at reflux for 9 h. Then the solution is cooled down to rt and concentrated at reduced pressure. The obtained residue is treated with 1 N aqueous NaOH solution and extracted with hexane. The organic phases are concentrated, and the obtained residue is purified by column chromatography (silica gel, EtOAc/hexane, 1:7) to provide the title compound (2.20 g, 32%) as a yellowish oil.

3-Bromo-7,7-dimethyl-7,8-dihydroquinolin-5(6*H*)-one is prepared in close analogy to the procedure described above starting with 5,5-dimethylcyclohexane-1,3-dione.

### Preparation 2

### 3-Bromo-5-methoxy-5,6,7,8-tetrahydroquinoline

A mixture of 3-bromo-7,8-dihydroquinolin-5(6*H*)-one (2.18 g, 9.6 mmol), NaBH₄ (0.73 g, 19.3 mmol) and 2-propanol (25 mL) is stirred at rt for 1.5 h and concentrated at reduced pressure. The obtained residue is purified by column chromatography (silica gel, EtOAc/hexane) to yield the intermediate 3-bromo-5,6,7,8-tetrahydroquinolin-5-ol (2.17 g, 99%) as a colorless oil.

To a solution of 3-bromo-5,6,7,8-tetrahydroquinolin-5-ol (2.17 g, 9.5 mmol) in anhydrous THF (17 mL) NaH (60% suspension in oil, 420 mg, 10.5 mmol) is added portionwise, and the resulting mixture is stirred at rt for 30 min. Then methyl iodide (0.71 mL, 11.4 mmol) is added, and the reaction mixture is stirred for 17.5 h. An additional portion of sodium hydride (60% suspension in oil, 105 mg, 2.6 mmol) is added. The mixture is stirred at rt for 30 min, then additional methyl iodide (0.18 mL, 2.9 mmol) is added. The reaction mixture is stirred at rt for 1 h, poured into water and extracted with EtOAc. The combined organic phases are concentrated at reduced pressure. The obtained residue is purified by column chromatography (silica gel, EtOAc/hexane) to provide the title compound (1.85 g, 80%) as an oil.

3-Bromo-5-methoxy-7,7-dimethyl-5,6,7,8-tetrahydroquinoline is prepared in close analogy to the procedure described above starting with 3-bromo-7,7-dimethyl-7,8-dihydroquinolin-5(6*H*)-one.

### General Procedure 1 - Sonogashira coupling of fused bicyclic 3-bromopyridine derivatives with substituted acetylenes

A fused bicyclic 3-bromopyridine derivative (5.75 mmol, 1.0 eq), optionally substituted acetylene (8.62 mmol, 1.5 eq), copper(**I**)iodide (54 mg, 0.29 mmol, 0.05 eq), and, optionally, triphenylphosphine (45 mg, 0.17 mmol, 0.03 eq) are dissolved in 25 mL of anhydrous DMF. The mixture is flushed thoroughly with argon. PdCl₂(Ph₃P)₂ (121 mg, 0.17 mmol, 0.03 eq) and 3 mL of TEA are added, and the reaction mixture is stirred at 60 °C for 4 h (if not otherwise stated). The reaction mixture is then diluted with water and extracted with chloroform. The organic extracts are combined, dried over anhydrous Na₂SO₄, and the solvent is evaporated. The residue is purified by flash column chromatography or preparative HPLC to give the title compound.

### Preparation 3

### 3-(Phenylethynyl)-5,6,7,8-tetrahydro-1,6-naphthyridine

3-Bromo-5,6,7,8-tetrahydro-1,6-naphthyridine (600 mg, 2.82 mmol) is dissolved in DCM (15 mL), Boc₂O (676 mg, 3.10 mmol) and TEA (570 mg, 5.63 mmol) are added and the mixture is stirred at rt for 1 h. The reaction mixture is washed with KHSO₄ (5% aq.), the organic phase is separated, dried over Na₂SO₄, and concentrated to give sufficiently pure *N*-Boc protected intermediate (832 mg, 94%) as a brown oil. The *N*-Boc protected 3-(phenylethynyl)-5,6,7,8-tetrahydro-1,6-naphthyridine (500 mg, 1.60 mmol), PdCl₂(Ph₃P)₂ (112 mg, 0.16 mmol), Cul (30 mg, 0.16 mmol), and ethynylbenzene (244 mg, 2.39 mmol) are dissolved in dry DMF (7 mL) under argon. Then dry TEA (807 mg 7.98 mmol) is added, and the reaction mixture is heated at 80 °C for 3 h. The reaction mixture is concentrated, diluted with DCM, washed with NaHCO₃ solution, the organic phase is dried over Na₂SO₄ and concentrated *in vacuo.* The residue is purified by flash column chromatography on silica gel to give *N*-Boc protected title compound (510 mg, 95%). This material is dissolved in DCM (10 mL), the solution is cooled to 0 °C, and TFA (1 mL) is added. The mixture is allowed to warm to rt and stirred for 3 h, then evaporated to dryness. The residue is partitioned between EtOAc and NaHCO₃ solution, and the organic phase is separated, dried over Na₂SO₄ and concentrated *in vacuo* to give the title compound (343 mg, 96%).

### General Procedure 2 - N-Acylation of 3-substituted 5,6,7,8-tetrahydro-1,6-naphthyridine

An acyl chloride, chloroformate, sulfonyl or carbamoyl chloride (0.3 mmol) is slowly added to a solution of 3-(phenylethynyl)-5,6,7,8-tetrahydro-1,6-naphthyridine (35 mg, 0.15 mmol) or 3-bromo-5,6,7,8-tetrahydro-1,6-naphthyridine (32 mg, 0.15 mmol) and TEA (46 mg, 0.45 mmol) in dry DCM (2 mL). The reaction mixture is stirred at rt for 3 to 5 h, then diluted with NaHCO₃ solution, the organic phase is separated, dried over Na₂SO₄ and concentrated in vacuo. The residue is purified by flash column chromatography on silica gel or preparative HPLC to provide title compound.

### Preparation 4

### 3'-Bromo-7',8'-dihydro-6'H-spiro[oxirane-2,5'-quinoline]

Trimethylsulfonium iodide (1.354 g, 6.63 mmol) is suspended in 15 mL THF, cooled to 0 °C, potassium *tert*-butylate (0.645 g, 5.76 mmol) solution in 15 mL THF is added, and the mixture is stirred at 0 °C for 10 min. Then a solution of 3-bromo-7,8-dihydroquinolin-5(6*H*)-one (1.0 g, 4.42 mmol) in THF (15 mL) is added dropwise. The reaction mixture is stirred for additional 30 min at 0 °C, then allowed to warm to rt and stirred overnight. Water is added, and the mixture is extracted with EtOAc. The organic layer is washed with brine, dried over anhydrous Na₂SO₄, filtered and evaporated. The residue is purified by flash column chromatography to give the title compound (680 mg, 64%).

### General Procedure 3 - Epoxide ring opening of 3'-bromo-7',8'-dihydro-6'H-spiro[oxirane-2,5'-quinoline] with primary amines and cyclization of the resulting amino alcohols to N-substituted 3'-bromo-7',8'-dihydro-6'H-spiro[oxazolidine-5,5'-quinolin]-2-one derivatives

A primary amine (13.75 mmol) is added to a solution of 3'-bromo-7',8'-dihydro-6'*H*-spiro[oxirane-2,5'-quinoline] (660 mg, 2.75 mmol) in EtOH (5 mL), and the reaction mixture is heated in a closed vial to 80 °C for 15 h. The solvent is evaporated, and the residue is dissolved in dry THF (25 mL). CDI (2.23 g, 13.75 mmol) and TEA (1.40 g, 13.75 mmol) are added, and the mixture is stirred at rt for 15 h. Water is then added, and the mixture is extracted with EtOAc. The organic layer is washed with brine, dried over anhydrous Na₂SO₄, filtered and evaporated. The residue is purified by flash column chromatography to provide the title compound.

### Preparation 5

### cis-9-Bromo-3,4,4a,5,6,10b-hexahydro-2H-[1,4]oxazino[2,3-f]quinoline

### 1. 3-Bromo-7,8-dihydroquinolin-5(6H)-one O-tosyl oxime

Sodium acetate (9.02 g, 110 mmol) and a solution of hydroxylamine hydrochloride (6.95 g, 100 mmol) in water (25 mL) are added to a solution of 3-bromo-7,8-dihydroquinolin-5(6*H*)-one (see **Preparation 1**) (20.00 g, 88.5 mmol) in 2-propanol (250 mL). The reaction mixture is stirred at reflux for 5 h. Water (200 mL) is added to the hot reaction mixture. Then the mixture is allowed to cool down to rt at stirring within 30 min. The formed precipitate is collected by filtration, washed with water and hexane and dried to give intermediate 3-bromo-7,8-dihydroquinolin-5(6*H*)-one oxime (18.60 g, 87%) as beige solid.

DMF (50 mL) and TEA (13.13 g, 130 mmol) are added to a suspension of 3-bromo-7,8-dihydroquinolin-5(6*H*)-one oxime (18.60 g, 77.18 mmol) and tosyl chloride (16.21 g, 85.00 mmol) in DCM (300 mL). The mixture is stirred at 40-50 °C for 6 h, allowed to stand at rt for 6 h, diluted with DCM (300 mL), washed with water, dried over Na₂SO₄ and concentrated at reduced pressure. The obtained residue is crystallized from EtOAc/hexane to provide 3-bromo-7,8-dihydroquinolin-5(6*H*)-one *O*-tosyl oxime (29.50 g, 97%) as brownish solid.

### 2. cis-9-Bromo-4,4a,5,6-tetrahydro-2H-[1,4]oxazino[2,3-f]quinolin-3(10bH)-one

3-Bromo-7,8-dihydroquinolin-5(6*H*)-one *O*-tosyl oxime (29.50 g, 74.63 mmol) is added to a solution of potassium *tert*-butylate (10.10 g, 90.00 mmol) in EtOH (abs., 70 mL) and benzene (200 mL) at 0 °C. Then the mixture is stirred at rt for 3 h. The formed precipitate of potassium tosylate is filtered off and washed with EtOAc (50 mL). The combined filtrate is treated with a solution of HCl in dioxane (18%, 100 mL) and concentrated aqueous HCl solution (10 mL). The resulting mixture is concentrated to dryness. The obtained residue is crystallized from EtOH/EtOAc to give a mixture of hydrochloride salts of 3-bromo-5,5-diethoxy-5,6,7,8-tetrahydroquinolin-6-amine and 6-amino-3-bromo-7,8-dihydroquinolin-5(6*H*)-one (27.50 g, 99%) as brownish solid (ratio 2:1 according to LCMS analysis).

To the obtained mixture (27.50 g, 74.63 mmol) suspended in DCM (500 mL) a solution of NaOH (24.00 g, 600 mmol) in water (90 mL) is added at rt. Then chloroacetyl chloride (18.52 g, 164 mmol) is added dropwise to the formed mixture at 0 °C. The resulting mixture is stirred at rt for 1 h. The organic phase is separated, and the aqueous phase is extracted with DCM (3x200 mL). The combined organic phases are dried over Na₂SO₄ and concentrated at reduced pressure. The obtained beige solid is dissolved in acetone (300 mL), diluted with water (100 mL) and treated with concentrated aqueous HCl solution (30 mL). The mixture is stirred at rt for 12 h and neutralized with an aqueous NaOH solution (10%). Acetone is removed at reduced pressure. The formed precipitate is collected by filtration, washed with water and crystallized from EtOAc/hexane to give *N*-(3-bromo-5-oxo-5,6,7,8-tetrahydroquinolin-6-yl)-2-chloroacetamide (8.69 g, 37%) as brownish solid.

A solution of L-selectride in THF (1M, 35.64 mL, 35.64 mmol) is added dropwise to a solution of *N*-(3-bromo-5-oxo-5,6,7,8-tetrahydroquinolin-6-yl)-2-chloroacetamide (8.69 g, 27.41 mmol) in dry THF (400 mL) under argon atmosphere at -60 °C. The formed mixture is stirred at the same temperature for 40 min, quenched with water (10 mL), warmed up to rt, concentrated at reduced pressure, diluted with DCM (700 mL), washed with water, dried over Na₂SO₄ and concentrated at reduced pressure. The obtained residue is purified by column chromatography (silica gel, DCM/EtOH, 30:1) and crystallized from EtOAc/hexane to yield racemic cis *N*-(3-bromo-5-hydroxy-5,6,7,8-tetrahydroquinolin-6-yl)-2-chloroacetamide (4.20 g, 48%) as brown solid.

A solution of NaOH (1.20 g, 30.00 mmol) in water (15 mL) is added to a solution of racemic *cis N*-(3-bromo-5-hydroxy-5,6,7,8-tetrahydroquinolin-6-yl)-2-chloroacetamide (4.20 g, 13.14 mmol) in 2-propanol (200 mL). The resulting mixture is stirred at rt for 16 h, concentrated at reduced pressure, diluted with DCM (300 mL), washed with water (150 mL), dried over Na₂SO₄ and concentrated at reduced pressure. The residue is crystallized from EtOAc/hexane to provide cis-9-bromo-4,4a,5,6-tetrahydro-2*H-*[1,4]oxazino[2,3-*f*]quinolin-3(10b*H*)-one (2.40 g, 65%) as beige solid. An additional amount may be isolated from the mother liquid by column chromatography (silica gel, DCM/EtOH, 30:1).

LiAlH₄ (570 mg, 15.40 mmol) is added portionwise to a suspension of cis-9-bromo-4,4a,5,6-tetrahydro-2*H*-[1,4]oxazino[2,3-f]quinolin-3(10b*H*)-one (2.40 g, 8.48 mmol) in dry THF (150 mL), and the resulting mixture is stirred at rt for 2 h. Then EtOAc (5 mL), EtOH (2 mL) and water (2 mL) are added to the mixture carefully. The reaction mixture is stirred for 30 min. The formed precipitate is filtered off and washed with DCM. The filtrate is concentrated at reduced pressure. The obtained residue is purified by column chromatography (silica gel, DCM/EtOH, 20:1). The resulting brown oil is diluted with MeCN (50 mL). The insoluble by-product is filtered off and washed with cold MeCN (10 mL). The filtrate is concentrated at reduced pressure and the residue is dried to yield the title compound (800 mg, 35%) as brown oil.

### Preparation 6

### cis-9-(Phenylethynyl)-3,4,4a,5,6,10b-hexahydro-2H-[1,4]oxazino[2,3-f]quinoline

The title compound is prepared in close analogy to the procedure described in **Preparation 5** starting from 3-(phenylethynyl)-7,8-dihydroquinolin-5(6*H*)-one which is synthesized according to the procedure described below.

A mixture of 3-bromo-7,8-dihydroquinolin-5(6*H*)-one (100.00 g, 0.442 mol, synthesis described in **Preparation 1**), K₂CO₃ (61.06 g, 0.442 mol), Pd(OAc)₂ (2.96 g, 13.2 mmol), and Ph₃P (13.65 g, 52.0 mmol) in degassed MeCN (850 mL) is stirred under argon atmosphere at rt for 15-20 min. Then ethynylbenzene (59.16 g, 0.580 mol) is added. The reaction mixture is stirred at reflux under argon atmosphere for 4 h. The formed precipitate is collected by filtration and washed with DCM. The filtrate is concentrated at reduced pressure. The obtained residue is purified by column chromatography (silica gel, DCM) to give the intermediate product 3-(phenylethynyl)-7,8-dihydroquinolin-5(6*H*)-one (85.14 g, 78%) as a brown solid.

### General Procedure 4 - N-derivatisation of 3,4,4a,5,6,10b-hexahydro-2H-[1,4]oxazino[2,3-f]quinolines

9-Substituted 3,4,4a,5,6,10b-hexahydro-2*H*-[1,4]oxazino[2,3-flquinolines are *N-*derivatized by means of an appropriate alkylating or acylating reagent, such as alkyl halogenides or carboxylic acid chlorides, in the presence of a suitable base, such as K₂CO₃ or TEA (Nozulak et al., J. Med. Chem. 1992, 35, 480-489), or by reductive amination methods using an appropriate aldehyde or ketone in the presence of NaBH(OAc)₃ (Abdel-Magid et al., J. Org. Chem. 1996, 61, 3849-3862).

### General Procedure 5 - Synthesis of fused bicyclic 3-bromopyridine derivatives by means of vinamidinium salts

To a solution of an appropriate ketone (1 eq.) in dry THF is added LiHMDS (1 M in THF, 1.05 eq) at -8 °C. The reaction mixture is stirred while warming to rt for 60 min. The solution is cooled to -78 °C and N-(2-bromo-3-(dimethylamino)allylidene)-*N-*methylmethanaminium hexafluorophosphate (1.02 eq) dissolved in dry DMF is added dropwise. The reaction mixture is stirred at -78 °C for 20 min, after which at -78 °C AcOH (1.5 eq) is added, followed by NH₄OAc (2.8 eq). The reaction mixture is allowed to reach rt, and the solution is stirred at 80 °C for 18 h. Then the mixture is allowed to cool to rt and partitioned between Et₂O and a saturated aqueous NaHCO₃ solution (1:1). The layers are separated, and the aqueous layer is extracted with additional Et₂O. The combined organic layers are washed with brine (2x), dried over Na₂SO₄ and concentrated to give the crude product which is further purified by means of column chromatography or preparative HPLC, if necessary, to provide the final product.

### Example 1

### (rac)-5-Methoxy-3-(phenylethynyl)-5,6,7,8-tetrahydroquinoline

According to General procedure 1, 3-bromo-7,8-dihydroquinolin-5(6*H*)-one (see **Preparation 1**) is reacted with ethynylbenzene in the presence of PdCl₂[PPh₃]₂, Cul and TEA in DMF at ambient temperature to provide the intermediate product 3-(phenylethynyl)-7,8-dihydroquinolin-5(6*H*)-one.

NaBH₄ (45 mg, 1.20 mmol) is added to a solution of 3-(phenylethynyl)-7,8-dihydroquinolin-5(6*H*)-one (300 mg, 1.20 mmol) in a mixture of MeOH (1 mL) and THF (5 mL) at -5 °C. The resulting mixture is stirred at 0 °C for 1 h, poured into aqueous solution of potassium carbonate and extracted with DCM (2x50 mL). The combined organic phases are dried over sodium sulfate and concentrated. The obtained residue is purified by column chromatography (silica gel, ethyl acetate/hexane), triturated with hexane and dried to give intermediate product 3-(phenylethynyl)-5,6,7,8-tetrahydroquinolin-5-ol (160 mg, 53%) as white solid.

NaH (60% in mineral oil, 80 mg, 2.20 mmol) is added to a solution of 3-(phenylethynyl)-5,6,7,8-tetrahydroquinolin-5-ol (200 mg, 0.80 mmol) in anhydrous THF (10 mL) at 0 °C, and the resulting mixture is stirred at the same temperature for 15 min. Then methyl iodide (285 mg, 2.00 mmol) is added dropwise, and the reaction mixture is stirred at 0 °C for 1 h and at rt overnight, diluted with water and extracted with DCM (2x40 mL), the combined organic phases are dried over sodium sulfate and concentrated. The obtained residue is purified by column chromatography (silica gel, EtOAc/hexane, 1:4) to provide the title compound (64 mg, 32%) as beige solid.
¹H NMR (DMSO-d₆), δ_{H}, 1.68-2.00 (m, 4H), 2.72-2.94 (m, 2H), 3.37 (s, 3H), 4.32-4.42 (m, 1 H), 7.38-7.48 (m, 3H), 7.56 (d, 2H), 7.85 (s, 1 H), 8.55 (s, 1 H). LC/MS (M+H)⁺ = 264

### Example 2

### 5-Methoxy-3-(phenylethynyl)-5,6,7,8-tetrahydroquinoline (R or S, abs. configuration unknown)

The title compound is separated from the racemic mixture (*rac*)-5-methoxy-3-(phenylethynyl)-5,6,7,8-tetrahydroquinoline (**Example 1**) by means of chiral HPLC (first-eluting enantiomer, CHIRALPAK® AD-H 5 µm - 250 x 30 mm, mobile phase: MeCN / 2-propanol 95:5). LC/MS (M+H)⁺ = 264

### Example 3

### (rac)-6-((5-Methoxy-5,6,7,8-tetrahydroquinolin-3-yl)ethynyl)pyridin-2-amine

According to General Procedure 1, 3-bromo-5-methoxy-5,6,7,8-tetrahydroquinoline (400 mg, 1.64 mmol) is reacted with 6-ethynylpyridin-2-amine (288 mg, 2.48 mmol) in the presence of PdCl₂[PPh₃]₂ (36 mg, 0.05 mmol), *t*-Bu₃P (40 µL, 0.16 mmol), TEA (460 µL, 3.28 mmol), Cs₂CO₃ (810 mg, 2.48 mmol) in MeCN (4 mL) under argon atmosphere at 80 °C for 6 h. The crude product is purified by column chromatography (silica gel, ethyl acetate/ethanol, 50:1) to provide the title compound (109 mg, 24%) as a yellowish solid.
¹H NMR (DMSO-d₆), δ_{H}, 1.72-1.99 (m, 4H), 2.77-2.96 (m, 2H), 3.38 (s, 3H), 4.36 (s, 1 H), 6.05 (s, 2H), 6.47 (d, 1 H), 6.76 (d, 1 H), 7.39 (t, 1 H), 7.83 (s, 1 H), 8.54 (s, 1 H). LC/MS (M+H)⁺ = 280, 246

### Example 4

### (rac)-5-Methoxy-7,7-dimethyl-3-(phenylethynyl)-5,6,7,8-tetrahydroquinoline

According to General Procedure 1, 3-bromo-5-methoxy-7,7-dimethyl-5,6,7,8-tetrahydroquinoline (200 mg, 0.74 mmol) is reacted with 6-ethynylbenzene (163 µL, 1.48 mmol) in the presence of PdCl₂[PPh₃]₂ (16 mg, 0.02 mmol), Cul (8 mg, 0.04 mmol), t-Bu₃P (18 µL, 0.07 mmol), and TEA (1.50 mL, 10.70 mmol) in anhydrous MeCN (1.5 mL) under argon atmosphere at 80 °C for 1.5 h. The crude product is purified by column chromatography (silica gel, EtOAc/hexane, 1:5) to provide the title compound (117 mg, 54%) as a colorless oil.
¹H NMR (DMSO-d₆), δ_{H}, 1.00 (s, 3H)., 1.15 (s, 3H), 1.58-1.67 (dd, 1 H), 1.99-2.07 (m, 1 H), 2.76 (q, 2H), 3.50 (s, 3H), 4.45 (t, 1 H), 7.25 (d, 3H), 7.53 (d, 2H), 7.93 (s, 1 H), 8.59 (s, 1 H). LC/MS (M+H)⁺ = 292, 260

### Example 5

### (rac)-6-((5-Methoxy-7,7-dimethyl-5,6,7,8-tetrahydroquinolin-3-yl)ethynyl)pyridin-2-amine

According to General Procedure 1, 3-bromo-5-methoxy-7,7-dimethyl-5,6,7,8-tetrahydroquinoline (396 mg, 1.46 mmol) is reacted with 6-ethynylpyridin-2-amine (293 mg, 2.46 mmol) in the presence of PdCl₂[PPh₃]₂ (31 mg, 0.04 mmol), *t*-Bu₃P (36 µL, 0.15 mmol), DBU (24 µL, 0.15 mmol), and Cs₂CO₃ (482 mg, 1.48 mmol) in DMF (5 mL) under argon atmosphere at 200 °C for 1 h. The crude product is purified by column chromatography (silica gel, DCM/ethanol) to provide the title compound (54 mg, 12%) as a white solid.
¹H NMR (DMSO-d₆), δ_{H}, 1.00 (s, 3H)., 1.15 (s, 3H), 1.57-1.66 (dd, 1 H), 1.97-2.06 (m, 1 H), 2.76 (q, 2H), 3.49 (s, 3H), 4.44 (t, 1 H), 4.51 (s, 2H), 6.47(d, 1 H), 6.93 (d, 1 H), 7.42 (t, 1 H), 7.99 (s, 1H), 8.63 (s, 1 H). LC/MS (M+H)⁺ = 308

### Example 6

### (rac)-3-(Imidazo[1,2-a]pyridin-2-ylethynyl)-5-methoxy-7,7-dimethyl-5,6,7,8-tetrahydroquinoline

A mixture of 3-bromo-7,7-dimethyl-7,8-dihydroquinolin-5(6*H*)-one (see **Preparation 1**) (2.00 g, 7.87 mmol), PdCl₂[PPh₃]₂ (0.18 g, 0.25 mmol), Cul (0.05 g, 0.25 mmol), diisopropylamine (5 mL) and MeCN (10 mL) is stirred under argon for 5 min. Ethynyltrimethylsilane (1.54 g, 15.75 mmol) is added, and the resulting mixture is refluxed for 4 h, cooled down to rt, diluted with water and extracted with EtOAc. The organic layer is dried over Na₂SO₄ and concentrated under reduced pressure. To a solution of the crude intermediate in THF (20 mL) 1M solution of TBAF in THF (8.5 mL) is added dropwise at -10 °C. The mixture is stirred at 0 °C for 1 h, diluted with water and extracted with DCM. The organic layer is dried over Na₂SO₄ and concentrated at reduced pressure. The residue is purified by chromatography (silica gel, DCM) to give 3-ethynyl-7,7-dimethyl-7,8-dihydroquinolin-5(6*H*)-one (1.36 g, 87% for two steps) as intermediate product A.

To a solution of 2-bromoimidazo[1,2-*a*]pyridine (14.22 g, 72.18 mmol) in MeCN (300 mL) Nal (28.00 g, 185.55 mmol) and an aqueous HI solution (57% wt, 30 mL) are added. The mixture is stirred at reflux for 6 h, and the second portion of an aqueous HI solution (57% wt, 20 mL) is added. The resulting mixture is stirred at reflux for 6 h, cooled down to rt, diluted with water, neutralized with 10% aqueous NaOH solution, and extracted with DCM (2×300 mL). The combined organic phase is washed with saturated aqueous Na₂S₂O₅ solution, dried over Na₂SO₄ and concentrated at reduced pressure. The obtained residue is crystallized from EtOAc/hexane to give 2-iodoimidazo[1,2-a]pyridine (13.90 g, 79%) as intermediate product B.

According to General Procedure 1, intermediate A (360 mg, 1.80 mmol) is reacted with intermediate B (480 mg, 1.98 mmol) in the presence of PdCl₂[PPh₃]₂ (63 mg, 0.09 mmol), *t*-Bu₃P (18 mg, 0.09 mmol), DBU (27 mg, 0.18 mmol), and TEA (2 mL) in MeCN (10 mL) under argon atmosphere at reflux for 6 h. The crude product is purified by column chromatography (silica gel, hexane/EtOAc) to provide 3-(imidazo[1,2-a]pyridin-2-ylethynyl)-7,7-dimethyl-7,8-dihydroquinolin-5(6*H*)-one (210 mg, 37%) as a yellowish solid, which is converted to the title compound in close analogy to the procedure described in **Example 1** (yields: 95% after reduction, 17% after O-methylation).
¹H NMR (CDCl₃), δ_{H}, 0.99 (s, 3H), 1.14 (s, 3H), 1.62 (dd, 1 H), 2.01 (dd, 1 H), 2.76 (q, 2H), 3.49 (s, 3H), 4.44 (t, 1 H), 6.80 (dd, 1 H), 7.19 (dd, 1 H), 7.57 (d, 1 H), 7.77 (s, 1 H), 7.98 (s, 1 H), 8.07 (d, 1 H), 8.63 (s, 1 H).
LC/MS (M+H)⁺ = 332

### Example 7

### (rac)-6-((4-Methoxy-2,2-dimethyl-3,4-dihydro-2H-pyrano[2,3-b]pyridin-6-yl)ethynyl)pyridin-2-amine

A solution of 3,5-dibromo-2-methoxypyridine (11.4 g, 42.7 mmol) in dry diethyl ether is cooled to -65 °C and treated with BuLi (19 mL 2.5 M solution in hexane, 47.5 mmol) under argon atmosphere. The mixture is stirred at -70 °C for 0.5 h. A solution of 3-methylbut-2-enal (3.44 g, 40.9 mmol) in dry THF (10 mL) is added to the mixture, and stirring is continued for another 0.5 h at -70 °C. Then the reaction mixture is warmed to rt and quenched with a saturated solution of sodium bicarbonate (100 mL). After extraction with DCM (3x100 mL) the combined organic layers are dried over Na₂SO₄ and concentrated under reduced pressure to yield the intermediate 1-(5-bromo-2-methoxypyridin-3-yl)-3-methylbut-2-en-1-ol (11 g, 95%).

MnO₂ (18 g, 207 mmol) is added to a solution of 1-(5-bromo-2-methoxypyridin-3-yl)-3-methylbut-2-en-1-ol (3 g, 11 mmol) in dry DCM (500 mL) in one portion. The mixture is stirred for 3 days and filtered. The filtrate is concentrated under reduced pressure, and the residue is purified by preparative TLC chromatography (SiO₂, eluent - hexane-ethylacetate 4:1) to give the intermediate 1-(5-bromo-2-methoxypyridin-3-yl)-3-methylbut-2-en-1-one (2.4 g, 81 %).

To a solution of 1-(5-bromo-2-methoxypyridin-3-yl)-3-methylbut-2-en-1-one (1.2 g, 4.4 mmol) in dry DCM (50 mL) a mixture of boron trichloride (2 g, 17.1 mmol) in dry DCM (50 mL) is added dropwise at -45 to -50 °C. After the addition the mixture is stirred for 0.5 h at -45 °C, allowed to warm to rt, and stirred for another 0.5 h. Then the reaction mixture is quenched by a saturated aqueous sodium bicarbonate solution. The organic layer is separated, dried over magnesium sulfate, and concentrated in vacuum to obtain 6-bromo-2,2-dimethyl-2*H*-pyrano[2,3-b]pyridin-4(3*H*)-one (0.5 g, 44%) which is converted to 6-bromo-4-methoxy-2,2-dimethyl-3,4-dihydro-2*H*-pyrano[2,3-*b*]pyridine in close analogy to the procedure described in Preparation 1 (yields: 95% after reduction, 80% after *O*-methylation).

According to General Procedure 1, 6-bromo-4-methoxy-2,2-dimethyl-3,4-dihydro-2*H*-pyrano[2,3-b]pyridine (360 mg, 1.32 mmol) is reacted with 6-ethynylpyridin-2-amine (172 mg, 1.45 mmol) in the presence of PdCl₂[PPh₃]₂ (46 mg, 0.07 mmol), t-Bu₃P (13 mg, 0.07 mmol), DBU (20 mg, 0.13 mmol), and TEA (2 mL) in MeCN (10 mL) under argon atmosphere at reflux for 6 h. The crude product is purified by column chromatography (silica gel, hexane/EtOAc) to provide the title compound (40 mg, 10%) as a light beige solid.
¹H NMR (CDCl₃), δ_{H}, 1.38 (s, 3H), 1.50 (s, 3H), 1.91 (dd, 1 H), 2.14 (dd, 1 H), 3.46 (s, 3H), 4.41 (dd, 1 H), 4.56 (s, 2H), 6.44 (d, 1 H), 6.86 (s, 1 H), 7.37 (dd, 1 H), 7.91 (s, 1 H), 8.34 (s, 1 H).
LC/MS (M+H)⁺ = 310

### Example 8

### (rac)- 6-(Imidazo[1,2-a]pyridin-2-ylethynyl)-4-methoxy-2,2-dimethyl-3,4-dihydro-2H-pyrano[2,3-b]pyridine

A mixture of 2-iodoimidazo[1,2-*a*]pyridine (for preparation see **Example 6**, 2.00 g, 8.20 mmol), ethynyltrimethylsilane (1.21 g, 12.30 mmol), PdCl₂[PPh₃]₂ (190 mg, 0.27 mmol), Cul (150 mg, 0.80 mmol) and TEA (3.4 mL, 24.0 mmol) in benzene (15 mL) is stirred at 50 °C under argon atmosphere for 12 h, cooled down to rt and concentrated at reduced pressure. The obtained residue is purified by column chromatography (silica gel, EtOAc/hexane) to yield the intermediate product 2-((trimethylsilyl)ethynyl)imidazo[1,2-*a*]pyridine (1.23 g, 70%) as a light brown oil which is used on the next stage without further purification.

TBAF solution in THF (1M, 0.20 mL, 0.20 mmol) is added slowly to a solution of 2-((trimethylsilyl)ethynyl)imidazo[1,2-a]pyridine (636 mg, 2.90 mmol) in THF (10 mL) at 0 °C. The resulting mixture is stirred at 0 °C for 1 h, diluted with water and extracted with DCM (3×10 mL). The combined organic phases are dried over Na₂SO₄ and concentrated at reduced pressure. The obtained residue is purified by column chromatography (silica gel, EtOAc/hexane) to give 2-ethynylimidazo[1,2-a]pyridine (230 mg, 55%) as a brown oil.

According to General Procedure 1, 6-bromo-4-methoxy-2,2-dimethyl-3,4-dihydro-2*H*-pyrano[2,3-b]pyridine (for preparation see **Example 7**, 160 mg, 0.59 mmol) is reacted with 2-ethynylimidazo[1,2-a]pyridine (93 mg, 0.65 mmol) in the presence of PdCl₂[PPh₃]₂ (20 mg, 0.03 mmol), *t*-Bu₃P (6 mg, 0.03 mmol), DBU (9 mg, 0.6 mmol), and TEA (2 mL) in MeCN (10 mL) under argon atmosphere at reflux for 6 h. The crude product is purified by column chromatography (silica gel, hexane/EtOAc) to provide the title compound (25 mg, 13%) as a light yellow oil.
¹H NMR (CDCl₃), δ_{H}, 1.41 (s, 3H), 1.53 (s, 3H), 1.95 (dd, 1 H), 2.17 (dd, 1 H), 3.49 (s, 3H), 4.45 (t, 1 H), 6.80 (dd, 1 H), 7.19 (dd, 1 H), 7.57 (d, 1 H), 7.75 (s, 1 H), 7.94 (s, 1 H), 8.06 (d, 1H), 8.39 (s, 1 H).
LC/MS (M+H)⁺ = 334

### Example 9

### 4-Methoxy-1-methyl-6-(phenylethynyl)-1,2,3,4-tetrahydro-1,8-naphthyridine

Acrylic acid (8.33 g, 115.6 mmol) is carefully added dropwise to a refluxing solution of 5-bromopyridin-2-amine (10.00 g, 57.80 mmol) in toluene (25 mL). The mixture is stirred at reflux for 3 days, concentrated at reduced pressure and diluted with aqueous KOH (6.47 g, 115.6 mmol) solution (150 mL). The formed solid is filtered, and the filtrate is extracted with DCM (3x). The aqueous phase is acidified to pH 5. The formed solid is collected by filtration, washed with water and dried to provide 3-((5-bromopyridin-2-yl)amino)propanoic acid (12.75 g, 90%) as beige solid.

A mixture of the propanoic acid intermediate (5.50 g, 22.44 mmol) and CF₃SO₃H (50 mL) is stirred at 90 °C for 3 h, cooled down to rt and poured onto crushed ice (300 g). Then KOH is added to reach pH 12. The resulting mixture is extracted with DCM. The organic phase is concentrated at reduced pressure, and the obtained residue is purified by column chromatography (silica gel, EtOAc/hexane, 1:5), recrystallized from EtOH and dried to provide the intermediate product 6-bromo-2,3-dihydro-1,8-naphthyridin-4(1*H*)-one (1.02 g, 20%) as a yellow solid.

t-Bu₃P (51 mg, 0.25 mmol) is added to a solution of 6-bromo-2,3-dihydro-1,8-naphthyridin-4(1*H*)-one (551 mg, 2.43 mmol) and TEA (3.7 mL) in DMF (3.5 mL) under argon atmosphere. The solution is stirred at rt for 5 min. Then PdCl₂[PPh₃]₂ (51 mg, 0.07 mmol) is added, and the resulting mixture is stirred for 10 min. Finally ethynylbenzene (498 mg, 4.88 mmol) is added dropwise, and the reaction mixture is stirred at 75 °C for 1.5 h, cooled down to rt, diluted with water (20 mL), and extracted with EtOAc. The organic phase is dried over Na₂SO₄ and concentrated at reduced pressure. The obtained residue is purified by column chromatography (silica gel, EtOAc/hexane, 1:2) and by preparative HPLC (C18, MeCN/water) to give intermediate compound 6-(phenylethynyl)-2,3-dihydro-1,8-naphthyridin-4(1*H*)-one (486 mg, 80%) as a yellow solid, which is converted to 6-(phenylethynyl)-1,2,3,4-tetrahydro-1,8-naphthyridin-4-ol (140 mg, 73%) in close analogy to the procedure described in **Example 1.**

NaH (60% in oil, 28 mg, 0.70 mmol) is added to a solution of 6-(phenylethynyl)-1,2,3,4-tetrahydro-1,8-naphthyridin-4-ol (130 mg, 0.52 mmol) in DMF (3 mL) under argon atmosphere at 0 °C, and the mixture is stirred at 0 °C for 30 min. Then a solution of Mel (148 mg, 1.04 mmol) in DMF (0.5 mL) is added dropwise, and the reaction mixture is stirred at 0 °C for 30 min and at rt for 2.5 h, diluted with water and extracted with EtOAc. The organic phase is dried over Na₂SO₄ and concentrated at reduced pressure. The obtained residue is purified by column chromatography (silica gel, ethyl acetate/hexane, 1:1) to provide the title compound (54 mg, 37%) as a yellowish oil.
¹H NMR (CDCl₃), δ_{H}, 1.85-1.95 (m, 1 H), 2.05-2.15 (m, 1 H), 3.17 (s, 3H), 3.22-3.32 (m, 1 H), 3.37 (s, 1 H), 3.53-3.63 (m, 1 H), 4.16-4.22 (m, 1 H), 7.25-7.35 (m, 3H), 7.40 (s, 1 H), 7.48 (d, 2H), 8.27 (s, 1 H). LC/MS (M+H)⁺ = 279

### Example 10

### (rac)-3'-(Phenylethynyl)-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with Boc₂NH to provide *tert*-butyl 3'-bromo-2-oxo-7',8'-dihydro-6'*H-*spiro[oxazolidine-5,5'-quinoline]-3-carboxylate, which is coupled with ethynylbenzene according to General Procedure 1. An acidic aqueous workup effects the removal of Boc group, and purification of the crude product by flash column chromatography provides the title compound as a colorless solid.
¹H NMR (CDCl₃), δ_{H}, 1.76-2.00 (m, 2H), 2.14-2.25 (m, 2H), 3.01 (t, 2H), 3.70 (AB m, 2H), 5.60 (s, 1 H), 7.26-7.38 (m, 3H), 7.53-7.57 (m, 2H), 7.96 (d, 1 H), 8.65 (d, 1 H). LC/MS (M+H)⁺ = 305

### Example 11

### (rac)-3-Methyl-3'-(phenylethynyl)-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with methylamine hydrochloride in the presence of TEA to provide 3'-bromo-3-methyl-7',8'-dihydro-6'*H*-spiro[oxazolidine-5,5'-quinolin]-2-one, which is coupled with ethynylbenzene according to General Procedure 1. Purification of the crude product by flash column chromatography provides the title compound as a colorless solid.
¹H NMR (CDCl₃), δ_{H}, 1.77-1.97 (m, 2H), 2.09-2.25 (m, 2H), 2.98-3.02 (m, 5H), 3.59 (AB m, 2H), 7.35-7.38 (m, 3H), 7.52-7.57 (m, 2H), 7.86 (d, 1 H), 8.63 (d, 1 H). LC/MS (M+H)⁺ = 319

### Example 12

### (rac)-3-Phenyl-3'-(phenylethynyl)-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with aniline to provide 3'-bromo-3-phenyl-7',8'-dihydro-6'*H-*spiro[oxazolidine-5,5'-quinolin]-2-one, which is coupled with ethynylbenzene according to General Procedure 1. Purification of the crude product by flash column chromatography provides the title compound as a colorless solid.
¹H NMR (CDCl₃), δ_{H}, 1.82-2.04 (m, 2H), 2.20-2.31 (m, 2H), 3.07 (t, 2H), 4.10 (AB m, 2H), 7.14-7.62 (m, 10H), 7.94 (d, 1H), 8.67 (d, 1H). LC/MS (M+H)⁺ = 381

### Example 13

### (rac)-cis-4-Methyl-9-(phenylethynyl)-3,4,4a,5,6,10b-hexahydro-2H-[1,4]oxazino[2,3-f]quinoline

According to General Procedure 4, *cis*-9-(phenylethynyl)-3,4,4a,5,6,10b-hexahydro-2*H*-[1,4]oxazino[2,3-*f*]quinoline is reacted with formaldehyde in the presence of NaBH(OAc)₃ to provide the title compound as HCl salt.
¹H NMR (CD₃OD), δ_{H}, 2.42-2.88 (m, 2H), 3.05-3.49 (m, 8H), 3.94-4.22 (m, 3H), 5.08-5.40 (2 s, 1 H), 7.41-7.48 (m, 3H), 7.61-7.68 (dd, 2H), 8.68 (s, 1H), 9.00 (s, 1H). LC/MS (M+H)⁺ = 305

### Example 14

### (rac)-cis-4-Ethyl-9-(phenylethynyl)-3,4,4a,5,6,10b-hexahydro-2H-[1,4]oxazino[2,3-f]quinoline

According to General Procedure 4, *cis*-9-(phenylethynyl)-3,4,4a,5,6,10b-hexahydro-2*H*-[1,4]oxazino[2,3-*f*]quinoline is reacted with iodoethane to provide the title compound as HCl salt.
¹H NMR (CD₃OD), δ_{H}, 1.46 (t, 3H), 2.36-2.76 (m, 2H), 3.25-3.57 (m, 7H), 4.02-4.23 (m, 3H), 5.10-5.38 (2 s, 1 H), 7.41-7.50 (m, 3H), 7.60-7.63 (dd, 2H), 8.64 (s, 1 H), 8.98 (s, 1H). LC/MS (M+H)⁺ = 319

### Example 15

### (rac)-cis-9-(Phenylethynyl)-4-propyl-3,4,4a,5,6,10b-hexahydro-2H-[1,4]oxazino[2,3-f]quinoline

According to General Procedure 4, *cis*-9-(phenylethynyl)-3,4,4a,5,6,10b-hexahydro-2*H*-[1,4]oxazino[2,3-*f*]quinoline (2.28 g, 7.85 mmol) is reacted with 1-iodopropane (1.74 g, 10.24 mmol) in the presence of NaHCO₃ (1.32 g, 15.70 mmol) at rt for 24 h. An additional amount of 1-iodopropane (260 mg, 1.53 mmol) is added, and the reaction mixture is stirred for 6 h. Then the mixture is diluted with water and extracted with EtOAc (3x80 mL). The combined organic layers are dried over Na₂SO₄ and concentrated at reduced pressure. The obtained residue is purified by column chromatography (silica gel, ethyl acetate/hexane, 1:3) to provide the title compound (1.26 g, 48%) as yellowish oil.
¹H NMR (DMSO-d₆), δ_{H}, 0.84 (t, 3H), 1.41 (m, 2H), 1.76 (m, 1 H), 2.14 (m, 1 H), 2.33 (m, 2H), 2.38-3.00 (m, 5H), 3.59 (m, 2H), 4.62 (s, 1H), 7.41 (br s, 3H), 7.59 (br s, 2H), 7.83 (s, 1 H), 8.55 (s, 1H). LC/MS (M+H)⁺ = 333

### Example 16

### cis-9-(Phenylethynyl)-4-propyl-3,4,4a,5,6,10b-hexahydro-2H-[1,4]oxazino[2,3-f]quinoline (RS or SR, abs. configuration unknown)

The title compound is separated from the racemic mixture (*rac*)-*cis*-9-(phenylethynyl)-4-propyl-3,4,4a,5,6,10b-hexahydro-2*H*-[1,4]oxazino[2,3-*f*]quinoline (**Example 15**) by means of chiral HPLC (first-eluting enantiomer, CHIRALPAK® IA 5 µm - 250 x 4.6 mm, mobile phase: EtOH / MeOH, diethylamine 50:50:0.1). LC/MS (M+H)⁺ = 333

### Example 17

### cis-9-(Phenylethynyl)-4-propyl-3,4,4a,5,6,10b-hexahydro-2H-[1,4]oxazino[2,3-f]quinoline (RS or SR, enantiomer of Example 16, abs. configuration unknown)

The title compound is separated from the racemic mixture (*rac*)-*cis*-9-(phenylethynyl)-4-propyl-3,4,4a,5,6,10b-hexahydro-2*H*-[1,4]oxazino[2,3-*f*]quinoline (**Example 15**) by means of chiral HPLC (second-eluting enantiomer, CHIRALPAK® IA 5 µm - 250 x 4.6 mm, mobile phase: EtOH / MeOH, diethylamine 50:50:0.1). LC/MS (M+H)⁺ = 333

### Example 18

### (rac)-cis-9-((2-Fluorophenyl)ethynyl)-4-propyl-3,4,4a,5,6,10b-hexahydro-2H-[1,4]oxazino[2,3-f]quinoline

According to General Procedure 4, *cis*-9-bromo-3,4,4a,5,6,10b-hexahydro-2*H-*[1,4]oxazino[2,3-*f*]quinoline (800 mg, 2.97 mmol) is reacted with 1-iodopropane (761 mg, 4.50 mmol) in the presence of NaHCO₃ (504 mg, 6.00 mmol) in DMF (10 mL) at rt for 48 h. The crude product is purified by column chromatography (silica gel, ethyl acetate/hexane, 1:2) to give intermediate *cis*-9-bromo-4-propyl-3,4,4a,5,6,10b-hexahydro-2*H*-[1,4]oxazino[2,3-*f*]quinoline (640 mg, 69%) as yellowish oil.

TEA (0.5 mL), PdCl₂[PPh₃]₂ (11 mg, 0.02 mmol), DBU (8 mg, 0.05 mmol),and *t-*Bu₃P (6 mg, 0.03 mmol) are added to a solution of *cis*-9-bromo-4-propyl-3,4,4a,5,6,10b-hexahydro-2*H*-[1,4]oxazino[2,3-*f*]quinoline (160 mg, 0.51 mmol, 1.0 eq.) in MeCN (3 mL) under argon atmosphere at rt. The mixture is stirred at rt for 5 min. Then 1-ethynyl-2-fluorobenzene (101 mg, 0.84 mmol) and Cul (2 mg, 0.01 mmol) are added, and the reaction mixture is stirred at reflux for 3.5 h, cooled down to rt and concentrated at reduced pressure. The residue is dissolved in EtOAc (15 mL) and hexane (35 mL) and washed with an aqueous HCl solution (5%, 3×30 mL). The aqueous phases are combined, neutralized with KOH and extracted with DCM (3x50 mL). The combined organic layers are dried over Na₂SO₄ and concentrated at reduced pressure. The residue is purified by column chromatography (silica gel, ethyl acetate/hexane, 1:2) to provide the title compound (105 mg, 59%) as a beige solid.
¹H NMR (DMSO-d₆), δ_{H}, 0.87 (t, 3H), 1.37-1.54 (m, 2H), 1.73-1.84 (m, 1 H), 2.11-2.24 (m, 1 H), 2.30-2.41 (m, 2H), 2.54-2.66 (m, 2H), 2.68-2.81 (m, 1 H), 2.86 (dd, 1 H), 2.91-3.04 (m, 1 H), 3.54-3.62 (m, 1 H), 3.63-3.71 (m, 1 H), 4.65 (d, 1 H), 7.25-7.39 (m, 2H), 7.47-7.54 (m, 1 H), 7.64-7.70 (m, 1 H), 7.85 (d, 1 H), 8.59 (d, 1 H). LC/MS (M+H)⁺ = 351

### Example 19

### (rac)-cis-9-((3-Fluorophenyl)ethynyl)-4-propyl-3,4,4a,5,6,10b-hexahydro-2H-[1,4]oxazino[2,3-f]quinoline

The title compound (110 mg, 61 %, yellow oil) is prepared according to the method described in **Example 18** using *cis*-9-bromo-4-propyl-3,4,4a,5,6,10b-hexahydro-2*H*-[1,4]oxazino[2,3-*f*]quinoline (160 mg, 0.51 mmol) in the presence of TEA (0.5 mL), PdCl₂[PPh₃]₂ (11 mg, 0.02 mmol), DBU (8 mg, 0.05 mmol), *t*-Bu₃P (6 mg, 0.03 mmol), 1-ethynyl-3-fluorobenzene (101 mg, 0.84 mmol) and Cul (2 mg, 0.01 mmol) in MeCN (3 mL).
¹H NMR (DMSO-d₆), δ_{H}, 0.87 (t, 3H), 1.38-1.52 (m, 2H), 1.73-1.83 (m, 1 H), 2.11-2.23 (m, 1 H), 2.30-2.40 (m, 2H), 2.54-2.66 (m, 2H), 2.69-2.80 (m, 1 H), 2.85 (dd, 1 H), 2.91-3.02 (m, 1 H), 3.54-3.61 (m, 1 H), 3.62-3.70 (m, 1 H), 4.65 (d, 1 H), 7.30 (dd, 1 H), 7.41-7.55 (m, 3H), 7.86 (s, 1 H), 8.59 (s, 1 H). LC/MS (M+H)⁺ = 351

### Example 20

### (rac)-cis-9-((4-Fluorophenyl)ethynyl)-4-propyl-3,4,4a,5,6,10b-hexahydro-2H-[1,4]oxazino[2,3-f]quinoline

The title compound (69 mg, 39%, yellowish oil) is prepared according to the method described in **Example 18** using *cis*-9-bromo-4-propyl-3,4,4a,5,6,10b-hexahydro-2*H*-[1,4]oxazino[2,3-*f*]quinoline (160 mg, 0.51 mmol) in the presence of TEA (0.5 mL), PdCl₂[PPh₃]₂ (11 mg, 0.02 mmol), DBU (8 mg, 0.05 mmol), *t*-Bu₃P (6 mg, 0.03 mmol), 1-ethynyl-4-fluorobenzene (101 mg, 0.84 mmol) and Cul (2 mg, 0.01 mmol) in MeCN (3 mL).
¹H NMR (DMSO-d₆), δ_{H}, 0.87 (t, 3H), 1.39-1.51 (m, 2H), 1.73-1.83 (m, 1 H), 2.11-2.23 (m, 1 H), 2.31-2.41 (m, 2H), 2.55-2.67 (m, 2H), 2.69-2.78 (m, 1 H), 2.85 (dd, 1 H), 2.91-3.02 (m, 1H), 3.54-3.62 (m, 1 H), 3.63-3.70 (m, 1 H), 4.64 (d, 1 H), 7.29 (dd, 2H), 7.65 (dd, 2H), 7.84 (s, 1H), 8.57 (s, 1 H). LC/MS (M+H)⁺ = 351

### Example 21

### (rac)-cis-4-Propyl-9-(m-tolylethynyl)-3,4,4a,5,6,10b-hexahydro-2H-[1,4]oxazino[2,3-f]quinoline

A mixture of *cis*-9-bromo-4-propyl-3,4,4a,5,6,10b-hexahydro-2*H-*[1,4]oxazino[2,3-*f*]quinoline (160 mg, 0.51 mmol, for preparation see **Example 18**), PdCl₂[PPh₃]₂ (11 mg, 0.02 mmol), Cul (2 mg, 0.01 mmol), and TEA (97 mg, 0.96 mmol) in MeCN (3 mL) is stirred under argon atmosphere at rt for 5 min. Then 1-ethynyl-3-methylbenzene (98 mg, 0.84 mmol) is added. The reaction mixture is stirred at reflux for 5 h, cooled down to rt and concentrated at reduced pressure. The residue is dissolved in EtOAc (10 mL) and hexane (20 mL) and washed with an aqueous HCl solution (5%, 3x30 mL). The aqueous phases are combined, neutralized with KOH and extracted with DCM (3x30 mL). The combined organic layers are dried over Na₂SO₄ and concentrated at reduced pressure. The residue is purified by column chromatography (silica gel, ethyl acetate/hexane, 1:3) followed by preparative HPLC (C18, MeCN/water) to provide the title compound (83 mg, 47%) as a yellowish oil. ¹H NMR (DMSO-d₆), δ_{H}, 0.87 (t, 3H), 1.37-1.53 (m, 2H), 1.72-1.83 (m, 1 H), 2.12-2.23 (m, 1 H), 2.30-2.40 (m, 2H), 2.33 (s, 3H), 2.54-2.66 (m, 2H), 2.69-2.79 (m, 1 H), 2.85 (dd, 1 H), 2.91-3.01 (m, 1 H), 3.54-3.62 (m, 1 H), 3.63-3.70 (m, 1 H), 4.64 (d, 1 H), 7.23-7.46 (m, 4H), 7.83 (s, 1 H), 8.56 (s, 1 H). LC/MS (M+H)⁺ = 347

### Example 22

### (rac)-trans-9-(Phenylethynyl)-4-propyl-3,4,4a,5,6,10b-hexahydro-2H-[1,4]oxazino[2,3-f]quinoline

The title compound (obtained as off-white solid HCl salt) is prepared in close analogy to the procedure described in **Example 15** starting with *trans*-9-(phenylethynyl)-3,4,4a,5,6,10b-hexahydro-2*H*-[1,4]oxazino[2,3-*f*]quinoline which is accessible via the method described in **Preparation 5** and **6** using NaBH₄ in MeOH instead of L-selectride for the reduction of the ketone intermediate 2-chloro-*N*-(5-oxo-3-(phenylethynyl)-5,6,7,8-tetrahydroquinolin-6-yl)acetamide.
¹H NMR (CD₃OD), δ_{H}, 1.09 (t, 3H), 1.79-1.95 (m, 2H), 2.18-2.36 (m, 1 H), 2.79-2.83 (m, 1 H), 3.13-3.20 (m, 1 H), 3.30-3.59 (m, 6H), 3.73-3.77 (m, 1 H), 4.32-4.42 (m, 2H), 5.05-5.08 (2 s, 1 H), 7.41 (m, 3H), 7.61 (dd, 2H), 8.61 (s, 1 H), 8.95 (s, 1 H). LC/MS (M+H)⁺ = 333

### Example 23

### (rac)-cis-4-Isobutyl-9-(phenylethynyl)-3,4,4a,5,6,10b-hexahydro-2H-[1,4]oxazino[2,3-f]quinoline

According to General Procedure 4, *cis*-9-(phenylethynyl)-3,4,4a,5,6,10b-hexahydro-2*H*-[1,4]oxazino[2,3-*f*]quinoline (200 mg, 0.69 mmol) is reacted with 1-bromo-2-methylpropane (283 mg, 2.07 mmol) in the presence of K₂CO₃ (414 mg, 3.00 mmol) and Nal (450 mg, 3.00 mmol) in MeCN (5 mL) at reflux for 24 h. Additional amounts of 1-bromo-2-methylpropane (283 mg, 2.07 mmol), K₂CO₃ (414 mg, 3.00 mmol) and Nal (450 mg, 3.00 mmol) are added. The reaction mixture is stirred at reflux for 48 h, cooled down to rt, diluted with water and extracted with DCM. The organic layer is dried over Na₂SO₄ and concentrated at reduced pressure. The obtained residue is purified by column chromatography (silica gel, ethyl acetate/hexane, 1:3) to provide the title compound (75 mg, 31 %) as yellow oil.
¹H NMR (DMSO-d₆), δ_{H}, 0.87 (dd, 6H), 1.72-1.83 (m, 2H), 2.11-2.23 (m, 2H), 2.25-2.32 (m, 1H), 2.34-2.43 (m, 1H), 2.60-2.84 (m, 3H), 2.93-3.02 (m, 1H), 3.58-3.73 (m, 2H), 4.62 (d, 1H), 7.42-7.50 (m, 3H), 7.56-7.63 (m, 2H), 7.84 (s, 1H), 8.57 (s, 1H). LC/MS (M+H)⁺ = 347

### Example 24

### (rac)-cis-9-(Phenylethynyl)-4-(2,2,2-trifluoroethyl)-3,4,4a,5,6,10b-hexahydro-2H-[1,4]oxazino[2,3-f]quinoline

Trifluoromethanesulfonic anhydride (3.29 mL, 19.60 mmol) is added dropwise to a mixture of 2,2,2-trifluoroethanol (1.24 mL, 17.00 mmol) and pyridine (1.36 mL, 19.60 mmol) in anhydrous DCM (10 mL) at 0 °C over 15 min. The resulting mixture is stirred at 0 °C for 1 h and diluted with water (5 mL). The organic layer is separated and dried over Na₂SO₄. The obtained solution of 2,2,2-triluoroethyl trifluoromethanesulfonate (1.00 mL, approx. 1.7 mmol) is added to a mixture of *cis*-9-(phenylethynyl)-3,4,4a,5,6,10b-hexahydro-2*H*-[1,4]oxazino[2,3-*f*]quinoline (200 mg, 0.69 mmol) and TEA (0.5 mL) in MeCN (3 mL). The resulting mixture is stirred at 50-60 °C for 20 h, cooled down to rt, diluted with water and extracted with DCM (100 mL). The organic layer is dried over Na₂SO₄ and concentrated at reduced pressure. The obtained residue is purified by column chromatography (silica gel, ethyl acetate/hexane, 1:2) to provide the title compound (118 mg, 46%) as a yellow oil. ¹H NMR (DMSO-d₆), δ_{H}, 1.77-1.86 (m, 1H), 2.18-2.30 (m, 1H), 2.56-2.64 (m, 1H), 2.74-2.92 (m, 2H), 2.94-3.08 (m, 2H), 3.27-3.48 (m, 2H), 3.66-3.75 (m, 2H), 4.64 (d, 1H), 7.43-7.48 (m, 3H), 7.56-7.62 (m, 2H), 7.85 (d, 1H), 8.60 (d, 1H). LC/MS (M+H)⁺ = 373

### Example 25

### (rac)-cis-9-(Phenylethynyl)-4-(3,3,3-trifluoropropyl)-3,4,4a,5,6,10b-hexahydro-2H-[1,4]oxazino[2,3-f]quinoline

A mixture of *cis*-9-(phenylethynyl)-3,4,4a,5,6,10b-hexahydro-2*H-*[1,4]oxazino[2,3-*f*]quinoline (200 mg, 0.69 mmol), 1,1,1-trifluoro-3-iodopropane (202 mg, 0.90 mmol), and NaHCO₃ (126 mg, 1.50 mmol) in DMF (3 mL) is stirred at 50-60 °C for 6 hours. Then an additional amount of 1,1,1-trifluoro-3-iodopropane (100 mg, 0.45 mmol) is added, and the mixture is stirred at the same temperature for 16 h, cooled down to rt, diluted with water (50 mL) and extracted with EtOAc (2×50 mL). The combined organic layers are dried over Na₂SO₄ and concentrated at reduced pressure. The obtained residue is purified by column chromatography (silica gel, EtOAc/hexane, 1:2) to provide the title compound (75 mg, 28%) as a yellowish oil.
¹H NMR (DMSO-d₆), δ_{H}, 1.75-1.84 (m, 1H), 2.12-2.23 (m, 1H), 2.40-2.54 (m, 3H), 2.61-2.89 (m, 4H), 2.91-3.01 (m, 2H), 3.58-3.71 (m, 2H), 4.64 (d, 1H), 7.43-7.49 (m, 3H), 7.56-7.62 (m, 2H), 7.84 (d, 1H), 8.58 (d, 1H). LC/MS (M+H)⁺ = 387

### Example 26

### (rac)-cis-Methyl 9-(phenylethynyl)-4a,5,6,10b-tetrahydro-2H-[1,4]oxazino[2,3-f]quinoline-4(3H)-carboxylate

*N*-Methyl morpholine (0.17 mL, 1.50 mmol) and methyl chloroformate (97 mg, 1.03 mmol) are added dropwise to a solution of *cis*-9-(phenylethynyl)-3,4,4a,5,6,10b-hexahydro-2*H*-[1,4]oxazino[2,3-*f*]quinoline (200 mg, 0.69 mmol) in anhydrous DCM (7 mL). The reaction mixture is stirred at rt for 1 h and diluted with water (30 mL) and DCM (50 mL). The organic layer is separated, dried over Na₂SO₄ and concentrated at reduced pressure. The obtained residue is purified by column chromatography (silica gel, EtOAc/DCM, 1:2) and crystallized from hexane to provide the title compound (118 mg, 49%) as a white solid.
¹H NMR (DMSO-d₆), δ_{H}, 1.65-1.73 (m, 1 H), 2.25-2.39 (m, 1 H), 2.89-3.20 (m, 3H), 3.62-3.78 (m, 5H), 3.91 (dd, 1 H), 4.18 (m, 1 H), 4.49 (d, 1 H), 7.43-7.49 (m, 3H), 7.56-7.63 (m, 2H), 7.88 (d, 1 H), 8.64 (d, 1H). LC/MS (M+H)⁺ = 349, 390

### Example 27

### (rac)-cis-N,N-Dimethyl-9-(phenylethynyl)-4a,5,6,10b-tetrahydro-2H [1,4]oxazino[2,3-f]quinoline-4(3H)-carboxamide

According to General Procedure 4, *cis*-9-(phenylethynyl)-3,4,4a,5,6,10b-hexahydro-2*H*-[1,4]oxazino[2,3-*f*]quinoline (170 mg, 0.59 mmol) is reacted with dimethylcarbamoyl chloride (0.5 mL) in the presence of TEA (0.8 mL) in dry DCM (7 mL). The crude product is purified by column chromatography (silica gel, EtOAc/EtOH, 30:1) followed by preparative HPLC (C18, MeCN/water) to provide the title compound (98 mg, 46%) as a colorless oil which solidifies upon standing at rt.
¹H NMR (DMSO-d₆), δ_{H}, 1.67-1.76 (m, 1 H), 2.32-2.48 (m, 1 H), 2.78 (s, 6H), 2.93-3.05 (m, 2H), 3.13-3.30 (m, 2H), 3.64-3.75 (m, 1 H), 3.80-3.90 (m, 2H), 4.51 (d, 1 H), 7.43-7.50 (m, 3H), 7.56-7.62 (m, 2H), 7.87 (d, 1 H), 8.64 (d, 1 H). LC/MS (M+H)⁺ = 362

### Example 28

### 1-Methyl-8-(phenylethynyl)-4,5-dihydro-1H-pyrazolo[3,4-f]quinoline

Methyl formate (159 mg, 2.65 mmol, 2 eq.) is added to a suspension of 120 mg of NaH (80% in mineral oil) (95 mg, 3.98 mmol, 3 eq.) in 6 mL of dry Et₂O. The resulting mixture is heated to reflux and 3-bromo-7,8-dihydroquinolin-5(6*H*)-one (300 mg, 1.33 mmol, 1 eq.), followed by a drop of methanol are added. The mixture is refluxed for 6 h and then stirred at rt overnight. The reaction mixture is diluted with DCM and water and AcOH is added to pH ~5. The organic layer is separated, and the aqueous layer is extracted with DCM (2 times). The combined organic phase is dried over anhydrous Na₂SO₄, filtered and evaporated to give 310 mg (92%) of sufficiently pure 3-bromo-5-oxo-5,6,7,8-tetrahydroquinoline-6-carbaldehyde as a yellow oil.

3-Bromo-5-oxo-5,6,7,8-tetrahydroquinoline-6-carbaldehyde (100 mg, 0.39 mmol, 1 eq.) is dissolved in 2 mL of MeOH and methylhydrazine (36 mg, 0.79 mmol, 2 eq.) is added. The mixture is stirred overnight at rt, then evaporated to dryness, and the residue is purified by flash column chromatography to give 80 mg (78%) of a ca. 1:1 mixture of 8-bromo-2-methyl-4,5-dihydro-2*H*-pyrazolo[3,4-*f*]quinoline and 8-bromo-1-methyl-4,5-dihydro-1*H*-pyrazolo[3,4-f]quinoline. The isomers are separated by preparative HPLC.
8-Bromo-1-methyl-4,5-dihydro-1H-pyrazolo[3,4-f]quinoline is coupled with ethynylbenzene according to General Procedure 1 to provide the title compound.
¹H NMR (CDCl₃), δ_{H}, 2.83 (t, 2H), 3.15 (t, 2H), 4.18 (s, 2H), 7.36-7.40 (m, 4H), 7.51-7.61 (m, 2H), 7.90 (d, 1 H), 8.55 (d, 1 H). LC/MS (M+H)⁺ = 286

### Example 29

### (rac)-cis-Methyl 8-(phenylethynyl)-2,3,3a,4,5,9b-hexahydro-1H-pyrrolo[2,3-f]quinoline-1-carboxylate

According to General Procedure 5, *tert*-butyl 6-oxooctahydro-1*H*-indole-1-carboxylate (1.00 g, 4.18 mmol) is reacted with *N*-(2-bromo-3-(dimethylamino)allylidene)-*N*-methylmethanaminium hexafluorophosphate, and the crude product is purified by flash column chromatography (silica, gradient 20% to 70% EtOAc/heptane) to yield a mixture of *tert*-butyl 8-bromo-2,3,3a,4,5,9b-hexahydro-1*H-*pyrrolo[2,3-*f*]quinoline-1-carboxylate and *tert*-butyl 6-bromo-2,3,3a,4,9,9a-hexahydro-1*H*-pyrrolo[3,2-*g*]quinoline-1-carboxylate which is used as such in the next reaction step.

To remove the Boc group TFA (2.3 mL, 6.49 mmol) is added to a solution of the obtained mixture (727 mg in total, 2.06 mmol) in DCM (2.3 mL). The reaction mixture is stirred at rt for 3 h, then concentrated *in vacuo* and co-evaporated with DCM (3x4 mL). The crude residue is used without further purification. Next, TEA (1.43 mL, 10.3 mmol) is added to a cooled solution (0 °C) of the crude reaction mixture of the intermediates in DCM (10 mL), immediately followed by the addition of methyl chloroformate (0.319 mL, 0.412 mmol). After stirring at 0 °C for 1 h a saturated aqueous solution of NaHCO₃ (10 mL) is added, and the mixture is stirred for approximately 0.5 h. The layers are separated with a phase separator, and the organic layer is concentrated *in vacuo.* Purification by flash column chromatography (gradient 25% to 75% EtOAc/heptane) provides the desired regioisomer methyl 8-bromo-2,3,3a,4,5,9b-hexahydro-1*H-*pyrrolo[2,3-*f*]quinoline-1-carboxylate as a solid (205 mg, 30% over 2 steps), and the second regioisomer methyl 6-bromo-2,3,3a,4,9,9a-hexahydro-1*H*-pyrrolo[3,2-*g*]quinoline-1-carboxylate, which is recrystallized from heptane/EtOAc (284 mg, 44% over 2 steps).

According to General Procedure 1, methyl 8-bromo-2,3,3a,4,5,9b-hexahydro-1*H*-pyrrolo[2,3-*f*]quinoline-1-carboxylate (90 mg, 0.289 mmol) is reacted with ethynylbenzene (64 µL, 0.578 mmol) in the presence of PdCl₂[PPh₃]₂ (10.2 mg, 14 µmol), Cul (2.8 mg, 14 µmol) and TEA (0.161 mL, 1.16 mmol) in dry DMF (2 mL) at 100 °C for 2 h. The crude product is purified by flash column chromatography (Grace pre-packed column, silica 12 g, gradient 25% to 75% EtOAc/heptane) to provide the title compound (41 mg, 42%) as a brown glass-like solid.
¹H NMR (DMSO-d₆), δ_{H}, 1.47-1.82 (m, 2H), 1.91-1.98 (m, 1 H), 2.05-2.18 (m, 1 H), 2.59-2.69 (m, 1H), 2.76-2.94 (m, 2H), 3.24-3.34 (m, 1H), 3.39-3.52 (m, 1H), 3.70 (br s, 3H), 4.94-5.02 (m, 1H), 7.42-7.47 (m, 3H), 7.57-7.62 (m, 2H), 7.73-7.81 and 8.00-8.05 (2 x m, rotamers, 1H), 8.54-8.57 (m, 1H). LC/MS (M+H)⁺ = 333

### Example 30

### Methyl 8-(phenylethynyl)-3,3a,4,5-tetrahydro-1H-pyrrolo[3,4-f]quinoline-2(9bH)-carboxylate

Under a nitrogen atmosphere, 2-benzylhexahydro-1*H*-isoindol-5(6H)-one (1 g, 4.36 mmol) is dissolved in DCM (12 mL). At 0 °C, methyl chloroformate (1.5 mL, 19.41 mmol) is added dropwise. The reaction mixture is stirred at ambient temperature for 48 h and then concentrated to dryness. Purification by flash column chromatography (silica, gradient 100% heptane to 75% EtOAc/heptane) yields the intermediate product methyl 5-oxohexahydro-1*H*-isoindole-2(3*H*)-carboxylate (402 mg, 46%) as a colourless oil.

According to General Procedure 5, methyl 5-oxohexahydro-1*H*-isoindole-2(3*H*)-carboxylate (395 mg, 2.0 mmol) is reacted with *N*-(2-bromo-3-(dimethylamino)allylidene)-*N*-methylmethanaminium hexafluorophosphate, and the crude product is purified by flash column chromatography (silica, gradient 100% heptane to 70% EtOAc/heptane) to give methyl 8-bromo-3,3a,4,5-tetrahydro-1*H-*pyrrolo[3,4-*f*]quinoline-2(9b*H*)-carboxylate (399 mg, 64%) as a brown oil, which solidifies upon standing.

According to General Procedure 1, methyl 8-bromo-3,3a,4,5-tetrahydro-1*H-*pyrrolo[3,4-f]quinoline-2(9b*H*)-carboxylate (120 mg, 0.386 mmol) is reacted with ethynylbenzene (0.085 mL, 0.771 mmol) in the presence of PdCl₂[PPh₃]₂ (13.53 mg, 0.019 mmol), Cul (2.94 mg, 0.015 mmol) and TEA (0.215 mL, 1.543 mmol) in dry DMF (3 m L) at 100 °C for 16 h. The crude reaction mixture is filtered over a pad of kieselguhr, which is thoroughly rinsed with EtOAc (150 mL). The filtrate is concentrated to dryness, then dissolved again in EtOAc (25 mL) and washed with brine (3x25 mL), dried over Na₂SO₄, filtered, and the filtrate is evaporated to dryness. Purification by flash column chromatography (adsorbed onto hydromatrix, silica, gradient 20% to 60% EtOAc/heptane) provides the title compound 52 mg (40%).
¹H NMR (CDCl₃), δ_{H}, 1.70-1.88 (m, 1 H), 1.86-2.05 (m, 1 H), 2.45-2.60 (m, 1 H), 2.85-3.10 (m, 2H), 3.18-3-50 (m, 3H), 3.64-3.76 (m, 4H), 3.87-4.05 (m, 1 H), 7.32-7.42 (m, 3H), 7.50-7.60 (m, 3H), 8.53-8.62 (m, 1 H). LC/MS (M+H)⁺ = 333

### Example 31

### Methyl 8-((3-fluorophenyl)ethynyl)-3,3a,4,5-tetrahydro-1H-pyrrolo[3,4-f]quinoline-2(9bH)-carboxylate

According to General Procedure 1, methyl 8-bromo-3,3a,4,5-tetrahydro-1*H-*pyrrolo[3,4-*f*]quinoline-2(9b*H*)-carboxylate (120 mg, 0.386 mmol, for preparation see **Example 30**) is reacted with 1-ethynyl-3-fluorobenzene (0.089 mL, 0.771 mmol) in the presence of PdC₂[PPh₃]₂ (13.53 mg, 0.019 mmol), Cul (2.94 mg, 0.015 mmol) and TEA (0.215 mL, 1.543 mmol) in dry DMF (3 mL) at 100 °C for 16 h. The crude reaction mixture is purified according to the work-up procedure described in **Example 30** to provide the title compound (54 mg, 40%).
¹H NMR (CDCl₃), δ_{H}, 1.71-1.88 (m, 1 H), 1.93-2.03 (m, 1 H), 2.48-2.61 (m, 1 H), 2.88-3.10 (m, 2H), 3.19-3.51 (m, 3H), 3.64-3.77 (m, 4H), 3.87-4.05 (m, 1 H), 7.03-7.13 (m, 1 H), 7.20-7.25 (m, 1 H), 7.29-7.38 (m, 2H), 7.51-7.59 (m, 1 H), 8.54-8.59 (m, 1 H). LC/MS (M+H)⁺ = 351

### Example 32

### (rac)-cis-Methyl 3-((3-fluorophenyl)ethynyl)-6a,7,9,9a-tetrahydro-5H-pyrrolo[3,4-h]quinoline-8(6H)-carboxylate

According to General Procedure 5, rac-*cis* tert-butyl 4-oxohexahydro-1*H-*isoindole-2(3*H*)-carboxylate (250 mg, 1.045 mmol) is reacted with *N*-(2-bromo-3-(dimethylamino)allylidene)-*N*-methylmethanaminium hexafluorophosphate, and the crude product is purified by flash column chromatography (silica, gradient 20% to 70% EtOAc/heptane) to provide the intermediate product *tert*-butyl 3-bromo-6a,7,9,9a-tetrahydro-5*H*-pyrrolo[3,4-*h*]quinoline-8(6*H*)-carboxylate (89 mg, 24%).

Boc-deprotection is accomplished according to the procedure described in **Example 29.** Then, TEA (0.317 mL, 2.271 mmol) is added to a cooled solution (0 °C) of the obtained crude intermediate 3-bromo-6,6a,7,8,9,9a-hexahydro-5*H*-pyrrolo[3,4-*h*]quinoline 2,2,2-trifluoroacetate in DCM (3 mL), immediately followed by the addition of methyl chloroformate (0.070 mL, 0.909 mmol). After stirring at 0 °C for 3 h a saturated aqueous solution of NaHCO₃ (10 mL) is added, and the mixture is stirred for 0.5 h. The layers are separated with a phase separator, and the organic layer is concentrated *in* vacuo. Purification by flash column chromatography (gradient 20% to 75% EtOAc/heptane) provides methyl 3-bromo-6a,7,9,9a-tetrahydro-5*H*-pyrrolo[3,4-*h*]quinoline-8(6*H*)-carboxylate (122 mg, 86% over 2 steps) as a slightly yellow oil.

According to General Procedure 1, 3-bromo-6a,7,9,9a-tetrahydro-5*H-*pyrrolo[3,4-*h*]quinoline-8(6*H*)-carboxylate (61 mg, 0.196 mmol) is reacted with ethynylbenzene (43 µL, 0.392 mmol) in the presence of PdCl₂[PPh₃]₂ (6.9 mg, 9.80 µmol), Cul (1.9 mg, 9.80 µmol) and TEA (0.11 mL, 0.784 mmol) in dry DMF (2 mL) at 100 °C for 2 h. The crude product is purified by flash column chromatography (Grace pre-packed column, silica 4 g, gradient 25% to 75% EtOAc/heptane) to give an oil, which is crystallized from pentane/Et₂O to provide the title compound (26 mg, 41 %) as brown crystals.
¹H NMR (DMSO-d₆), δ_{H}, 1.52-1.63 (m, 1 H), 1.81-1.88 (m, 1 H), 2.56-2.67 (m, 1 H), 2.73-2.88 (m, 2H), 3.24-3.37 (m, 2H), 3.46-3.63 (m, 2H), 3.56 and 3.57 (2 x s, rotamers, 3H), 3.79-3.84 (m, 1 H), 7.43-7.48 (m, 3H), 7.55-7.60 (m, 2H), 7.73-7.76 (m, 1 H), 8.53-8.56 (m, 1 H). LC/MS (M+H)⁺ = 333

### Example 33

### 2-Methyl-1-(3-(phenylethynyl)-7,8-dihydro-1,6-naphthyridin-6(5H)-yl)propan-1-one

According to General Procedure 2, 3-(phenylethynyl)-5,6,7,8-tetrahydro-1,6-naphthyridine is acylated with isobutyryl chloride to provide the title compound as a colorless solid.
¹H NMR (CDCl₃), δ_{H}, 1.15 and 1.26 (both d, 3H total), 2.90 (m, 1 H), 3.05 and 3.09 (both t, 2H total), 3.85 and 3.95 (both t, 2h total), 4.67 and 4.77 (both s, 2H total), 7.36-7.40 (m, 3H), 7.50-7.59 (m, 2H), 7.60 (d, 1 H), 8.58 (d, 1 H). LC/MS (M+H)⁺ = 305

### Example 34

### 2,2-Dimethyl-1-(3-(phenylethynyl)-7,8-dihydro-1,6-naphthyridin-6(5H)-yl)propan-1-one

According to General Procedure 2, 3-(phenylethynyl)-5,6,7,8-tetrahydro-1,6-naphthyridine is acylated with pivaloyl chloride to provide the title compound as a colorless solid.
¹H NMR (CDCl₃), δ_{H}, 1.34 (s, 9H), 3.06 (t, 2H), 3.97 (t, 2H), 4.76 (s, 2H), 7.34-7.36 (m, 3H), 7.51-7.56 (m, 2H), 7.58 (d, 1 H), 8.59 (d, 1 H). LC/MS (M+H)⁺ = 319

### Example 35

### 1-(3-((6-Aminopyridin-2-yl)ethynyl)-7,8-dihydro-1,6-naphthyridin-6(5H)-yl)-2,2-dimethylpropan-1-one

According to General Procedure 2, 3-bromo-5,6,7,8-tetrahydro-1,6-naphthyridine is acylated with pivaloyl chloride to provide 1-(3-bromo-7,8-dihydro-1,6-naphthyridin-6(5*H*)-yl)-2,2-dimethylpropan-1-one, which is coupled with 6-ethynylpyridin-2-amine according to General Procedure 1 to provide the title compound as a colorless solid.
¹H NMR (CDCl₃), δ_{H}, 1.31 (s, 9H), 3.04 (t, 2H), 3.94 (t, 2H), 4.72 (s, 2H), 6.48 (d, 1 H), 6.90 (d, 1 H), 7.40 (t, 1 H), 7.57 (d, 1 H), 8.57 (d, 1H). LC/MS (M+H)⁺ = 335

### Example 36

### (3-Methyloxetan-3-yl)(3-(phenylethynyl)-7,8-dihydro-1,6-naphthyridin-6(5H)-yl)methanone

According to General Procedure 2, 3-(phenylethynyl)-5,6,7,8-tetrahydro-1,6-naphthyridine is acylated with 3-methyloxetane-3-carbonyl chloride to provide the title compound as a colorless solid. ¹H NMR (CDCl₃), δ_{H}, 1.60 (s, 3H), 3.07 (t, 2H), 3.37 (t, 2H), 4.42 (m, 2H), 4.76 (s, 2H), 5.05 (m, 2H), 7.35-7.38 (m, 3H), 7.50-7.53 (m, 2H), 7.61 (d, 1 H), 8.58 (d, 1 H). LC/MS (M+H)⁺ = 333

### Example 37

### (rac)-6-(tert-Butylsulfinyl)-3-(phenylethynyl)-5,6,7,8-tetrahydro-1,6-naphthyridine

According to General Procedure 2, 3-(phenylethynyl)-5,6,7,8-tetrahydro-1,6-naphthyridine is acylated with 2-methylpropane-2-sulfinic chloride to provide the title compound as a colorless solid.
¹H NMR (CDCl₃), δ_{H}, 1.22 (s, 9H), 3.09 (t, 2H), 3.37-3.63 (m, 2H), 4.28 (AB m, 2H), 7.34-7.38 (m, 3H), 7.50-7.55 (m, 3H), 8.57 (d, 1 H).
LC/MS (M+H)⁺ = 339

### Example 38

### 6-(Cyclopropylsulfonyl)-3-(phenylethynyl)-5,6,7,8-tetrahydro-1,6-naphthyridine

According to General Procedure 2, 3-(phenylethynyl)-5,6,7,8-tetrahydro-1,6-naphthyridine is acylated with cyclopropanesulfonyl chloride to provide the title compound as a colorless solid.
¹H NMR (CDCl₃), δ_{H}, 1.01 (m, 2H), 1.23 (m, 2H), 2.30 (m, 1 H), 3.16 (t, 2H), 3.74 (t, 2H), 4.53 (s, 2H), 7.35-7.39 (m, 3H), 7.51-7.55 (m, 3H), 8.61 (d, 1 H).
LC/MS (M+H)⁺ = 339

### Example 39

### 3-(Phenylethynyl)-6-(phenylsulfonyl)-5,6,7,8-tetrahydro-1,6-naphthyridine

According to General Procedure 2, 3-(phenylethynyl)-5,6,7,8-tetrahydro-1,6-naphthyridine is acylated with benzenesulfonyl chloride to provide the title compound as a colorless solid.
¹H NMR (CDCl₃), δ_{H}, 3.10 (t, 2H), 3.49 (t, 2H), 4.29 (s, 2H), 7.35-7.38 (m, 3H), 7.50-7.62 (m, 6H), 7.83-7.87 (m, 2H), 8.55 (d, 1 H).
LC/MS (M+H)⁺ = 375

### Example 40

### Ethyl 3-(phenylethynyl)-7,8-dihydro-1,6-naphthyridine-6(5H)-carboxylate

According to General Procedure 2, 3-bromo-5,6,7,8-tetrahydro-1,6-naphthyridine (213 mg, 1.00 mmol) is acylated with ethyl chloroformate (163 mg, 1.50 mmol) in the presence of TEA (347 mg, 3.43 mmol) and DMAP (15 mg, 0.12 mmol) to provide ethyl 3-bromo-7,8-dihydro-1,6-naphthyridine-6(5*H*)-carboxylate which is coupled with ethynylbenzene in the presence of TEA (1.6 mL), *t*-Bu₃P (17 mg, 0.084 mmol) and PdCl₂[PPh₃]₂ (17 mg, 0.024 mmol) in anhydrous MeCN (10 mL) according to General Procedure 1 to provide the title compound (67 mg, 28%) as a yellowish solid.
¹H NMR (DMSO-d₆), δ_{H}, 1.23 (t, 3H), 2.93 (t, 2H), 3.72 (t, 2H), 4.11 (q, 2H), 4.62 (s, 2H), 7.42-7.47 (m, 3H), 7.54-7.60 (m, 2H), 7.82 (d, 1 H), 8.55 (d, 1 H).
LC/MS (M+H)⁺ = 307, 231

### Example 41

### Isobutyl 3-(phenylethynyl)-7,8-dihydro-1,6-naphthyridine-6(5H)-carboxylate

According to General Procedure 2, 3-(phenylethynyl)-5,6,7,8-tetrahydro-1,6-naphthyridine is acylated with 3-methylbutanoyl chloride to provide the title compound as a colorless solid.
¹H NMR (CDCl₃), δ_{H}, 0.96 (d, 6H), 1.97 (m, 1 H), 3.04 (t, 2H), 3.82 (t, 2H), 3.92 (d, 2H), 4.65 (s, 2H), 7.35-7.39 (m, 3H), 7.51-7.57 (m, 3H), 8.58 (d, 1 H).
LC/MS (M+H)⁺ = 335

### Example 42

### tert-Butyl 3-(phenylethynyl)-7,8-dihydro-1,6-naphthyridine-6(5H)-carboxylate

Synthesis of the title compound is given in **Preparation 3.**
¹H NMR (CDCl₃), δ_{H}, 1.49 (s, 9H), 3.02 (t, 2H), 3.76 (t, 2H), 4.60 (s, 2H), 7.35-7.39 (m, 3H), 7.51-7.57 (m, 3H), 8.57 (d, 1 H).
LC/MS (M+H)⁺ = 335

### Example 43

### Phenyl 3-(phenylethynyl)-7,8-dihydro-1,6-naphthyridine-6(5H)-carboxylate

According to General Procedure 2, 3-(phenylethynyl)-5,6,7,8-tetrahydro-1,6-naphthyridine is acylated with phenyl carbonochloridate to provide the title compound as a colorless solid.
¹H NMR (CDCl₃), δ_{H}, 3.08 (t, 2H), 3.85 and 3.95 (both t, 2H total), 4.68 and 4.79 (both s, 2H total), 7.07 (d, 2H), 7.15 (t, 2H), 7.29-7.33 (m, 4H), 7.46-7.49 (m, 2H), 7.53 (d, 1 H), 8.55 (d, 1H).
LC/MS (M+H)⁺ = 355

### Example 44

### N,N-Dimethyl-3-(phenylethynyl)-7,8-dihydro-1,6-naphthyridine-6(5H)-carboxamide

According to General Procedure 2, 3-(phenylethynyl)-5,6,7,8-tetrahydro-1,6-naphthyridine is acylated with dimethylcarbamic chloride to provide the title compound as a colorless solid.
¹H NMR (CDCl₃), δ_{H}, 2.90 (s, 6H), 3.09 (t, 2H), 3.57 (t, 2H), 4.40 (s, 2H), 7.34-7.38 (m, 3H), 7.51-7.55 (m, 3H), 8.57 (d, 1 H).
LC/MS (M+H)⁺ = 306

### Example 45

### 3-((3-Fluorophenyl)ethynyl)-N,N-dimethyl-7,8-dihydro-1,6-naphthyridine-6(5H)-carboxamide

According to Preparation 3, tert-butyl 3-bromo-7,8-dihydro-1,6-naphthyridine-6(5H)-carboxylate is coupled with 1-ethynyl-3-fluorobenzene to provide, after removal of the Boc group, 3-((3-fluorophenyl)ethynyl)-5,6,7,8-tetrahydro-1,6-naphthyridine, which is then acylated with dimethylcarbamic chloride according to General Procedure 2, to provide the title compound as a colorless solid.
¹H NMR (CDCl₃), δ_{H}, 2.90 (s, 6H), 3.09 (t, 2H), 3.57 (t, 2H), 4.40 (s, 2H), 7.07 (m, 1H), 7.21-7.35 (m, 3H), 7.55 (d, 1 H), 8.57 (d, 1 H).
LC/MS (M+H)⁺ = 324

### Example 46

### 3-((3-Cyanophenyl)ethynyl)-N,N-dimethyl-7,8-dihydro-1,6-naphthyridine-6(5H)-carboxamide

According to Preparation 3, *tert*-butyl 3-bromo-7,8-dihydro-1,6-naphthyridine-6(5*H*)-carboxylate is coupled with 3-ethynylbenzonitrile to provide, after removal of the Boc group, 3-((5,6,7,8-tetrahydro-1,6-naphthyridin-3-yl)ethynyl)benzonitrile, which is then acylated with dimethylcarbamic chloride according to General Procedure 2, to provide the title compound as a colorless solid.
¹H NMR (CDCl₃), δ_{H}, 2.90 (s, 6H), 3.10 (t, 2H), 3.56 (t, 2H), 4.40 (s, 2H), 7.44-7.81 (m, 5H), 8.58 (d, 1H).
LC/MS (M+H)⁺ = 331

### Example 47

### 3-((6-Aminopyridin-2-yl)ethynyl)-N,N-dimethyl-7,8-dihydro-1,6-naphthyridine-6(5H)-carboxamide

According to Preparation 3, tert-butyl 3-bromo-7,8-dihydro-1,6-naphthyridine-6(5*H*)-carboxylate is coupled with 6-ethynylpyridin-2-amine to provide, after removal of the Boc group, 6-((5,6,7,8-tetrahydro-1,6-naphthyridin-3-yl)ethynyl)pyridin-2-amine, which is then acylated with dimethylcarbamic chloride according to General Procedure 2, to provide the title compound as a colorless solid.
¹H NMR (CDCl₃), δ_{H}, 2.90 (s, 6H), 3.09 (t, 2H), 3.56 (t, 2H), 4.39 (s, 2H), 4.93 (br. s, 2H), 6.50 (d, 1 H), 6.91 (d, 1 H), 7.44 (t, 1 H), 7.60 (d, 1 H), 8.62 (d, 1H).
LC/MS (M+H)⁺ = 322

### Example 48

### 3-((3-Cyanophenyl)ethynyl)-N,N-diethyl-7,8-dihydro-1,6-naphthyridine-6(5H)-carboxamide

According to Preparation 3, *tert*-butyl 3-bromo-7,8-dihydro-1,6-naphthyridine-6(5*H*)-carboxylate is coupled with 3-ethynylbenzonitrile to provide, after removal of the Boc group, 3-((5,6,7,8-tetrahydro-1,6-naphthyridin-3-yl)ethynyl)benzonitrile, which is then acylated with diethylcarbamic chloride according to General Procedure 2, to provide the title compound as a colorless solid.
¹H NMR (CDCl₃), δ_{H}, 1.17 (t, 6H), 3.09 (t, 2H), 3.28 (q, 4H), 3.55 (t, 2H), 4.38 (s, 2H), 7.45-7.65 (m, 3H), 7.74 (d, 1 H), 7.82 (d, 1 H), 8.58 (d, 1 H).
LC/MS (M+H)⁺ = 359

### Example 49

### 3-(Phenylethynyl)-N,N-bis(2,2,2-trifluoroethyl)-7,8-dihydro-1,6-naphthyridine-6(5H)-carboxamide

According to General Procedure 2, 3-(phenylethynyl)-5,6,7,8-tetrahydro-1,6-naphthyridine is acylated with bis(2,2,2-trifluoroethyl)carbamic chloride to provide the title compound as a colorless solid.
¹H NMR (CDCl₃), δ_{H}, 3.11 (t, 2H), 3.67 (t, 2H), 3.99 (q, 4H), 4.83 (s, 2H), 7.35-7.38 (m, 3H), 7.51-7.57 (m, 3H), 8.60 (d, 1 H).
LC/MS (M+H)⁺ = 442

### Example 50

### (3-(Phenylethynyl)-7,8-dihydro-1,6-naphthyridin-6(5H)-yl)(piperidin-1-yl)methanone

According to General Procedure 2, 3-(phenylethynyl)-5,6,7,8-tetrahydro-1,6-naphthyridine is acylated with piperidine-1-carbonyl chloride to provide the title compound as a colorless solid.
¹H NMR (CDCl₃), δ_{H}, 1.59 (m, 6H), 3.08 (t, 2H), 3.26 (m, 4H), 3.56 (t, 2H), 4.40 (s, 2H), 7.35-7.38 (m, 3H), 7.51-7.55 (m, 3H), 8.57 (d, 1 H).
LC/MS (M+H)⁺ = 346

### Example 51

### 3-((6-(Piperidine-1-carbonyl)-5,6,7,8-tetrahydro-1,6-naphthyridin-3-yl)ethynyl)benzonitrile

According to Preparation 3, *tert*-butyl 3-bromo-7,8-dihydro-1,6-naphthyridine-6(5*H*)-carboxylate is coupled with 3-ethynylbenzonitrile to provide, after removal of the Boc group, 3-((5,6,7,8-tetrahydro-1,6-naphthyridin-3-yl)ethynyl)benzonitrile, which is then acylated with piperidine-1-carbonyl chloride according to General Procedure 2, to provide the title compound as a colorless solid.
¹H NMR (CDCl₃), δ_{H}, 1.55 (m, 6H), 3.03 (t, 2H), 3.21 (m, 4H), 3.50 (t, 2H), 4.35 (s, 2H), 7.42 (t, 1H), 7.49 (s, 1H), 7.56 (d, 1H), 7.67 (d, 1 H), 7.75 (d, 1 H), 8.51 (d, 1 H).
LC/MS (M+H)⁺ = 371

### Example 52

### N,N-Dimethyl-3-(phenylethynyl)-7,8-dihydro-1,6-naphthyridine-6(5H)-sulfonamide

According to General Procedure 2, 3-(phenylethynyl)-5,6,7,8-tetrahydro-1,6-naphthyridine is acylated with dimethylsulfamoyl chloride to provide the title compound as a colorless solid.
¹H NMR (CDCl₃), δ_{H}, 2.86 (s, 6H), 3.12 (t, 2H), 3.63 (t, 2H), 4.43 (s, 2H), 7.35-7.39 (m, 3H), 7.51-7.56 (m, 3H), 8.59 (d, 1 H).
LC/MS (M+H)⁺ = 342

### Example 53

### 3-(Phenylethynyl)-6-(pyrrolidin-1-ylsulfonyl)-5,6,7,8-tetrahydro-1,6-naphthyridine

According to General Procedure 2, 3-(phenylethynyl)-5,6,7,8-tetrahydro-1,6-naphthyridine is acylated with pyrrolidine-1-sulfonyl chloride to provide the title compound as a colorless solid.
¹H NMR (CDCl₃), δ_{H}, 1.89-1.94 (m, 4H), 3.11 (t, 2H), 3.33-3.38 (m, 4H), 3.63 (t, 2H), 4.43 (s, 2H), 7.35-7.39 (m, 3H), 7.51-7.55 (m, 3H), 8.58 (d, 1 H).
LC/MS (M+H)⁺ = 368

### Example 54

### 3-(Phenylethynyl)-6-(piperidin-1-ylsulfonyl)-5,6,7,8-tetrahydro-1,6-naphthyridine

According to General Procedure 2, 3-(phenylethynyl)-5,6,7,8-tetrahydro-1,6-naphthyridine is acylated with piperidine-1-sulfonyl chloride to provide the title compound as a colorless solid.
¹H NMR (CDCl₃), δ_{H}, 1.49-1.59 (m, 6H), 3.04 (t, 2H), 3.17 (m, 4H), 3.55 (t, 2H), 4.36 (s, 2H), 7.29-7.31 (m, 3H), 7.46-7.48 (m, 3H), 8.52 (d, 1 H).
LC/MS (M+H)⁺ = 382

### Example 55

### 4-((3-(Phenylethynyl)-7,8-dihydro-1,6-naphthyridin-6(5H)-yl)sulfonyl)morpholine

According to General Procedure 2, 3-(phenylethynyl)-5,6,7,8-tetrahydro-1,6-naphthyridine is acylated with morpholine-4-sulfonyl chloride to provide the title compound as a colorless solid.
¹H NMR (CDCl₃), δ_{H}, 3.12 (t, 2H), 3.24 (m, 4H), 3.67 (t, 2H), 3.74 (m, 4H), 4.46 (s, 2H), 7.35-7.38 (m, 3H), 7.51-7.56 (m, 3H), 8.60 (d, 1 H).
LC/MS (M+H)⁺ = 384

### Example 56

### 6-(Azepan-1-ylsulfonyl)-3-(phenylethynyl)-5,6,7,8-tetrahydro-1,6-naphthyridine

According to General Procedure 2, 3-(phenylethynyl)-5,6,7,8-tetrahydro-1,6-naphthyridine is acylated with azepane-1-sulfonyl chloride to provide the title compound as a colorless solid.
¹H NMR (CDCl₃), δ_{H}, 1.65 (m, 4H), 1.74 (m, 4H), 3.11 (t, 2H), 3.40 (m, 4H), 3.56 (t, 2H), 4.37 (s, 2H), 7.35-7.38 (m, 3H), 7.51-7.56 (m, 3H), 8.58 (d, 1 H).
LC/MS (M+H)⁺ = 396

### Example 57

### (rac)-8-Methyl-3-(phenylethynyl)-9,10,10a,11-tetrahydro-5H-pyrimido[1,6-g][1,6]naphthyridin-7(8H)-one

### 1. 2-Methylhexahydro-1H-pyrido[1,2-c]pyrimidine-1,6(2H)-dione

CDI (2.80g, 17.24 mmol) is suspended in 15 mL of dry DCM, the mixture is cooled in the ice bath and 3,3-diethoxy-*N*-methylpropan-1-amine (Synthesis 1995; 1995(5), p. 592-604), (2,78 g, 17,24 mmol) is added. After 30 min the ice bath is removed and the mixture is stirred overnight. The mixture is partitioned between DCM and water, the organic layer is separated, dried over anhydrous Na₂SO₄, filtered and evaporated to give 3.55 g (80%) of sufficiently pure *N*-(3,3-diethoxypropyl)-*N*-methyl-1*H*-imidazole-1-carboxamide as red oil. This intermediate is dissolved in MeCN (25 mL) and methyl iodide (7.89 g, 55.62 mmol) is added. The mixture is stirred at rt overnight, the solvent is evaporated and the residue is dried under reduced pressure to give sufficiently pure 1-((3,3-diethoxypropyl)(methyl)carbamoyl)-3-methyl-1*H*-imidazol-3-ium iodide.

But-3-en-1-amine (492 mg, 6.92 mmol) is dissolved in DCM (20 mL), and this solution is added to a suspension of 1-((3,3-diethoxypropyl)(methyl)carbamoyl)-3-methyl-1*H*-imidazol-3-ium iodide (5.50 g, 13.85 mmol) in DCM (10 mL), followed by addition of TEA (3.50 g, 34.61 mmol). The mixture is stirred overnight at rt, then partitioned between DCM and sat. aq. NaHCO₃ solution. The organic phase is separated, dried over anhydrous Na₂SO₄, filtered and evaporated. The residue is purified by flash column chromatography on silica gel to give 3-(but-3-en-1-yl)-1-(3,3-diethoxypropyl)-1-methylurea (820 mg, 46%) as a yellow oil.

3-(But-3-en-1-yl)-1-(3,3-diethoxypropyl)-1-methylurea (530 mg, 2.05 mmol) is dissolved in DCM (5 mL), then TFA (5 mL) is added, and the mixture is stirred for 2 h at rt, then evaporated to dryness. The residue is dissolved MeOH (5 mL), and potassium carbonate (284 mg, 2.05 mmol) is added. The mixture is stirred for 3 h at rt, then evaporated to 0.5 mL volume and directly purified by flash column chromatography on silica gel to give 6-hydroxy-2-methyloctahydro-1H-pyrido[1,2-c]pyrimidin-1-one (320 mg, 85%) as a yellow oil.

A solution of oxalyl chloride (558 mg, 4.39 mmol) in dry DCM (20 mL) is cooled to -78 °C, and DMSO (687 mg, 8.79 mmol) is added dropwise. The mixture is stirred for 1 h at -78 °C, then a solution of 6-hydroxy-2-methyloctahydro-1H-pyrido[1,2-c]pyrimidin-1-one (270 mg, 1.46 mmol) in dry DCM (3 mL) is added dropwise. The solution is stirred for 1 h at -78 °C, then TEA (1779 mg, 17.58 mmol) is added, and the mixture is allowed to warm to ambient temperature. Saturated aq. NaHCO₃ solution is added, the organic layer is separated and the aqueous layer is washed with DCM (2x). The combined organic phase is dried over anhydrous Na₂SO₄, filtered and evaporated. The residue is purified by flash column chromatography on silica gel to give 2-methylhexahydro-1*H-*pyrido[1,2-c]pyrimidine-1,6(2*H*)-dione (156 mg, 49%) as a red oil.

### 2. 3-Bromo-8-methyl-9,10,10a,11-tetrahydro-5H-pyrimido[1,6-g][1,6]naphthyridin-7(8H)-one

In close analogy to General Procedure 5, 2-methylhexahydro-1*H*-pyrido[1,2-c]pyrimidine-1,6(2*H*)-dione (71 mg, 0.39 mmol) is dissolved in dry THF (2 mL), and a solution of LiHMDS (0.43 mmol, 0.43 mL of 1M THF solution) is added dropwise at 0 °C. The mixture is stirred for 1 h at rt and then added to a suspension of *N*-(2-bromo-3-(dimethylamino)allylidene)-*N*-methylmethanaminium hexafluorophosphate (150 mg, 0.43 mmol) in dry THF (0.5 mL) at -20 °C. The resulting mixture is stirred for 1 h at -20 °C, then AcOH (90 mg, 1.17 mmol) is added and the cooling bath is removed. After 1 h, ammonium acetate (90 mg, 1.17 mmol) is added, and the mixture is heated at reflux for 3 h. The reaction mixture is then cooled, diluted with brine, extracted with DCM, the organic phase is separated, dried over anhydrous Na₂SO₄, filtred and evaporated. The residue is purified by flash column chromatography on silica gel to give 3-bromo-8-methyl-9,10,10a,11-tetrahydro-5*H*-pyrimido[1,6-*g*][1,6]naphthyridin-7(8*H*)-one (14 mg, 12%), which is coupled with ethynylbenzene according to General Procedure 1 to provide the title compound.
¹H NMR (CDCl₃), δ_{H}, 1.95-2.06 (m. 1 H), 2.45 (dq, 1 H), 2.90-2.97 (m, 2H), 3.00 (s, 3H), 3.08-3.17 (m, 1 H), 3.23-3.28 (m, 1 H), 3.51 (td, 1 H), 4.64 (dd, 1 H), 4.86 (dd, 1 H), 7.35-7.38 (m, 3H), 7.52-7.55 (m, 2H), 7.62 (d, 1 H), 8.60 (d, 1 H).
LC/MS (M+H)⁺ = 318

### Example 58

### (rac)-Methyl 3-(phenylethynyl)-6,7,8,9-tetrahydro-5H-5,8-epiminocyclohepta[b]pyridine-10-carboxylate

According to General Procedure 5, methyl 3-oxo-8-azabicyclo[3.2.1]octane-8-carboxylate (accessible by acylation of 8-azabicyclo[3.2.1]octan-3-one with methyl chloroformate or as described by Kamogawa et al., Heterocycles 2010, 82, 325-332)
(250 mg, 1.365 mmol) is reacted with *N*-(2-bromo-3-(dimethylamino)allylidene)-*N-*methylmethanaminium hexafluorophosphate, and the crude product is purified by flash column chromatography (silica, gradient 20% to 70% EtOAc/heptane) to provide the intermediate product methyl 3-(phenylethynyl)-6,7,8,9-tetrahydro-5*H*-5,8-epiminocyclohepta-[*b*]pyridine-10-carboxylate (179 mg, 44%) as a yellow oil.

According to General Procedure 1, methyl 3-(phenylethynyl)-6,7,8,9-tetrahydro-5*H*-5,8-epiminocyclohepta-[*b*]pyridine-10-carboxylate (89 mg, 0.30 mmol) is reacted with ethynylbenzene (0.066 mL, 0.599 mmol) in the presence of PdCl₂[PPh₃]₂ (10.51 mg, 0.015 mmol), Cul (2.85 mg, 0.015 mmol) and TEA (0.167 mL, 1.198 mmol) in dry DMF (2 mL) at 100 °C for 1 h. The crude product is purified by flash column chromatography (Grace pre-packed column, 12 g, gradient 20% to 75% EtOAc/heptane) provides the title compound (82 mg, 86%) as a sticky orange foam.
¹H NMR (DMSO-d₆), δ_{H}, 1.65-1.75 (m, 1 H), 1.81-1.86 (m, 1 H), 2.08-2.29 (m, 2H), 2.77 (d, 1 H), 3.23-3.29 (m, 1 H), 3.58 (br s, 3H), 4.52-4.57 (m, 1 H), 5.04 (d, 1 H), 7.43-7.47 (m, 3H), 7.60-7.65 (m, 2H), 7.83-7.85 (m, 1 H), 8.54-8.56 (m, 1 H).
LC/MS (M+H)⁺ = 319

### Example 59

### (rac)-Methyl 3-((3-fluorophenyl)ethynyl)-6,7,8,9-tetrahydro-5H-5,8-epiminocyclohepta[b]pyridine-10-carboxylate

According to General Procedure 1, methyl 3-bromo-6,7,8,9-tetrahydro-5*H*-5,8-epiminocyclohepta[*b*]pyridine-10-carboxylate (for preparation see **Example 58**) (89 mg, 0.300 mmol) is reacted with 1-ethynyl-3-fluorobenzene (0.069 mL, 0.599 mmol) in the presence of PdCl₂[PPh₃]₂ (10.51 mg, 0.015 mmol), Cul (2.85 mg, 0.015 mmol) and TEA (0.167 mL, 1.198 mmol) in dry DMF (2 mL) at 100 °C for 1 h. The crude product is purified by flash column chromatography (Grace pre-packed column, 12 g, gradient 20% to 75% EtOAc/heptane) provides the title compound (64 mg, 64%) as a sticky orange foam.
¹H NMR (DMSO-d₆), δ_{H}, 1.65-1.75 (m, 1 H), 1.80-1.88 (m, 1 H), 2.09-2.29 (m, 2H), 2.78 (d, 1 H), 3.22-3.30 (m, 1 H), 3.58 (br s, 3H), 4.52-4.57 (m, 1 H), 5.05 (d, 1 H), 7.24-7.35 (m, 1 H), 7.40-7.54 (m, 3H), 7.84-7.87 (m, 1 H), 8.55-8.58 (m, 1 H).
LC/MS (M+H)⁺ = 337

### Example 60

### (rac)-cis-Methyl 3-(phenylethynyl)-7,8,9,9a-tetrahydro-5H-cyclopenta[h][1,6]naphthyridine-6(6aH)-carboxylate

A suspension of 1-benzylhexahydro-1*H*-cyclopenta[*b*]pyridin-4(4a*H*)-one hydrochloride (1.006 g, 3.79 mmol) in EtOH (20 mL) is purged with nitrogen (3x), after which palladium (10% on activated carbon, 0.403 g, 0.379 mmol) is added. The black suspension is flushed with hydrogen (3 evacuate/refill cycles) and stirred at rt under hydrogen (balloon) for 18 h, followed by 3 h at 40 °C. The reaction mixture is flushed with nitrogen for 30 min and filtered over kieselguhr. The filter cake is rinsed with EtOH (2x50 mL). The filtrate is concentrated, and the residue is dissolved in 5% MeOH/DCM (95:5) and dried over Na₂SO₄. Filtration and concentration gives an off-white foam, which is stripped with Et₂O (3x50 mL) to afford 785 mg of a sticky foam. This is dissolved in DCM (20 mL) and cooled in an ice bath. To the chilled solution is added TEA (1.87 mL, 13.4 mmol) and dropwise methyl chloroformate (0.519 mL, 6.70 mmol). The cooling bath is removed, and the slightly turbid yellow solution is stirred for one hour while reaching rt. The reaction mixture is diluted with DCM (50 mL) and washed with a saturated aqueous NaHCO₃ solution (50 mL) and brine (50 mL). The organic phase is dried over Na₂SO₄, filtered and concentrated to give a yellow oil. Purification by flash column chromatography (silica, gradient 5% to 25% EtOAc/heptane) affords the intermediate product rac-*cis* methyl 4-oxooctahydro-1*H*-cyclopenta[b]pyridine-1-carboxylate (398 mg, 53% over two steps) as a light yellow oil.

According to General Procedure 5, methyl 4-oxooctahydro-1*H-*cyclopenta[*b*]pyridine-1-carboxylate (398 mg, 2.018 mmol) is reacted with *N*-(2-bromo-3-(dimethylamino)allylidene)-*N*-methylmethanaminium hexafluorophosphate, and the crude product is purified by flash column chromatography (silica, gradient 2% to 20% EtOAc/heptane) to provide the intermediate product methyl 3-bromo-7,8,9,9a-tetrahydro-5*H*-cyclopenta[*h*][1,6]naphthyridine-6(6a*H*)-carboxylate (155 mg, 24%) as a yellow oil.

According to General Procedure 1, 3-bromo-7,8,9,9a-tetrahydro-5*H-*cyclopenta[*h*][1,6]naphthyridine-6(6a*H*)-carboxylate (77 mg, 0.247 mmol) is reacted with ethynylbenzene (50.5 mg, 0.495 mmol) in the presence of PdCl₂[PPh₃]₂ (8.7 mg, 0.012 mmol), Cul (1.9 mg, 9.9 µmol) and TEA (0.138 mL, 0.990 mmol) in dry DMF (3 mL) at 100 °C for 2 h. The crude product is purified by flash column chromatography (silica, first gradient 2% to 20% DME/heptane, followed by gradient 2% to 20% EtOAc/heptane) to provide the title compound (35 mg, 42%) as a colorless viscous oil.
¹H NMR (DMSO-d₆), δ_{H}, 1.48-1.60 (m, 3H), 1.83-1.99 (m, 2H), 2.04-2.13 (m, 1 H), 3.36 (m, 1 H), 3.64 (s, 3H), 4.28 (d, 1 H), 4.73 (br m, 1 H), 4.88 (d, 1 H), 7.43-7.48 (m, 3H), 7.48-7.60 (m, 2H), 7.87 (d, 1 H), 8.61 (d, 1 H).
LC/MS (M+H)⁺ = 333

### Example 61

### (rac)-cis-Methyl 3-((3-fluorophenyl)ethynyl)-7,8,9,9a-tetrahydro-5H-cyclopenta[h][1,6]naphthyridine-6(6aH)-carboxylate

According to General Procedure 1, rac-cis 3-bromo-7,8,9,9a-tetrahydro-5*H-*cyclopenta[*h*][1,6]naphthyridine-6(6a*H*)-carboxylate (for preparation see **Example 60**) (77 mg, 0.247 mmol) is reacted with 1-ethynyl-3-fluorobenzene (59.4 mg, 0.495 mmol) in the presence of PdCl₂[PPh₃]₂ (8.7 mg, 0.012 mmol), Cul (1.9 mg, 9.9 µmol) and TEA (0.138 mL, 0.990 mmol) in dry DMF (3 mL) at 100 °C for 2 h. The crude product is purified by flash column chromatography (silica, gradient 2% to 20% DME/heptane) to provide the title compound (62 mg, 71 %) as a colorless viscous oil.
¹H NMR (DMSO-d₆), δ_{H}, 1.48-1.60 (m, 3H), 1.83-1.99 (m, 2H), 2.05-2.13 (m, 1 H), 3.36 (m, 1 H), 3.64 (s, 3H), 4.25 (d, 1 H), 4.73 (br m, 1 H), 4.88 (d, 1 H), 7.29-7.34 (m, 1 H), 7.41-7.52 (m, 3H), 7.88 (d, 1 H), 8.63 (d, 1 H).

### LC/MS (M+H)⁺ = 351

### Example 62

### (rac)-cis-Methyl 3-(phenylethynyl)-6a,7,8,9,10,10a-hexahydrobenzo[h][1,6]naphthyridine-6(5H)-carboxylate

To a chilled (ice bath) solution of octahydroquinolin-4(1*H*)-one (947 mg, 6.18 mmol) in DCM (25 mL) is added TEA (1.72 mL, 12.4 mmol) and dropwise methyl chloroformate (0.72 mL, 9.3 mmol). The yellow solution is allowed to reach rt and stirred for 18 h. The reaction mixture is diluted with DCM (50 mL) and washed with brine (50 mL). The organic phase is dried over Na₂SO₄, filtered and concentrated to give rac-trans methyl 4-oxooctahydroquinoline-1 (2*H*)-carboxylate (1.28 g, 94%) as a yellow oil, which is used in the next step without further purification.

To a solution of *trans*-methyl 4-oxooctahydroquinoline-1(2*H*)-carboxylate (510 mg, 2.41 mmol) in MeOH (11 mL) is added K₂CO₃ (1.00 g, 7.24 mmol), and the light yellow suspension is stirred at rt for 3 days. The reaction mixture is filtered and concentrated, and the residue is partitioned between DCM (50 mL) and water (50 mL). The layers are separated, and the aqueous phase is extracted with additional DCM (2x50 mL). The combined organic fractions are washed with brine (100 mL), dried over Na₂SO₄, filtered and concentrated *in vacuo.* Purification by flash column chromatography (silica, gradient 2% to 20% DME/heptane) affords rac-*cis* methyl 4-oxooctahydroquinoline-1 (2*H*)-carboxylate (372 mg, 72%) as a colorless oil.

According to General Procedure 5, methyl 4-oxooctahydroquinoline-1 (2*H*)-carboxylate (372 mg, 1.76 mmol) is reacted with *N*-(2-bromo-3-(dimethylamino)allylidene)-*N*-methylmethanaminium hexafluorophosphate, and the crude product is purified by flash column chromatography (silica, gradient 2% to 20% EtOAc/heptane)) to provide the intermediate product rac-*cis* methyl 3-bromo-6a,7,8,9,10,10a-hexahydrobenzo[*h*][1,6]naphthyridine-6(5*H*)-carboxylate (176 mg, 26%, corrected for the purity of 87%) as a colorless oil.

According to General Procedure 1, 3-bromo-6a,7,8,9,10,10a-hexahydrobenzo[*h*][1,6]naphthyridine-6(5*H*)-carboxylate (92 mg, 0.28 mmol) is reacted with ethynylbenzene (0.062 mL, 0.57 mmol) in the presence of PdCl₂[PPh₃]₂ (9.9 mg, 0.014 mmol), Cul (2.2 mg, 0.011 mmol) and TEA (0.158 mL, 1.13 mmol) in dry DMF (3 mL) at 100 °C for 2 h. The crude product is purified by flash column chromatography (silica, first gradient 2% to 20% EtOAc/heptane, followed by a second gradient of 2% to 20% DME/heptane) to provide the title compound (55 mg, 56%) as a light yellow viscous oil.
¹H NMR (DMSO-d₆), δ_{H}, 0.83-0.94 (m, 1 H), 1.08-1.40 (m, 3H), 1.55-1.62 (m, 3H), 2.87 (br d, 1 H), 3.22 (br s, 1 H), 3.67 (s, 3H), 4.26-4.49 (br m, 2H), 4.81 (d, 1 H), 7.45-7.47 (m, 3H), 7.56-7.70 (m, 2H), 7.83 (s, 1 H), 8.65 (d, 1 H).
LC/MS (M+H)⁺ = 347

### Example 63

### (rac)-cis-Methyl 3-((3-fluorophenyl)ethynyl)-6a,7,8,9,10,10a-hexahydrobenzo[h][1,6]naphthyridine-6(5H)-carboxylate

According to General Procedure 1, rac-cis 3-bromo-6a,7,8,9,10,10a-hexahydrobenzo[*h*][1,6]naphthyridine-6(5*H*)-carboxylate (for preparation see **Example 62**) (88 mg, 0.271 mmol) is reacted with 1-ethynyl-3-fluorobenzene (65 mg, 0.54 mmol) in the presence of PdCl₂[PPh₃]₂ (9.5 mg, 0.014 mmol), Cul (2.1 mg, 0.011 mmol) and TEA (0.151 mL, 1.08 mmol) in dry DMF (3 mL) at 100 °C for 2 h. The crude product is purified by flash column chromatography (silica, first gradient 2% to 20% EtOAc/heptane, followed by a second gradient of 2% to 20% DME/heptane) to provide the title compound (65 mg, 70%) as a light yellow viscous oil.
¹H NMR (DMSO-d₆), δ_{H}, 0.82-0.94 (m, 1 H), 1.06-1.40 (m, 3H), 1.52-1.65 (m, 3H), 2.86 (br d, 1 H), 3.22 (br s, 1 H), 3.67 (s, 3H), 4.29-4.49 (br m, 2H), 4.81 (d, 1 H), 7.29-7.35 (m, 1 H), 7.42-7.53 (m, 3H), 7.84 (s, 1 H), 8.66 (d, 1 H).
LC/MS (M+H)⁺ = 365

### Example 64

### (rac)-3-Propyl-3'-(m-tolylethynyl)-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with propan-1-amine to provide 3'-bromo-3-propyl-7',8'-dihydro-6'H-spiro[oxazolidine-5,5'-quinolin]-2-one, which is coupled with 1-ethynyl-3-methylbenzene according to General Procedure 1 to provide the title compound.
¹H NMR (CDCl₃), δ_{H}, 0.98 (t, 3H), 1.62 (m, 2H), 1.81-1.93 (m, 1 H), 2.10-2.25 (m, 3H), 2.35 (s, 3H), 2.97-3.03 (m, 2H), 3.25-3.38 (m, 2H), 3.58 (s, 2H), 7.15-7.37 (m, 4H), 7.85 (d, 1H), 8.62 (d, 1H).
LC/MS (M+H)⁺ = 361

### Example 65

### (rac)-cis-Azetidin-1-yl-(9-(phenylethynyl)-5,6-dihydro-2H-[1,4]oxazino[2,3-f]quinolin-4(3H,4aH,10bH)-yl)methanone

4-Nitrophenyl chloroformate (103 mg, 0.51 mmol) is added dropwise to a mixture of cis-9-(phenylethynyl)-3,4,4a,5,6,10b-hexahydro-2*H*-[1,4]oxazino[2,3-*f*]quinoline (135 mg, 0.46 mmol) and TEA (0.84 mL, 6.00 mmol) in dry THF (3 mL) at rt. The mixture is stirred at rt for 30 min. Then azetidine hydrochloride (300 mg, 3.19 mmol) is added. The reaction mixture is stirred at 50 °C for 4 h, allowed to stand at rt for 16 h, diluted with water and extracted with EtOAc (3x50 mL). The combined organic phases are washed with a saturated aqueous K₂CO₃ solution (3x30 mL), dried over Na₂SO₄ and concentrated at reduced pressure. The obtained residue is purified by column chromatography (silica gel, EtOAc/EtOH, 30:1) to provide the title compound (82 mg, 48%) as a brownish oil.
¹H NMR (DMSO-d₆), δ_{H}, 1.64-1.73 (m, 1 H), 2.10-2.22 (m, 2H), 2.31-2.43 (m, 1 H), 2.93-3.14 (m, 3H), 3.49 (d, 1 H), 3.58-3.68 (m, 1 H), 3.77-4.02 (m, 6H), 4.44 (d, 1 H), 7.42-7.49 (m, 3H), 7.55-7.62 (m, 2H), 7.86 (d, 1 H), 8.64 (d, 1 H).
LC/MS (M+H)⁺ = 374

### Example 66

### (rac)-3'-((3-Fluorophenyl)ethynyl)-3-propyl-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with propan-1-amine to provide 3'-bromo-3-propyl-7',8'-dihydro-6'H-spiro[oxazolidine-5,5'-quinolin]-2-one, which is coupled with 1-ethynyl-3-fluorobenzene according to General Procedure 1 to provide the title compound.
¹H NMR (CDCl₃), δ_{H}, 0.98 (t, 3H), 1.62 (m, 2H), 1.81-1.96 (m, 1 H), 2.04-2.26 (m, 3H), 2.98-3.03 (m, 2H), 3.25-3.38 (m, 2H), 3.58 (s, 2H), 7.02-7.12 (m, 1 H), 7.20-7.36 (m, 3H), 7.86 (d, 1H), 8.63 (d, 1H).
LC/MS (M+H)⁺ = 365

### Example 67

### (rac)-3'-((3-Fluorophenyl)ethynyl)-3-phenyl-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with aniline to provide 3'-bromo-3-phenyl-7',8'-dihydro-6'*H-*spiro[oxazolidine-5,5'-quinolin]-2-one, which is coupled with 1-ethynyl-3-fluorobenzene according to General Procedure 1. Purification of the crude product by flash column chromatography provides the title compound as a colorless solid.
¹H NMR (CDCl₃), δ_{H}, 1.86-2.05 (m, 2H), 2.19-2.31 (m, 2H), 3.06 (t, 2H), 4.09 (AB m, 2H), 7.00-7.45 (m, 7H), 7.59 (d, 2H), 7.93 (d, 1 H), 8.66 (d, 1 H).
LC/MS (M+H)⁺ = 399

### Example 68

### 3-(m-Tolylethynyl)-7,8-dihydro-5H-thiopyrano[4,3-b]pyridine 6,6-dioxide

To a solution of tetrahydro-thiopyran-4-one (1.00 g, 9.0 mmol) in dry THF (25 mL) is added a solution of LiHMDS (1M in THF, 13 mL, 13 mmol) at -10 °C. The reaction mixture is stirred while warming to rt for 60 min. Then *N*-(2-bromo-3-(dimethylamino)allylidene)-*N*-methylmethanaminium hexafluorophosphate (3.32 g, 10 mmol) is added. The reaction mixture is stirred at rt for 30 min, then transferred to a mixture of NH₄OAc (3.31 g, 43.0 mmol), AcOH (2.46 mL, 43.0 mmol) and THF (1mL). The reaction mixture is then heated to reflux for 4h, then allowed to cool to rt and partitioned between Et₂O and saturated aqueous NaHCO₃ solution. The layers are separated, and the aqueous layer is extracted with additional Et₂O. The combined organic layers are washed with brine, dried over Na₂SO₄ and concentrated. The residue is purified by flash column chromatography, to give 0.67 g (34 %) of 3-bromo-7,8-dihydro-5H-thiopyrano[4,3-b]pyridine as a brown oil.

To a solution of 3-bromo-7,8-dihydro-5H-thiopyrano[4,3-b]pyridine (0.66 g, 2.87 mmol) in DCM (40 mL) is added mCPBA (1.42 g, 5.74 mmol) and the resulting mixture is stirred for 18h at rt The reaction mixture is quenched with water, extracted with ethyl acetate and the organic phase is dried over anhydrous Na₂SO₄, filtered and evaporated. The residue is purified by flash column chromatography to give 0.417 g (55 %) of 3-bromo-7,8-dihydro-5H-thiopyrano[4,3-b]pyridine 6,6-dioxide as a white powder. 3-Bromo-7,8-dihydro-5H-thiopyrano[4,3-b]pyridine 6,6-dioxide is coupled with 1-ethynyl-3-methylbenzene according to General Procedure 1 to provide the title compound.
¹H NMR (CDCl₃), δ_{H}, 2.36 (s, 3H), 3.42 (t, 2H), 3.62 (t, 2H), 4.30 (s, 2H), 7.17-7.36 (m, 4H), 7.55 (d, 1H), 8.66 (d, 1H).
LC/MS (M+H)⁺ = 298

### Example 69

### 3-((3-Fluorophenyl)ethynyl)-7,8-dihydro-5H-thiopyrano[4,3-b]pyridine 6,6-dioxide

3-Bromo-7,8-dihydro-5H-thiopyrano[4,3-b]pyridine 6,6-dioxide (**Example 68**) is coupled with 1-ethynyl-3-fluorobenzene according to General Procedure 1 to provide the title compound.
¹H NMR (CDCl₃), δ_{H}, 3.43 (t, 2H), 3.63 (t, 2H), 4.30 (s, 2H), 7.03-7.36 (m, 4H), 7.57 (d, 1 H), 8.67 (d, 1H).
LC/MS (M+H)⁺ = 302

### Example 70

### 3-((4-Fluorophenyl)ethynyl)-7,8-dihydro-5H-thiopyrano[4,3-b]pyridine 6,6-dioxide

3-Bromo-7,8-dihydro-5H-thiopyrano[4,3-b]pyridine 6,6-dioxide (**Example 68**) is coupled with 1-ethynyl-4-fluorobenzene according to General Procedure 1 to provide the title compound.
¹H NMR (CDCl₃), δ_{H}, 3.43 (t, 2H), 3.63 (t, 2H), 4.30 (s, 2H), 7.06 (t, 2H), 7.48-7.55 (m, 3H), 8.65 (d, 1H).
LC/MS (M+H)⁺ = 302

### Example 71

### (6aR,9aR)-Methyl 3-(phenylethynyl)-6,6a,9,9a-tetrahydro-5H-pyrrolo[2,3-h]quinoline-7(8H)-carboxylate

Aziridine (16.9 g, 0.39 mol) is dissolved in MeOH (500 mL) and treated with cyclohex-2-enone (45.4 g, 0.47 mol) at rt. Copper (25.0 g, 0.39 mol) is added, and the solution is stirred for 3 h, filtered and evaporated to dryness.The crude intermediate product is purified by column chromatography (silica gel, petrol ether/EtOAc 1:1) to give 3-(aziridin-1-yl)cyclohexanone (39.3 g, 72%) as a yellow oil.

3-(Aziridin-1-yl)cyclohexanone (22.4 g, 0.18 mol) is dissolved in dry toluene (130 mL) and cooled down to 0 °C. To the clear solution is added alkyl chloroformate (0.18 mol) within 30 min. The temperature is kept between 0-10 °C. Then the solution is warmed up to rt and stirred for 1 h. Solvent and excess of reagents are removed in *vacuo* to yield methyl (2-chloroethyl)(3-oxocyclohexyl)carbamate (40.1 g, 98%) as a red oil.

To the solution of methyl (2-chloroethyl)(3-oxocyclohexyl)carbamate (40.1 g, 0.17 mol) in DCM (170 mL) is added pyrrolidine (12.2 g, 0.17 mmol) and TEA (17.4 g, 0.17 mmol). The reaction mixture is stirred overnight at rt and finally diluted with isopropyl acetate (510 mL) and water (340 mL). Under stirring the pH is adjusted to 2 and stirring is continued for further 30 min. After phase separation the organic phase is washed three times with water (200 mL), dried over Na₂SO₄, filtered and evaporated to furnish methyl 4-oxooctahydro-1*H*-indole-1-carboxylate (23.8 g, 70%, crude product) as a red oil. The obtained isomers are separated by means of chiral HPLC (column: CHIRALPAK AD-H, 0.46 cm A.D. x 15 cm L, mobile phase: hexane / isopropanol / MeOH 90:7.5:2.5 (v/v/v), flow rate = 1.0 mL/min).
Peak 1 ((S, S)-isomer), Rt = 6.8 min
Peak 2 ((R, R)-isomer), Rt = 8.9 min (see also WO-2010/018154)

According to General Procedure 5, (3a*R*,7a*R*)-methyl 4-oxooctahydro-1*H-*indole-1-carboxylate (284 mg, 1.44 mmol) is reacted with *N*-(2-bromo-3-(dimethylamino)allylidene)-*N*-methylmethanaminium hexafluorophosphate, and the crude product is purified by flash column chromatography (silica, gradient 3% to 25% EtOAc/heptane) to give (6a*R*,9a*R*)-methyl 3-bromo-6,6a,9,9a-tetrahydro-5*H*-pyrrolo[2,3-h]quinoline-7(8*H*)-carboxylate (83 mg, 18%) as a light yellow oil.

According to General Procedure 1, (6a*R*,9a*R*)-methyl 3-bromo-6,6a,9,9a-tetrahydro-5*H*-pyrrolo[2,3-h]quinoline-7(8*H*)-carboxylate (47 mg, 0.151 mmol) is reacted with ethynylbenzene (0.033 mL, 0.30 mmol) in the presence of PdCl₂[PPh₃]₂ (5.3 mg, 7.6 µmol), Cul (1.2 mg, 6.0 µmol) and TEA (0.084 mL, 0.60 mmol) in dry DMF (3 mL) at 100 °C for 16 h. The crude product is purified by flash column chromatography (silica, gradient 2% to 20% EtOAc/heptane) to provide the title compound (29 mg, 57%) as a brown waxy solid.
¹H NMR (CDCl₃), δ_{H}, 1.61-1.74 (m, 1 H), 1.94-1.98 (m, 1 H), 2.13-2.34 (m, 1 H), 2.47-2.55 (m, 1 H), 2.71-2.78 (m, 2H), 3.43-3.53 (m, 3H), 3.72 (s, 3H), 4.08-4.26 (m, 1 H), 7.33-7.37 (m, 3H), 7.50-7.56 (m, 3H), 8.56 (d, 1 H).
LC/MS (M+H)⁺ = 333

### Example 72

### (6aR,9aR)-Methyl 3-((3-fluorophenyl)ethynyl)-6,6a,9,9a-tetrahydro-5H-pyrrolo[2,3-h]quinoline-7(8H)-carboxylate

According to General Procedure 1 and the procedure described in **Example 71**, (6a*R*,9a*R*)-methyl 3-bromo-6,6a,9,9a-tetrahydro-5*H*-pyrrolo[2,3-h]quinoline-7(8*H*)-carboxylate (34 mg, 0.109 mmol, for preparation see **Example 71**) is reacted with 1-ethynyl-3-fluorobenzene (2 eq.) to provide the title compound (26 mg, 67%) as a brown sticky solid.
¹H NMR (CDCl₃), δ_{H}, 1.61-1.74 (m, 1 H), 1.91-2.05 (m, 1 H), 2.12-2.36 (m, 1 H), 2.46-2.56 (m, 1 H), 2.71-2.79 (m, 2H), 3.40-3.57 (m, 3H), 3.72 (s, 3H), 4.08-4.26 (m, 1 H), 7.03-7.09 (m, 1 H), 7.19-7.24 (m, 1 H), 7.28-7.36 (m, 2H), 7.54 (br d, 1 H), 8.55 (d, 1 H). LC/MS (M+H)⁺ = 351

### Example 73

### (rac)-cis-Methyl 3-((3-fluorophenyl)ethynyl)-5a,6,8,8a-tetrahydropyrrolo[3',4':4,5]cyclopenta[1,2-b]pyridine-7(5H)-carboxylate

A mixture of *tert*-butyl 4-oxohexahydrocyclopenta[c]pyrrole-2(1*H*)-carboxylate (4.63 g, 20.57 mmol) and trifluoroacetic acid (20 mL, 269 mmol) in DCM (20 mL) is stirred at rt for 1 h. The solvent is removed under reduced pressure, and the residue is co-evaporated with DCM (3x20 mL). The crude product is dissolved in DCM (100 mL). TEA (35 mL, 252 mmol) is then added at 0 °C, followed by the dropwise addition of methyl chloroformate (2.4 mL, 31.1 mmol), resulting in the formation of a white precipitate. After stirring for additional 10 min at 0 °C the mixture is allowed to warm to rt overnight. The mixture is concentrated *in vacuo* and dissolved in DCM (100 mL). The organic layer is washed with a mixture of saturated aqueous NaHCO₃ solution and water (1:1, 100 mL). The aqueous layer is then extracted with DCM (2x75 mL). The organic layers are combined, washed with brine (100 mL), dried over Na₂SO₄ and concentrated *in vacuo.* Purification by flash column chromatography (silica, gradient 40% EtOAc/heptane to 100% EtOAc) yields methyl 4-oxohexahydrocyclopenta[c]pyrrole-2(1*H*)-carboxylate (1.23 g, 32% over 2 steps) of the title compound as a yellow oil, which crystallized upon standing.

According to General Procedure 5, methyl 4-oxohexahydrocyclopenta[*c*]pyrrole-2(1*H*)-carboxylate (226 mg, 1.234 mmol) is reacted with *N*-(2-bromo-3-(dimethylamino)allylidene)-*N*-methylmethanaminium hexafluorophosphate, and the crude product is purified by flash column chromatography (silica, gradient 100% heptane to 75% EtOAc/heptane) to give the intermediate product methyl 3-bromo-5a,6,8,8a-tetrahydropyrrolo[3',4':4,5]cyclopenta[1,2-*b*]pyridine-7(5*H*)-carboxylate (60 mg, 16%) as a white solid.

According to General Procedure 1, methyl 3-bromo-5a,6,8,8a-tetrahydropyrrolo[3',4':4,5]cyclopenta[1,2-b]pyridine-7(5*H*)-carboxylate (85 mg, 0.286 mmol) is reacted with 1-ethynyl-3-fluorobenzene (0.066 mL, 0.572 mmol) in the presence of PdCl₂[PPh₃]₂ (10.04 mg, 0.014 mmol), Cul (2.179 mg, 0.011 mmol) and TEA (0.159 mL, 1.144 mmol) in dry DMF (3 mL) at 100 °C for 16 h. The crude product is purified by flash column chromatography (silica, gradient 10% to 70% EtOAc/heptane) to provide the title compound (27.5 mg, 28%).
¹H NMR (DMSO-d₆), δ_{H}, 2.77-2.86 (m, 2H), 3.10-3.23 (m, 2H), 3.52 (s, 3H), 3.64-3.84 (m, 4H), 7.28-7.35 (m, 1 H), 7.40-7.53 (m, 3H), 7.85-7.88 (m, 1 H), 8.58-8.62 (m, 1 H). LC/MS (M+H)⁺ = 337

### Example 74

### (rac)-3-Cyclopropyl-3'-(phenylethynyl)-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with cyclopropanamine to provide 3'-bromo-3-cyclopropyl-7',8'-dihydro-6'*H*-spiro[oxazolidine-5,5'-quinolin]-2-one, which is coupled with ethynylbenzene according to General Procedure 1 to provide the title compound.
¹H NMR (CDCl₃), δ_{H}, 0.78-0.85 (m, 4H), 1.78-1.92 (m, 1 H), 2.09-2.20 (m, 3H), 2.64 (m, 1 H), 2.98 (t, 2H), 3.57 (AB m, 2H), 7.35-7.38 (m, 3H), 7.52-7.57 (m, 2H), 7.83 (d, 1 H), 8.63 (d, 1H).
LC/MS (M+H)⁺ = 345

### Example 75

### 5,5-Dimethyl-3-(phenylethynyl)-7,8-dihydro-5H-thiopyrano[4,3-b]pyridine 6,6-dioxide

To a stirred solution of 3-bromo-7,8-dihydro-5H-thiopyrano[4,3-*b*]pyridine 6,6-dioxide (**Example 68**), (0.10 g, 0.38 mmol) and iodomethane (0.22 g, 1.53 mmol) in THF (3 mL) is added LiHMDS solution (1M in THF, 0.84 mL, 0.84 mmol) over 5 minutes at -78°C. The resulting orange solution is stirred for 16h, while slowly warming to ambient temperature. The reaction mixture is quenched with sat. NH₄Cl solution (3 drops), diluted with chloroform (50 mL), dried over anhydrous Na₂SO₄, filtered and evaporated. The residue is purified by preparative HPLC to give 35 mg (32 %) of 3-bromo-5,5-dimethyl-7,8-dihydro-5*H*-thiopyrano[4,3-*b*]pyridine 6,6-dioxide and 30 mg (26%) of 3-bromo-5,5,7-trimethyl-7,8-dihydro-5*H*-thiopyrano[4,3-*b*]pyridine 6,6-dioxide both as a white powders.

3-Bromo-5,5-dimethyl-7,8-dihydro-5*H*-thiopyrano[4,3-*b*]pyridine 6,6-dioxide is coupled with ethynylbenzene according to General Procedure 1 to provide the title compound.
¹H NMR (CDCl₃), δ_{H}, 1.78 (s, 6H), 3.46 (t, 2H), 3.64 (t, 2H), 7.35-7.42 (m, 3H), 7.53-7.58 (m, 2H), 7.74 (d, 1 H), 8.63 (d, 1 H).
LC/MS (M+H)⁺ = 312

### Example 76

### (rac)-3-Cyclopropyl-3'-((3-fluorophenyl)ethynyl)-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with cyclopropanamine to provide 3'-bromo-3-cyclopropyl-7',8'-dihydro-6'*H*-spiro[oxazolidine-5,5'-quinolin]-2-one, which is coupled with 1-ethynyl-3-fluorobenzene according to General Procedure 1 to provide the title compound.
¹H NMR (CDCl₃), δ_{H}, 0.78-0.86 (m, 4H), 1.79-1.94 (m, 1 H), 2.09-2.20 (m, 3H), 2.65 (m, 1 H), 2.98 (t, 2H), 3.57 (AB m, 2H), 7.03-7.14 (m, 1 H), 7.21-7.40 (m, 3H), 7.83 (d, 1 H), 8.63 (d, 1H).
LC/MS (M+H)⁺ = 363

### Example 77

### Ethyl 2-oxo-2-(3-(phenylethynyl)-7,8-dihydro-1,6-naphthyridin-6(5H)-yl)acetate

According to General Procedure 2, 3-(phenylethynyl)-5,6,7,8-tetrahydro-1,6-naphthyridine is acylated with ethyl 2-chloro-2-oxoacetate to provide the title compound as a colorless solid.
¹H NMR (CDCl₃), δ_{H}, 1.31-1.34 (m, 3H), 3.02-3.09 (m, 2H), 3.73 and 3.92 (both t, 2H in total), 4.28-4.34 (m, 2H), 4.61 and 4.71 (both s, 2H in total), 7.29-7.32 (m, 3H), 7.44-7.51 (m, 2H), 7.53 (d, 1 H), 8.54 (d, 1 H).
LC/MS (M+H)⁺ = 335

### Example 78

### 6-((4-Methylpiperazin-1-yl)sulfonyl)-3-(phenylethynyl)-5,6,7,8-tetrahydro-1,6-naphthyridine

According to General Procedure 2, 3-(phenylethynyl)-5,6,7,8-tetrahydro-1,6-naphthyridine is acylated with 4-methylpiperazine-1-sulfonyl chloride to provide the title compound as a colorless solid.
¹H NMR (CDCl₃), δ_{H}, 2.28 (s, 3H), 2.44 (m, 4H), 3.06 (t, 2H), 3.28 (m, 4H), 3.59 (t, 2H), 4.39 (s, 2H), 7.29-7.32 (m, 3H), 7.44-7.51 (m, 3H), 8.54 (d, 1 H).
LC/MS (M+H)⁺ = 397

### Example 79

### (rac)-Methyl 3-(phenylethynyl)-5a,6,8,8a-tetrahydropyrrolo[3',4':4,5]cyclopenta[1,2-b]pyridine-7(5H)-carboxylate

According to General Procedure 1 and the procedure described in **Example 73**, methyl 3-bromo-5a,6,8,8a-tetrahydropyrrolo[3',4':4,5]cyclopenta[1,2-*b*]pyridine-7(5*H*)-carboxylate (60 mg, 0.202 mmol, for preparation see **Example 73**) is reacted with ethynylbenzene (2 eq.) to provide the title compound (47 mg, 30%) as an off-white solid. ¹H NMR (DMSO-d₆), δ_{H}, 2.76-2.87 (m, 2H), 3.09-3.24 (m, 2H), 3.53 (s, 3H), 3.64-3.84 (m, 4H), 7.42-7.48 (m, 3H), 7.55-7.60 (m, 2H), 7.83-7.87 (m, 1 H), 8.57-8.61 (m, 1 H). LC/MS (M+H)⁺ = 319

### Example 80

### (rac)-cis-Methyl 7-(phenylethynyl)-2,3,3a,4,5,9b-hexahydro-1H-pyrrolo[3,2-h]quinoline-1-carboxylate

In a 250 mL autoclave, 7-hydroxyindole (4.01 g, 30.1 mmol) is suspended in ethanol (96%) (75 mL) and water (25 mL). Rhodium (5 wt% on activated Al₂O₃) (1.25 g, 0.305 mmol, 0.01 eq) is added, and the black suspension is stirred under 50 bar of hydrogen pressure at 50 °C for 5 days. The reaction mixture is allowed to cool to rt and flushed with nitrogen for 30 min. The black suspension is filtered over kieselguhr, which is rinsed with 3 portions of EtOH (100 mL). The filtrate is concentrated, and the brown oil is dissolved in DCM (500 mL). The organic layer is washed with brine (500 mL), dried over Na₂SO₄, filtered and concentrated *in vacuo.* The residue is dissolved in DCM (100 mL) followed by the addition of TEA (4.40 mL, 31.8 mmol) and the dropwise addition of methyl chloroformate (1.72 mL, 22.2 mmol) (*caution,* slightly exothermic). The dark brown solution is stirred for 2 h at rt. The reaction mixture is diluted with DCM (100 mL) and successively washed with an aqueous 0.5 M KHSO₄ solution (100 mL) and brine (100 mL). The organic layer is dried over Na₂SO₄ and concentrated *in vacuo.* Purification by flash column chromatography (silica, gradient 6% to 60% EtOAc/heptane) affords methyl 7-hydroxyoctahydro-1*H*-indole-1-carboxylate (1.19 g, 19% over two steps) as a brown liquid.

Methyl 7-hydroxyoctahydro-1*H*-indole-1-carboxylate (1.19 g, 5.97 mmol) is dissolved in DCM (100 mL), followed by the addition of Dess-Martin periodinane (3.04 g, 7.17 mmol), and the dark brown solution is stirred at rt. After stirring for 30 min, 10% aqueous Na₂S₂O₃ solution (100 mL) is added, and the mixture is vigorously stirred for another 30 min. The layers are separated, and the organic layer is diluted with DCM (50 mL), washed with brine (100 mL), dried over Na₂SO₄ and concentrated *in vacuo.* Purification by flash column chromatography (gradient 7% to 70% EtOAc/heptane) provides methyl 7-oxooctahydro-1*H*-indole-1-carboxylate (0.92 g, 78%) as a light brown oil.

According to General Procedure 5, methyl 7-oxooctahydro-1*H*-indole-1-carboxylate (510 mg, 2.59 mmol) is reacted with *N*-(2-bromo-3-(dimethylamino)allylidene)-*N*-methylmethanaminium hexafluorophosphate, and the crude product is purified by flash column chromatography (silica, gradient 100% heptane to 100% EtOAc) to give the intermediate product methyl 7-bromo-2,3,3a,4,5,9b-hexahydro-1*H*-pyrrolo[3,2-*h*]quinoline-1-carboxylate (264 mg, 32%).

According to General Procedure 1, methyl 7-bromo-2,3,3a,4,5,9b-hexahydro-1*H*-pyrrolo[3,2-*h*]quinoline-1-carboxylate (120 mg, 0.386 mmol) is reacted with ethynylbenzene (0.085 mL, 0.771 mmol) in the presence of PdCl₂[PPh₃]₂ (13.53 mg, 0.019 mmol), Cul (2.94 mg, 0.015 mmol) and TEA (0.215 mL, 1.543 mmol) in dry DMF (6 mL) at 100 °C for 16 h. The crude product is purified by flash column chromatography (adsorbed onto hydromatrix, silica, gradient 100% heptane to 80% EtOAc/heptane) to provide the title compound (103 mg, 80%) as a brown paste.
¹H NMR (DMSO-d₆), δ_{H}, 1.48-1.62 (m, 1 H), 1.81-1.99 (m, 1 H), 2.05-2.19 (m, 1 H), 2.32-2.43 (m, 1 H), 2.70-2.79 (m, 2H), 3.35-3.42 (m, 2H, overlap with solvent peak), 3.43-3.53 (m, 1 H), 3.58-3.68 (m, 3H), 4.05-4.14 (m, 1 H), 7.42-7.47 (m, 3H), 7.54-7.60 (m, 2H), 7.73-7.76 (m, 1 H), 8.54-8.58 (m, 1 H).
LC/MS (M+H)⁺ = 333

### Example 81

### 6-(Isopropylsulfonyl)-3-(phenylethynyl)-5,6,7,8-tetrahydro-1,6-naphthyridine

According to General Procedure 2, 3-(phenylethynyl)-5,6,7,8-tetrahydro-1,6-naphthyridine is acylated with propane-2-sulfonyl chloride to provide the title compound as a colorless solid.
¹H NMR (CDCl₃), δ_{H}, 1.31 (d, 3H), 3.05 (t, 2H), 3.20 (m, 1 H), 3.67 (t, 2H), 4.48 (s, 2H), 7.29-7.32 (m, 3H), 7.46-7.51 (m, 3H), 8.54 (d, 1 H).
LC/MS (M+H)⁺ = 341

### Example 82

### (rac)-3'-(Phenylethynyl)-3-propyl-7',8'-dihydro-6'H-spiro[oxazolidine-5,5'-quinolin]-2-one

According to General Procedure 3, 3'-bromo-7',8'-dihydro-6'*H*-spiro[oxirane-2,5'-quinoline] is reacted with propan-1-amine to provide 3'-bromo-3-propyl-7',8'-dihydro-6'*H-*spiro[oxazolidine-5,5'-quinolin]-2-one, which is coupled with ethynylbenzene according to General Procedure 1 to provide the title compound.
¹H NMR (CDCl₃), δ_{H}, 0.97 (t, 3H), 1.62 (m, 2H), 1.81-1.94 (m, 1 H), 2.09-2.26 (m, 3H), 2.97-3.03 (m, 2H), 3.25-3.41 (m, 2H), 3.59 (s, 2H), 7.35-7.38 (m, 3H), 7.52-7.57 (m, 2H), 7.86 (d, 1H), 8.63 (d, 1H).
LC/MS (M+H)⁺ = 347

**Table 1**

| | | | | | | | |
|---|---|---|---|---|---|---|---|
| 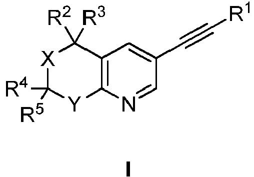 | | | | | | | |

| **Example No.** | **R¹** | **R³** | **R²** | **R⁴** | **R⁵** | **X** | **Y** |
|---|---|---|---|---|---|---|---|
| **1** | Ph | H | OMe | H | H | CH₂ | CH₂ |
| **2** | Ph | H | OMe | H | H | CH₂ | CH₂ |
| **3** | 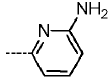 | H | OMe | H | H | CH₂ | CH₂ |
| **4** | Ph | H | OMe | Me | Me | CH₂ | CH₂ |
| **5** | 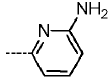 | H | OMe | Me | Me | CH₂ | CH₂ |
| **6** | 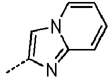 | H | OMe | Me | Me | CH₂ | CH₂ |
| **7** | 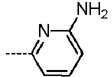 | H | OMe | Me | Me | CH₂ | O |
| **8** | 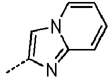 | H | OMe | Me | Me | CH₂ | O |
| **9** | Ph | H | OMe | H | H | CH₂ | NMe |
| **10** | Ph | 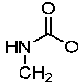 | | H | H | CH₂ | CH₂ |
| **11** | Ph | 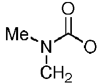 | | H | H | CH₂ | CH₂ |
| **12** | Ph | 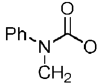 | | H | H | CH₂ | CH₂ |
| **33** | Ph | H | H | H | H | 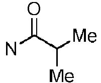 | CH₂ |
| **34** | Ph | H | H | H | H | 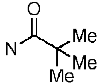 | CH₂ |
| **35** | 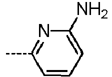 | H | H | H | H | 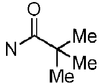 | CH₂ |
| **36** | Ph | H | H | H | H | 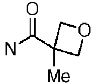 | CH₂ |
| **37** | Ph | H | H | H | H | 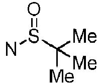 | CH₂ |
| **38** | Ph | H | H | H | H | 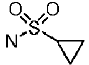 | CH₂ |
| **39** | Ph | H | H | H | H | 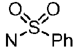 | CH₂ |
| **40** | Ph | H | H | H | H | 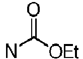 | CH₂ |
| **41** | Ph | H | H | H | H | 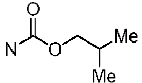 | CH₂ |
| **42** | Ph | H | H | H | H | 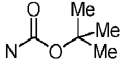 | CH₂ |
| **43** | Ph | H | H | H | H | 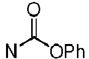 | CH₂ |
| **44** | Ph | H | H | H | H | 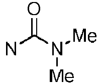 | CH₂ |
| **45** | 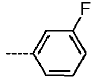 | H | H | H | H | 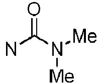 | CH₂ |
| **46** | 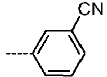 | H | H | H | H | 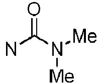 | CH₂ |
| **47** | 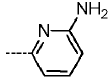 | H | H | H | H | 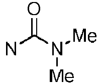 | CH₂ |
| **48** | 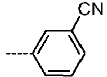 | H | H | H | H | 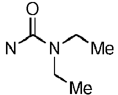 | CH₂ |
| **49** | Ph | H | H | H | H | 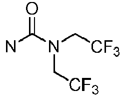 | CH₂ |
| **50** | Ph | H | H | H | H | 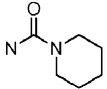 | CH₂ |
| **51** | 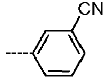 | H | H | H | H | 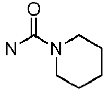 | CH₂ |
| **52** | Ph | H | H | H | H | 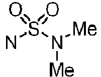 | CH₂ |
| **53** | Ph | H | H | H | H | 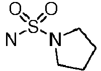 | CH₂ |
| **54** | Ph | H | H | H | H | 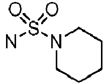 | CH₂ |
| **55** | Ph | H | H | H | H | 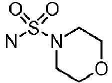 | CH₂ |
| **56** | Ph | H | H | H | H | 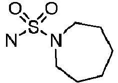 | CH₂ |
| **58** | Ph | H | CH₂CH₂ | | H | 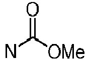 | CH₂ |
| **59** | 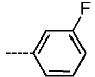 | H | CH₂CH₂ | | H | 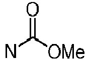 | CH₂ |
| **64** | 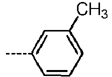 | 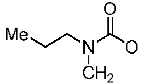 | | H | H | CH₂ | CH₂ |
| **66** | 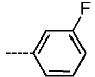 | 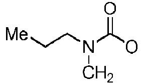 | | H | H | CH₂ | CH₂ |
| **67** | 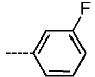 | 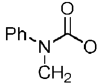 | | H | H | CH₂ | CH₂ |
| **68** | 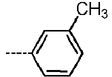 | H | H | H | H | SO₂ | CH₂ |
| **69** | 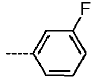 | H | H | H | H | SO₂ | CH₂ |
| **70** | 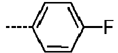 | H | H | H | H | SO₂ | CH₂ |
| **74** | Ph | 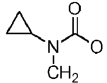 | | H | H | CH₂ | CH₂ |
| **75** | Ph | Me | Me | H | H | SO₂ | CH₂ |
| **76** | 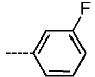 | 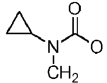 | | H | H | CH₂ | CH₂ |
| **77** | Ph | H | H | H | H | 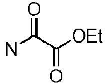 | CH₂ |
| **78** | Ph | H | H | H | H | 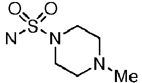 | CH₂ |
| **81** | Ph | H | H | H | H | 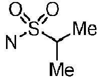 | CH₂ |
| **82** | Ph | 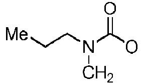 | | H | H | CH₂ | CH₂ |

**Table 2**

| | | | | | | | | |
|---|---|---|---|---|---|---|---|---|
| 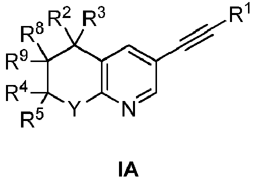 | | | | | | | | |

| **Example No.** | **R¹** | **R³** | **R²** | **R⁸** | **R⁴** | **R⁹** | **R⁵** | **Y** |
|---|---|---|---|---|---|---|---|---|
| **13** | Ph | H | 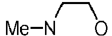 | | H | H | H | CH₂ |
| **14** | Ph | H | 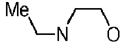 | | H | H | H | CH₂ |
| **15** | Ph | H | 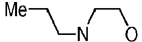 | | H | H | H | CH₂ |
| **16** | Ph | H | 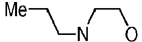 | | H | H | H | CH₂ |
| **17** | Ph | H | 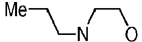 | | H | H | H | CH₂ |
| **18** | 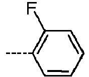 | H | 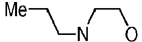 | | H | H | H | CH₂ |
| **19** | 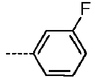 | H | 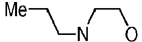 | | H | H | H | CH₂ |
| **20** | 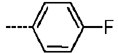 | H | 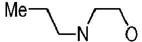 | | H | H | H | CH₂ |
| **21** | 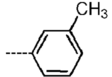 | H | 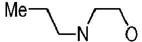 | | H | H | H | CH₂ |
| **22** | Ph | H | 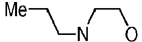 | | H | H | H | CH₂ |
| **23** | Ph | H | 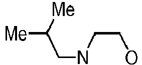 | | H | H | H | CH₂ |
| **24** | Ph | H | 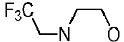 | | H | H | H | CH₂ |
| **25** | Ph | H | 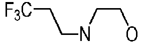 | | H | H | H | CH₂ |
| **26** | Ph | H | 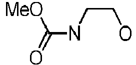 | | H | H | H | CH₂ |
| **27** | Ph | H | 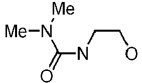 | | H | H | H | CH₂ |
| **29** | Ph | H | 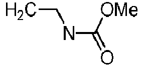 | | H | H | H | CH₂ |
| **30** | Ph | H | 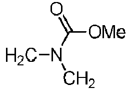 | | H | H | H | CH₂ |
| **31** | 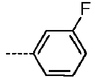 | H | 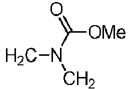 | | H | H | H | CH₂ |
| **73** | 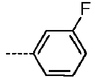 | H | H | 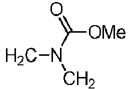 | | H | H | bond |
| **79** | Ph | H | H | 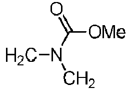 | | H | H | bond |

**Table 3**

| | | | | | | | | |
|---|---|---|---|---|---|---|---|---|
| 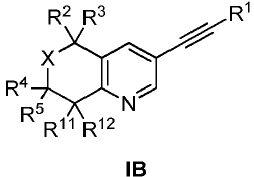 | | | | | | | | |

| **Example No.** | **R¹** | **R³** | **R²** | **R⁵** | **R⁴** | **R¹¹** | **R¹²** | **X** |
|---|---|---|---|---|---|---|---|---|
| **32** | 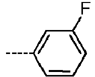 | H | H | H | 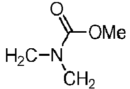 | | H | CH₂ |
| **60** | Ph | H | H | H | CH₂CH₂CH₂ | | H | 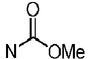 |
| **61** | 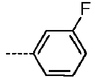 | H | H | H | CH₂CH₂CH₂ | | H | 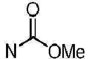 |
| **62** | Ph | H | H | H | CH₂CH₂CH₂CH₂ | | H | 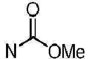 |
| **63** | 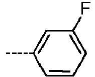 | H | H | H | CH₂CH₂CH₂CH₂ | | H | 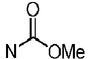 |
| **71** | Ph | H | H | H | 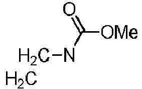 | | H | CH₂ |
| **72** | 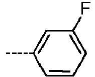 | H | H | H | 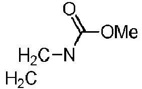 | | H | CH₂ |
| **80** | Ph | H | H | H | 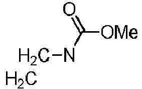 | | H | CH₂ |

**Table 4**

| | | | | | | | | |
|---|---|---|---|---|---|---|---|---|
| 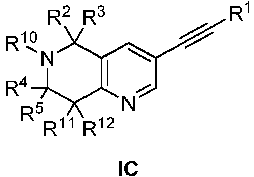 | | | | | | | | |

| **Example No.** | **R¹** | **R²** | **R³** | **R¹⁰** | **R⁴** | **R⁵** | **R¹¹** | **R¹²** |
|---|---|---|---|---|---|---|---|---|
| **57** | Ph | H | H | 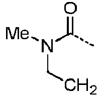 | | H | H | H |

**Table 5**

| | | | | | | |
|---|---|---|---|---|---|---|
| 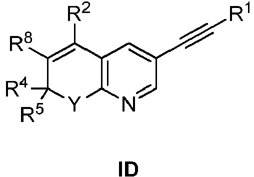 | | | | | | |

| **Example No.** | **R¹** | **R²** | **R⁸** | **R⁴** | **R⁵** | **Y** |
|---|---|---|---|---|---|---|
| **28** | Ph | 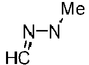 | | H | H | CH₂ |

### EXAMPLES OF REPRESENTATIVE PHARMACEUTICAL COMPOSITIONS

In combination with commonly used solvents, excipients, auxiliary agents and carriers, the instant compounds may be processed into tablets, coated tablets, capsules, drip solutions, suppositories, injection and infusion preparations, and the like and may be therapeutically applied by the oral, rectal, parenteral, and additional routes. Representative pharmaceutical compositions according to the present invention follow:
(a) Tablets suitable for oral administration which contain the active ingredient, may be prepared by conventional tabletting techniques.
(b) For suppositories, any usual suppository base may be employed for incorporation thereinto by usual procedure of the active ingredient, such as a polyethyleneglycol which is a solid at normal room temperature, but which melts at or about body temperature.
(c) For parenteral (including intravenous and subcutaneous) sterile solutions, the active ingredient together with conventional ingredients in usual amounts are employed, such as for example sodium chloride and double-distilled water q.s., according to conventional procedure, such as filtration, aseptic filling into ampoules or IV-drip bottles, and autoclaving for sterility.

Other suitable pharmaceutical compositions will be immediately apparent to one skilled in the art.

### FORMULATION EXAMPLES

The following examples are again given by way of illustration only and are not to be construed as limiting.

### EXAMPLE 1

### Tablet Formulation

A suitable formulation for a tablet containing 10 milligrams of active ingredient is as follows:

| | mg |
|---|---|
| Active Ingredient | 10 |
| Lactose | 61 |
| Microcrystalline Cellulose | 25 |
| Talcum | 2 |
| Magnesium stearate | 1 |
| Colloidal silicon dioxide | 1 |

### EXAMPLE 2

### Tablet Formulation

Another suitable formulation for a tablet containing 100 mg is as follows:

| | mg |
|---|---|
| Active Ingredient | 100 |
| Polyvinylpyrrolidone, crosslinked | 10 |
| Potato starch | 20 |
| Polyvinylpyrrolidone | 19 |
| Magnesium stearate | 1 |
| Microcrystalline Cellulose | 50 |
| Film coated and colored. | |

| **The film coating material consists of:** | |
|---|---|
| Hypromellose | 10 |
| Microcryst. Cellulose | 5 |
| Talcum | 5 |
| Polyethylene glycol | 2 |
| Color pigments | 5 |

### EXAMPLE 3

### Capsule Formulation

A suitable formulation for a capsule containing 50 milligrams of active ingredient is as follows:

| | mg |
|---|---|
| Active Ingredient | 50 |
| Corn starch | 26 |
| Dibasic calcium phosphate | 50 |
| Talcum | 2 |
| Colloidal silicon dioxide | 2 |

filled in a gelatin capsule.

### EXAMPLE 4

### Solution for injection

A suitable formulation for an injectable solution is as follows:

| | | |
|---|---|---|
| Active Ingredient | mg | 10 |
| Sodium chloride | mg | q.s. |
| Water for Injection | mL | add 1.0 |

### EXAMPLE 5

### Liquid oral formulation

A suitable formulation for 1 liter of a an oral solution containing 2 milligrams of active ingredient in one milliliter of the mixture is as follows:

| | mg |
|---|---|
| Active Ingredient | 2 |
| Saccharose | 250 |
| Glucose | 300 |
| Sorbitol | 150 |
| Orange flavor | 10 |
| Colorant | q.s. |
| Purified water | add 1000 mL |

### EXAMPLE 6

### Liquid oral formulation

Another suitable formulation for 1 liter of a liquid mixture containing 20 milligrams of active ingredient in one milliliter of the mixture is as follows:

| | G |
|---|---|
| Active Ingredient | 20.00 |
| Tragacanth | 7.00 |
| Glycerol | 50.00 |
| Saccharose | 400.00 |
| Methylparaben | 0.50 |
| Propylparaben | 0.05 |
| Black currant-flavor | 10.00 |
| Soluble Red color | 0.02 |
| Purified water | add 1000 mL |

### EXAMPLE 7

### Liquid oral formulation

Another suitable formulation for 1 liter of a liquid mixture containing 2 milligrams of active ingredient in one milliliter of the mixture is as follows:

| | G |
|---|---|
| Active Ingredient | 2 |
| Saccharose | 400 |
| Bitter orange peel tincture | 20 |
| Sweet orange peel tincture | 15 |
| Purified water | add 1000 mL |

### EXAMPLE 8

### Aerosol formulation

180 g aerosol solution contain:

| | G |
|---|---|
| Active Ingredient | 10 |
| Oleic acid | 5 |
| Ethanol | 81 |
| Purified Water | 9 |
| Tetrafluoroethane | 75 |

15 ml of the solution are filled into aluminum aerosol cans, capped with a dosing valve, purged with 3.0 bar.

### EXAMPLE 9

### TDS formulation

100 g solution contain:

| | G |
|---|---|
| Active Ingredient | 10.0 |
| Ethanol | 57.5 |
| Propyleneglycol | 7.5 |
| Dimethylsulfoxide | 5.0 |
| Hydroxyethylcellulose | 0.4 |
| Purified water | 19.6 |

1.8 ml of the solution are placed on a fleece covered by an adhesive backing foil. The system is closed by a protective liner which will be removed before use.

### EXAMPLE 10

### Nanoparticle formulation

10 g of polybutylcyanoacrylate nanoparticles contain:

| | G |
|---|---|
| Active Ingredient | 1.00 |
| Poloxamer | 0.10 |
| Butylcyanoacrylate | 8.75 |
| Mannitol | 0.10 |
| Sodium chloride | 0.05 |

Polybutylcyanoacrylate nanoparticles are prepared by emulsion polymerization in a water/0.1 N HCl/ethanol mixture as polymerizsation medium. The nanoparticles in the suspension are finally lyophilized under vacuum.

### EXAMPLE 11

### Suspension formulation

1.0 g of the suspension contains the following:

| | g |
|---|---|
| Active Ingredient | 0.10 |
| Hypromellose | 0.01 |
| Purified water | Ad 1.0 g |

Hypromellose is dispersed in water homogeneously with a high speed mixer/blender. After about one hour of hydration time of the hypromellose, the active ingredient is blended homogeneously into the hypromellose solution. The viscosity of the suspension may be adjusted by the amount of hypromellose, resulting in a very stable suspension with a very slow tendency of particle sedimentation and particle agglomeration.

### EXAMPLE 12

### Solution for Injection

1.0 ml of solution contain:

| | g |
|---|---|
| Active Ingredient | 0.05 |
| Mannitol | q.s. |
| DMSO | 0.10 |
| Water for injection | Ad 1.0 ml |

The active ingredient is dissolved in DMSO by stirring and heating (solution 1). The mannitol is dissolved in WFI (solution 2). After cooling down to rt solution 1 is mixed with solution 2 by continuous stirring. The solution is sterilized by filtration of by autoclaving.

### PHARMACOLOGY

The active principles of the present invention, and pharmaceutical compositions containing them and method of treating therewith, are characterized by unique and advantageous properties. The compounds and pharmaceutical compositions thereof exhibit, in standard accepted reliable test procedures, the following valuable properties and characteristics.

### METHODS

### BINDING ASSAYS FOR THE CHARACTERIZATION OF mGluR5 ANTAGONIST PROPERTIES

### Preparation of rat cortical membranes:

Tissue preparation is performed according to Foster & Wong (Br. J. Pharmacol., 1987, 91, 403-409) with some modifications. Anaesthetised male Sprague-Dawley rats (200-250 g, Janvier, Le Genest-Isle, France) are decapitated and their brains are removed rapidly. The cortex is dissected out and processed as described by Parsons, et al. (J. Pharmacol. Exp. Ther., 1997, 283(3): 1264-1275). For isolation of the cell membranes, the cortices are homogenized in 20 volumes of ice-cold 0.32 M sucrose (Sigma-Aldrich, Taufkirchen, Germany) using a glass-Teflon homogenizer. The homogenate is centrifuged at 1000 x g for 10 minutes, the pellet is discarded and the supernatant centrifuged at 20,000 x g for 20 minutes. The resulting pellet is resuspended in 20 volumes of distilled water and centrifuged for 20 minutes at 8000 x g. The supernatant and the buffy coat are then centrifuged six times (48,000 x g for 20 minutes) in the presence of 5 mM Tris-HCl, pH 7.5. All centrifugation steps are carried out at 4°C. After resuspension in 5 volumes of 5 mM Tris-HCl, pH 7.5, the membrane suspension is frozen rapidly at -80°C.

On the day of assay, the membrane suspensions are thawed, centrifuged (48,000 x g for 20 minutes) and then resuspended in 50 mM Tris-HCl, pH 7.5 (assay buffer). The amount of protein in the final membrane preparation is determined according to the method of Lowry, et al. (J. Biol. Chem., 1951, 193, 256-275). The final concentration used for our studies is 400 µg / ml.

### [³H]-M-MPEP (2-(3-methoxyphenylethynyl)-6-methylpyridine) Displacement Studies

The Tecan robotic system designed for binding assays is loaded with the membrane solution, a 96 well stock plate containing the solutions for bound control (buffer/DMSO 10%), unlabeled MPEP (10 µM) for evaluation of non-specific binding, all compounds to be tested (at 20-fold concentrations), various empty 96-well plates for dilutions and the final assay plates, as well as a 96-well plate filled with the radioligand.

Before performing displacement studies, saturation experiments are performed to determine the equilibrium dissociation constant (Kd, Cheng and Prussoff, Biochem. Pharmacol., 1973, 22: 3099-3108) of [³H]-M-MPEP, which is a parameter for the affinity of the radioligand to the binding site. On the basis of the saturation experiments, a final [³H]-M-MPEP concentration of 2 nM was selected.

Firstly, the assay plates are loaded with membrane solution and are shaken at 4°C. The mother plates were then prepared by pipetting the compounds (n = 5) into assay buffer/10% DMSO to obtain the desired final concentrations. After transferring radioligand into the assay plates, the compounds are added (including the non-specific binding control containing MPEP). The final DMSO concentration is always 1 %. The assay plates are incubated and shaken at 4°C for 1 h, before the mixture is exhausted as rapidly as possible via a vacuum manifold using the Multiscreen HTS glass fibre (type B) high flow filter plates (Millipore, Schwalbach, Germany) under a constant vacuum of 450 mbar. The membranes are washed four times with cold assay buffer (100 µL). 50 µL of Ultima Gold scintillation cocktail (PerkinElmer) is added to the wet filter plates and incubated at rt overnight before counting the disintegration per minutes using a liquid scintillation counter (MicroBeta, PerkinElmer).

### Characterization

For the evaluation of the test compound binding affinity to the mGluR5 binding site and its potency to displace [³H]-M-MPEP, the measured radioactivity of the radioligand alone was set as 100% bound control and the non-specific binding of the radioligand represented the 0% control. The residual radioactivity after displacement of the test compound was then corrected with respect to the set controls. Data are given as median IC₅₀ and Kᵢ, respectively.

### FUNCTIONAL ASSAY OF mGluR5 RECEPTORS

### Cytosolic Calcium studies with stably transfected cells

Chinese hamster ovary cells (CHO-K1 cells), stably transfected for inducible expression of a human metabotropic glutamate receptor mGluR5 to form a cell line called h5CHO, are seeded into black clear bottom 96 well plates at a density of 35.000 cells per well. The standard growth medium used (Dulbecco's modified Eagle Medium, DMEM plus L-proline) contains the appropriate inducer isopropyl-β-D-thiogalactopyranosid (IPTG) to achieve optimal receptor expression. One day after seeding the growth medium is exchanged for reconstituted Ca-Kit (Molecular Devices, USA) and incubated for one hour. Ca-Kit is reconstituted in an assay buffer containing 20 mM HEPES pH 7.4, glutamic-pyruvate transaminase, pyridoxal phosphate and sodium pyruvate in Hank's balanced salt solution (HBBS). Agonistic compounds to the receptor elicit increases in cytosolic calcium which can be measured as increases in fluorescence signals by use of a fluorescence imaging plate reader (Molecular Devices). To analyze their potency to negatively modulate the Ca-response test compounds, dissolved in a final DMSO concentration of 0.5%, are added on-line 5 minutes before the agonist to the receptor (L-quisqualic acid at a concentration giving ~80% of the maximal signal). EC₅₀ values for positive allosteric modulators were determined by stimulating the cells which have been preincubated the modulators by an EC₂₀ concentration of L-glutamate.

### Astrocyte culture

Primary astrocyte cultures are prepared from cortices of newborn rats as described by Booher and Sensenbrenner (1972, Neurobiology 2(3):97-105). Briefly, Sprague-Dawley rat pups (2 - 4 d old) are decapitated and neocortices are dissected, disintegrated with a nylon filter (pore size 80 µm) and carefully triturated. The cell suspension is plated on poly-D-lysine pre-coated flasks (Costar, Netherlands) and cultivated in Dulbecco's Modified Eagle's Medium (DMEM, Invitrogen, Germany) supplemented with 10% foetal calf serum (FCS, Sigma, Germany), 4 mM glutamine and 50 µg/ml gentamycin (both Biochrom, Germany) at 37°C in a humidified atmosphere of 5% CO₂/ 95% air for 7 days with exchanging the medium at day 2 and 6.

After 7 days in vitro (DIV), cells are shaken overnight at 250 rpm to remove oligodendrocytes and microglia. The next day, astrocytes are rinsed twice with CMF-PBS (calcium- and magnesium-free phosphate buffered saline, Biochrom, Germany), trypsinized and subplated on poly-D-lysine pre-coated 96-well plates (Greiner, Germany) at a density of 40,000 cells/well. 24 h after establishing the secondary culture the astrocytes are rinsed with PBS⁺⁺ (phosphate buffered saline, Biochrom, Germany) and fed with astrocyte-defined medium (ADM) consisting of DMEM containing 1x G5-supplement (Invitrogen, Germany), 0.5 µg/ml heparan sulfate, and 1.5 µg/ml fibronectin (both Sigma, Germany) (Miller et al., (1993) Brain Res. 618(1):175-8). 3 days later the medium is exchanged and the cells incubated for another 2-3 days, so that at the time of experiments astrocytes are 14-15 DIV.

### Immunocytochemistry

Immunostaining is performed to confirm the presence of astrocytic markers such as the glial fibrillary acidic protein (GFAP) as well as to monitor the expression of mGluR5 receptors.

### Cytosolic Calcium studies with astrocytes

The increase of cytosolic calcium after stimulation with the mGluR5 agonist L-quisqualate is measured using a fluorometric imaging plate reader (FLIPR) and the Ca-Kit (both Molecular Devices). Prior to addition of agonist or antagonist the medium is aspirated and cells are loaded for 2 h at RT with 150 µl of loading buffer consisting of Ca-sensitive dye reconstituted in sodium chloride (123 mM), potassium chloride (5.4 mM), magnesium chloride (0.8 mM), calcium chloride (1.8 mM), D-glucose (15 mM), and HEPES (20 mM), pH 7.3. Subsequently, plates are transferred to FLIPR to detect calcium increase with the addition of L-quisqualate (100 nM) measured as relative fluorescence units (RFU). If antagonists are tested, these compounds are pre-incubated for 10 minutes at RT before addition of the respective agonist.

For positive modulators, concentration-response curves for quisqualate are performed in the presence and absence of 10 µM modulator to determine the extent of potentiation / agonist potency increase. Thereafter, concentration-response curves for the positive modulator are performed in the presence of a fixed concentration of quisqualate showing the biggest window for potentiation (normally 10-30 nM).

### Data analysis

The fluorescence signal increase after addition of agonist reflects the increase of cytosolic calcium. Inconsistencies in the amount of cells per well are normalised by using the spatial uniformity correction of the FLIPR operating software Screenworks. The mean of replicated temporal data (n=3-5) is calculated and used for graphical representation. For the evaluation of the pharmacology, the calcium changes in response to different concentrations of agonist or antagonist are determined using a maximum minus minimum (MaxMin) calculation.

All responses (RFU-values) are determined as percentage of control (= maximum response). EC₅₀ and IC₅₀ are calculated according the logistic equation using XLFit (IDBS, UK) or Prism 4.0 (GraphPad Software, USA). The compounds of the present invention have a potency (EC₅₀ or IC₅₀) within a range of about 0.5 nM to about 100 µM. The left shifts for positive or slient allosteric modulators (PAMs or SAMs, respectively) are determined by comparison of EC₅₀ values from agonist dose response curves run in the presence of 10 µM modulator or vehicle (Vanejevs et al., J. Med. Chem., 2008, 51, 634-647).

The compounds of the present invention show show an affinity to the allosteric site, i.e., the MPEP site, shown by the displacement of the radioligand [³H]-MMPEP.

Various functional consequences associated with this affinity are observed. Negative allosteric modulators (NAMs), which inhibit the calcium signals of mGluR5 orthostatic ligands, and positive allosteric modulators (PAMs), which modulate by amplification of small glutamate signals and thereby yield a left shift in agonist concentration response curves, (they might be classified into weak PAMs with a shift above 1.5-fold and strong PAMs with a shift above 3-fold) are observed. Moreover silent allosteric modulators (SAMs), which bind to the allosteric site (i.e., have the capacity to displace MMPEP) but do not or only moderately influence the calcium signal of orthosteric agonists, are also observed.

Results for representative compounds of the invention are shown in Tables A1-A3.

**Table A1**

| **Compound** | **Chemical Name** | **hEC₅₀ [µM]** | **h left shift** | **rEC₅₀ [µM]** | **r left shift** |
|---|---|---|---|---|---|
| Example 1 | (*rac*)-5-Methoxy-3-(phenylethynyl)-5,6,7,8-tetrahydroquinoline | 0.0321 | 2.6-fold | 0.0227 | 4.9-fold |
| Example 2 | (*R*)- or (S)-5-Methoxy-3-(phenylethynyl)-5,6,7,8-tetrahydroquinoline | 0.0209 | 2.3-fold | 0.0125 | 4.4-fold |
| Example 10 | (*rac*)-3'-(Phenylethynyl)-7',8'dihydro-6'*H*-spiro[oxazolidine-5,5'-quinolin]-2-one | n.d. | 1.8-fold | n.d. | n.d. |
| Example 11 | (*rac*)-3-Methyl-3'-(phenylethynyl)-7',8'-dihydro-6'*H*-spiro[oxazolidine-5,5'-quinolin]-2-one | 0.0537 | 1.8-fold | 0.0586 | 2.9-fold |
| Example 12 | (*rac*)-3-Phenyl-3'-(phenylethynyl)-7',8'-dihydro-6'*H*-spiro[oxazolidine-5,5'-quinolin]-2-one | 0.966 | 1.9-fold | 0.856 | n.d. |
| Example 13 | (*rac*)-*cis*-4-Methyl-9-(phenylethynyl)-3,4,4a,5,6,10b-hexahydro-2*H*-[1,4]oxazino[2,3-*f*]quinoline | 0.425 | 1.7-fold | n.d. | 1.7-fold |
| Example 14 | (*rac*)-*cis*-4-Ethyl-9-(phenylethynyl)-3,4,4a,5,6,10b-hexahydro-2*H*-[1,4]oxazino[2,3-*f*]quinoline | 0.659 | 2.2-fold | 0.580 | 3.6-fold |
| Example 15 | *(rac)-cis-9-*(Phenylethynyl)-4-propyl-3,4,4a,5,6,10b-hexahydro-2*H*-[1,4]oxazino[2,3-*f*]quinoline | 0.0886 | 3.2-fold | 0.175 | 5.2-fold |
| Example 16 | *cis*-9-(Phenylethynyl)-4-propyl-3,4,4a,5,6,10b-hexahydro-2*H*-[1,4]oxazino[2,3-*f*]quinoline | 0.047 | 3.8-fold | 0.453 | 6.0-fold |
| Example 17 | *cis*-9-(Phenylethynyl)-4-propyl-3,4,4a,5,6,10b-hexahydro-2*H*-[1,4]oxazino[2,3-*f*]quinoline | 0.388 | 2.3-fold | 0.067 | 3.5-fold |
| Example 18 | *(rac)-cis*-9-((2-Fluorophenyl)ethynyl)-4-propyl-3,4,4a,5,6,10b-hexahydro-2*H*- [1,4]oxazino[2,3-*f*]quinoline | 0.247 | 2.6-fold | 0.613 | 2.6-fold |
| Example 19 | *(rac)-cis*-9-((3-Fluorophenyl)ethynyl)-4-propyl-3,4,4a,5,6,10b-hexahydro-2*H*-[1,4]oxazino[2,3-*f*]quinoline | 0.188 | 1.9-fold | 0.323 | 2.6-fold |
| Example 20 | (*rac*)-*cis*-9-((4-Fluorophenyl)ethynyl)-4-propyl-3,4,4a,5,6,10b-hexahydro-2*H*-[1,4]oxazino[2,3-*f*]quinoline | 0.394 | 1.8-fold | 1.35 | 3.3-fold |
| Example 22 | (*rac*)-*trans*-9-(Phenylethynyl)-4-propyl-3,4,4a,5,6,10b-hexahydro-2*H*-[1,4]oxazino[2,3-*f*]quinoline | 1.80 | 2.2-fold | n.d. | 1.8-fold |
| Example 23 | (*rac*)-*cis*-4-Isobutyl-9-(phenylethynyl)-3,4,4a,5,6,10b-hexahydro-2*H*-[1,4]oxazino[2,3-*f*]quinoline | 0.00400 | 5.3-fold | 0.132 | 11.7-fold |
| Example 24 | (*rac*)-*cis*-9-(Phenylethynyl)-4-(2,2,2-trifluoroethyl)-3,4,4a,5,6,10b-hexahydro-2*H*-[1,4]oxazino[2,3-*f*]quinoline | 0.0653 | 2.1-fold | 0.134 | 4.2-fold |
| Example 25 | (*rac*)-*cis*-9-(Phenylethynyl)-4-(3,3,3-trifluoropropyl)-3,4,4a,5,6,10b-hexahydro-2*H*-[1,4]oxazino[2,3-*f*]quinoline | 0.0369 | 2.0-fold | 0.136 | 3.6-fold |
| Example 26 | (*rac*)-*cis*-Methyl 9-(phenylethynyl)-4a,5,6,10b-tetrahydro-2*H*-[1,4]oxazino[2,3-*f*]quinoline-4(3*H*)-carboxylate | 0.958 | 2.9-fold | 0.599 | 4.4-fold |
| Example 27 | (*rac*)-*cis*-*N,N*-Dimethyl-9-(phenylethynyl)-4a,5,6,10b-tetrahydro-2*H*-[1,4]oxazino[2,3-*f*]quinoline-4(3*H*)-carboxamide | 0.884 | 2.4-fold | 0.427 | 3.3-fold |
| Example 28 | 1-Methyl-8-(phenylethynyl)-4,5-dihydro-1*H*-pyrazolo[3,4-*f*]quinoline | 0.0192 | 2.4-fold | n.d. | 2.2-fold |
| Example 30 | Methyl 8-(phenylethynyl)-3,3a,4,5-tetrahydro-1*H*-pyrrolo[3,4-*f*]quinoline-2(9b*H*)-carboxylate | 0.345 | 2.5-fold | 0.256 | 3.0-fold |
| Example 31 | Methyl 8-((3-fluorophenyl)ethynyl)-3,3a,4,5-tetrahydro-1*H*-pyrrolo[3,4-*f*]quinoline-2(9b*H*)-carboxylate | 0.333 | 2.6-fold | 0.261 | 1.5-fold |
| Example 34 | 2,2-Dimethyl-1-(3-(phenylethynyl)-7,8-dihydro-1,6-naphthyridin-6(5*H*)-yl)propan-1-one | 0.171 | 2.3-fold | 0.0679 | 2.8-fold |
| Example 36 | (3-Methyloxetan-3-yl)(3-(phenylethynyl)-7,8-dihydro-1,6-naphthyridin-6(5*H*)-yl)methanone | 0.917 | 2.4-fold | 4.04 | 2.7-fold |
| Example 37 | *(rac)-6-(tert-*Butylsulfinyl)-3-(phenylethynyl)-5,6,7,8-tetrahydro-1,6-naphthyridine | 0.472 | 2.5-fold | 0.508 | 3.3-fold |
| Example 38 | 6-(Cyclopropylsulfonyl)-3-(phenylethynyl)-5,6,7,8-tetrahydro-1,6-naphthyridine | n.d. | 1.7-fold | 0.481 | 2.5-fold |
| Example 41 | Isobutyl 3-(phenylethynyl)-7,8-dihydro-1,6-naphthyridine-6(5*H*)-carboxylate | 0.146 | 2.2-fold | 0.00436 | 1.2-fold |
| Example 42 | *tert*-Butyl 3-(phenylethynyl)-7,8-dihydro-1,6-naphthyridine-6(5*H*)-carboxylate | 0.281 | 3.1-fold | 0.131 | 1.9-fold |
| Example 43 | Phenyl 3-(phenylethynyl)-7,8-dihydro-1,6-naphthyridine-6(5*H*)-carboxylate | 0.381 | 2.4-fold | 0.0380 | n.d. |
| Example 49 | 3-(Phenylethynyl)-*N,N*-bis(2,2,2-trifluoroethyl)-7,8-dihydro-1,6-naphthyridine-6(5*H*)-carboxamide | n.d. | 1.7-fold | 0.0118 | 2.3-fold |
| Example 50 | (3-(Phenylethynyl)-7,8-dihydro-1,6-naphthyridin-6(5*H*)-yl)(piperidin-1-yl)methanone | 0.843 | 2.1-fold | n.d. | 1.9-fold |
| Example 52 | *N,N*-Dimethyl-3-(phenylethynyl)-7,8-dihydro-1,6-naphthyridine-6(5*H*)-sulfonamide | 0.240 | 2.0-fold | 0.0831 | 1.8-fold |
| Example 53 | 3-(Phenylethynyl)-6-(pyrrolidin-1-ylsulfonyl)-5,6,7,8-tetrahydro-1,6-naphthyridine | n.d. | 1.6-fold | 0.152 | 2.8-fold |
| Example 54 | 3-(Phenylethynyl)-6-(piperidin-1-ylsulfonyl)-5,6,7,8-tetrahydro-1,6-naphthyridine | 0.726 | 2.0-fold | 0.472 | 2.5-fold |
| Example 55 | 4-((3-(Phenylethynyl)-7,8-dihydro-1,6-naphthyridin-6(5*H*)-yl)sulfonyl)morpholine | 0.440 | 2.7-fold | 0.325 | 3.8-fold |
| Example 56 | 6-(Azepan-1-ylsulfonyl)-3-(phenylethynyl)-5,6,7,8-tetrahydro-1,6-naphthyridine | 0.499 | 3.0-fold | 0.296 | 3.3-fold |
| Example 58 | (*rac*)-Methyl 3-(phenylethynyl)-6,7,8,9-tetrahydro-5*H*-5,8-epiminocyclohepta[*b*]py ridine-10-carboxylate | 0.201 | 2.3-fold | 0.113 | 3.9-fold |
| Example 59 | (*rac*)-Methyl 3-((3-fluorophenyl)ethynyl)-6,7,8,9-tetrahydro-5*H*-5,8-epiminocyclohepta[*b*]py ridine-10-carboxylate | 0.277 | 1.8-fold | 0.102 | 3.3-fold |
| Example 65 | (*rac*)-*cis*-Azetidin-1-yl-(9-(phenylethynyl)-5,6-dihydro-2*H*-[1,4]oxazino[2,3-*f*]quinolin-4(3*H*,4a*H*,10b*H*)-yl)methanone | n.d. | 1.8-fold | 3.20 | n.d. |
| Example 67 | (*rac*)-3'-((3-Fluorophenyl)ethynyl)-3-phenyl-7',8'-dihydro-6'*H*-spiro[oxazolidine-5,5'-quinolin]-2-one | 1.31 | 2.1-fold | 0.0883 | 1.7-fold |
| Example 71 | (6a*R*,9a*R*)-Methyl 3-(phenylethynyl)-6,6a,9,9a-tetrahydro-5*H*-pyrrolo[2,3-*h*]quinoline-7(8*H*)-carboxylate | 0.241 | 2.2-fold | 0.0327 | 1.3-fold |
| Example 72 | (6a*R*,9a*R*)-Methyl 3-((3-fluorophenyl)ethynyl)-6,6a,9,9a-tetrahydro-5*H*-pyrrolo[2,3-*h*]quinoline-7(8*H*)-carboxylate | n.d. | 1.7-fold | 0.0273 | 1.3-fold |
| Example 73 | (*rac*)-*cis*-Methyl 3-((3-fluorophenyl)ethynyl)-5a,6,8,8a-tetrahydropyrrolo[3',4':4 ,5]cyclopenta[1,2-*b*]pyridine-7(5*H*)-carboxylate | 0.546 | 2.7-fold | 0.219 | 2.7-fold |
| Example 74 | (*rac*)-3-Cyclopropyl-3'-(phenylethynyl)-7',8'-dihydro-6'*H*-spiro[oxazolidine-5,5'-quinolin]-2-one | n.d. | 1.6-fold | 0.0101 | 1.5-fold |
| Example 75 | 5,5-Dimethyl-3-(phenylethynyl)-7,8-dihydro-5*H*-thiopyrano[4,3-*b*]pyridine 6,6-dioxide | 0.971 | 1.6-fold | n.d. | n.d. |
| Example 77 | Ethyl 2-oxo-2-(3-(phenylethynyl)-7,8-dihydro-1,6-naphthyridin-6(5*H*)-yl)acetate | 0.0254 | 2.8-fold | 0.0221 | 2.8-fold |
| Example 78 | 6-((4-Methylpiperazin-1-yl)sulfonyl)-3-(phenylethynyl)-5,6,7,8-tetrahydro-1,6-naphthyridine | 2.02 | 1.7-fold | 2.05 | n.d. |
| Example 79 | (*rac*)-Methyl 3-(phenylethynyl)-5a,6,8,8a-tetrahydropyrrolo[3',4':4 ,5]cyclopenta[1,2-b]pyridine-7(5*H*)-carboxylate | 0.0647 | 2.6-fold | 0.167 | 4.1-fold |
| Example 82 | (*rac*)-3'-(Phenylethynyl)-3-propyl-7',8'-dihydro-6'*H*-spiro[oxazolidine-5,5'-quinolin]-2-one | 0.727 | 1.9-fold | 0.233 | 1.6-fold |

**Table A2**

| **Compound** | **Chemical Name** | **hIC₅₀ [µM]** | **rIC₅₀ [µM]** |
|---|---|---|---|
| Example 3 | (*rac*)-6-((5-Methoxy-5,6,7,8-tetrahydroquinolin-3-yl)ethynyl)pyridin-2-amine | 4.23 | n.d. |
| Example 4 | (*rac*)-5-Methoxy-7,7-dimethyl-3-(phenylethynyl)-5,6,7,8-tetrahydroquinoline | 0.341 | n.d. |
| Example 5 | (*rac*)-6-((5-Methoxy-7,7-dimethyl-5,6,7,8-tetrahydroquinolin-3-yl)ethynyl)pyridin-2-amine | 0.0667 | 0.0457 |
| Example 6 | (*rac*)-3-(Imidazo[1,2-*a*]pyridin-2-ylethynyl)-5-methoxy-7,7-dimethyl-5,6,7,8-tetrahydroquinoline | 0.695 | n.d. |
| Example 7 | (*rac*)-6-((4-Methoxy-2,2-dimethyl-3,4-dihydro-2*H*-pyrano[2,3-*b*]pyridin-6-yl)ethynyl)pyridin-2-amine | 0.203 | n.d. |
| Example 8 | (*rac*)-6-(Imidazo[1,2-*a*]pyridin-2-ylethynyl)-4-methoxy-2,2-dimethyl-3,4-dihydro-2*H*-pyrano[2,3-b]pyridine | 0.225 | n.d. |
| Example 35 | 1-(3-((6-Aminopyridin-2-yl)ethynyl)-7,8-dihydro-1,6-naphthyridin-6(5*H*)-yl)-2,2-dimethylpropan-1-one | 0.617 | n.d. |
| Example 44 | *N,N*-Dimethyl-3-(phenylethynyl)-7,8-dihydro-1,6-naphthyridine-6(5*H*)-carboxamide | 0.247 | n.d. |
| Example 45 | 3-((3-Fluorophenyl)ethynyl)-*N,N*-dimethyl-7,8-dihydro-1,6-naphthyridine-6(5*H*)-carboxamide | 0.114 | 0.0559 |
| Example 46 | 3-((3-Cyanophenyl)ethynyl)-*N,N*-dimethyl-7,8-dihydro-1,6-naphthyridine6(5*H*)-carboxamide | 0.0508 | 0.0208 |
| Example 47 | 3-((6-Aminopyridin-2-yl)ethynyl)-*N,N*-dimethyl-7,8-dihydro-1,6-naphthyridine-6(5*H*)-carboxamide | 0.261 | n.d. |
| Example 48 | 3-((3-Cyanophenyl)ethynyl)-*N,N*-diethyl-7,8-dihydro-1,6-naphthyridine-6(5*H*)-carboxamide | 0.194 | n.d. |
| Example 51 | 3-((6-(Piperidine-1-carbonyl)-5,6,7,8-tetrahydro-1,6-naphthyridin-3-yl)ethynyl)benzonitrile | 0.740 | 2.33 |
| Example 57 | (*rac*)-8-Methyl-3-(phenylethynyl)-9,10,10a,11-tetrahydro-5*H*-pyrimido[1,6-*g*][1,6]naphthyridin-7(8*H*)-one | 0.190 | n.d. |
| Example 61 | (*rac*)-*cis*-Methyl 3-((3-fluorophenyl)ethynyl)-7,8,9,9a-tetrahydro-5*H*-cyclopenta[*h*][1,6]naphthyridine-6(6a*H*)-carboxylate | 0.132 | n.d. |
| Example 62 | (*rac*)-*cis*-Methyl 3-(phenylethynyl)-6a,7,8,9,10,10a-hexahydrobenzo[*h*][1,6]naphthyridine-6(5*H*)-carboxylate | 1.40 | n.d. |
| Example 63 | (*rac*)-*cis*-Methyl 3-((3-fluorophenyl)ethynyl)-6a,7,8,9,10,10a-hexahydrobenzo[*h*][1,6]naphthyridine-6(5*H*)-carboxylate | 1.32 | n.d. |
| Example 68 | 3-(*m*-Tolylethynyl)-7,8-dihydro-5*H*-thiopyrano[4,3-*b*]pyridine 6,6-dioxide | 0.0755 | n.d. |

**Table A3**

| **Compound** | **Chemical Name** | **h left shift** | **r left shift** | ***K*ᵢ (MMPEP) [µM]** |
|---|---|---|---|---|
| Example 21 | (*rac*)-*cis*-4-Propyl-9-(m-tolylethynyl)-3,4,4a,5,6,10b-hexahydro-2*H*-[1,4]oxazino[2,3-*f*]quinoline | 1.3-fold | 1.9-fold | 1.72 |
| Example 29 | (*rac*)-*cis*-Methyl 8-(phenylethynyl)-2,3,3a,4,5,9b-hexahydro-1*H*-pyrrolo[2,3-*f*]quinoline-1-carboxylate | 1.3-fold | 0.8-fold | 0.289 |
| Example 32 | (*rac*)-*cis*-Methyl 3-((3-fluorophenyl)ethynyl)-6a,7,9,9a-tetrahydro-5*H*-pyrrolo[3,4-h]quinoline-8(6*H*)-carboxylate | 0.9-fold | n.d. | 0.0493 |
| Example 33 | 2-Methyl-1-(3-(phenylethynyl)-7,8-dihydro-1,6-naphthyridin-6(5*H*)-yl)propan-1-one | 1.4-fold | 1.3-fold | 0.395 |
| Example 40 | Ethyl 3-(phenylethynyl)-7, 8-dihydro-1,6-naphthyridine-6(5*H*)-carboxylate | 1.3-fold | 1.6-fold | 0.0565 |
| Example 64 | (*rac*)-3-Propyl-3'-(*m*-tolylethynyl)-7',8'-dihydro-6'*H*-spiro[oxazolidine-5,5'-quinolin]-2-one | 1.2-fold | 0.9-fold | 0.399 |
| Example 66 | (*rac*)-3'-((3-Fluorophenyl)ethynyl)-3-propyl-7',8'-dihydro-6'*H*-spiro[oxazolidine-5,5'-quinolin]-2-one | 1.6-fold | 1.3-fold | 0.857 |
| Example 76 | (*rac*)-3-Cyclopropyl-3'-((3-fluorophenyl)ethynyl)-7',8'-dihydro-6'*H*-spiro[oxazolidine-5,5'-quinolin]-2-one | 1.6-fold | 1.4-fold | n.d. (r EC₅₀: 0.0505) |
| Example 80 | (*rac*)-cis-Methyl 7-(phenylethynyl)-2,3,3a,4,5,9b-hexahydro-1*H*-pyrrolo[3,2-*h*]quinoline-1-carboxylate | 1.5-fold | 1.8-fold | n.d. (r EC₅₀: 0.0355) |
| Example 81 | 6-(Isopropylsulfonyl)-3-(phenylethynyl)-5,6,7,8-tetrahydro-1,6-naphthyridine | 1.5-fold | 1.5-fold | n.d. (r EC₅₀: 0.0511) |
| Example 2* | (*R*)- or (*S*)-5-Methoxy-3-(phenylethynyl)-5,6,7,8-tetrahydroquinoline | 2.3-fold | 4.4-fold | 0.204 |
| Example 17* | *cis*-9-(Phenylethynyl)-4-propyl-3,4,4a,5,6,10b-hexahydro-2*H*-[1,4]oxazino[2,3-*f*]quinoline | 2.3-fold | 3.5-fold | 3.62 |

| | | | | |
|---|---|---|---|---|
| *Examples 2 and 17 (both PAMs) are listed here to show exemplarily the displacement of [³H]-MMPEP by PAMs. | | | | |

A few examples show a very limited effect on the agonist response curve of the receptor mGluR5 (i.e., a left shift close to 1). Nevertheless, they bind to the allosteric site and displace [³H]-MMPEP from this site. Therefore these compounds are classified as as SAMs (silent allosteric modulators) or as very weak PAMs, with the distinction between SAMs and weak PAMs being somewhat arbitrary.

In conclusion, from the foregoing, it is apparent that the present invention provides novel and valuable applications and uses of the compounds of the present invention, which compounds comprise the active principle according to the present invention, as well as novel pharmaceutical compositions thereof and methods of preparation thereof and of treating therewith.

The high order of activity of the active agent of the present invention and compositions thereof, as evidenced by the tests reported, is indicative of utility based on its valuable activity in human beings as well as in lower animals. Clinical evaluation in human beings has not been completed. It will be clearly understood that the distribution and marketing of any compound or composition falling within the scope of the present invention for use in human beings will of course have to be predicated upon prior approval by governmental agencies which are responsible for and authorized to pass judgment on such questions.

The instant compounds of Formula I represent a novel class of mGluR5 modulators. In view of their potency, they will be useful therapeutics in a wide range of disorders, in particular CNS disorders, which involve excessive glutamate induced excitation.

These compounds accordingly find application in the treatment of the disorders of a living animal body, especially a human, as listed earlier in the description.

These compounds also find application in the treatment of indications in a living animal body, especially a human, wherein a particular condition does not necessarily exist but wherein a particular physiological parameter may be improved through administration of the instant compounds, including cognitive enhancement.

Neuroprotection as well as cognitive enhancement may also be achieved by administration of the instant compounds in combination with a NMDA receptor antagonist like Memantine.

The method-of-treating a living animal body with a compound of the invention, for the inhibition of progression or alleviation of the selected ailment therein, is as previously stated by any normally-accepted pharmaceutical route, employing the selected dosage which is effective in the alleviation of the particular ailment desired to be alleviated. Use of the compounds of the present invention in the manufacture of a medicament for the treatment of a living animal for inhibition of progression or alleviation of selected ailments or conditions, particularly ailments or conditions susceptible to treatment with a Group I mGluR modulator is carried out in the usual manner comprising the step of admixing an effective amount of a compound of the invention with a pharmaceutically-acceptable diluent, excipient, or carrier, and the method-of-treating, pharmaceutical compositions, and use of a compound of the present invention in the manufacture of a medicament.

Representative pharmaceutical compositions prepared by admixing the active ingredient with a suitable pharmaceutically-acceptable excipient, diluent, or carrier, include tablets, capsules, solutions for injection, liquid oral formulations, aerosol formulations, TDS formulations, and nanoparticle formulations, thus to produce medicaments for oral, injectable, or dermal use, also in accord with the foregoing.

The present invention is not to be limited in scope by the specific embodiments described herein. Indeed, various modifications of the invention in addition to those described herein will become apparent to those skilled in the art from the foregoing description.

All patents, applications, publications, test methods, literature, and other materials cited herein are hereby incorporated by reference.

## Claims

1. A compound selected from those of Formula I 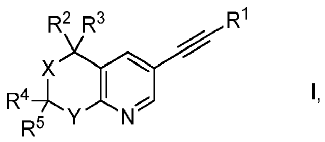 wherein
R¹ represents aryl, heteroaryl, cycloC₃₋₁₂alkyl, cycloC₅₋₁₂alkenyl, or heterocyclyl; R² represents H, F, OH, C₁₋₆alkyl, C₁₋₆alkoxy, heterocyclyl, or NR⁶R⁷;
R³ represents H, F, or C₁₋₆alkyl;
or R² and R³ together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, Cₗ-₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R⁴ represents H, F, or C₁₋₆alkyl;
or R² and R⁴ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R⁵ represents H, F, or C₁₋₆alkyl;
or R⁴ and R⁵ together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R⁶ represents H, C₁₋₆alkyl, or acyl;
R⁷ represents H or C₁₋₆alkyl;
X represents CR⁸R⁹, NR¹⁰, S, S=O, or SO₂;
Y represents a bond, -CR¹¹R¹²-, -CR¹³R¹⁴-CH₂-, O, -CR¹⁵R¹⁶-O-, NR¹⁷, or -CR¹⁸R¹⁹-NR²⁰-;
R⁸ represents H, C₁₋₆alkyl, acyl, heterocyclyl, heterocyclylsulfinyl;
or R² and R⁸ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoroethyl, trifluoropropyl, trifluoromethoxy, C₁₋₆alkyl, aryl, heteroaryl,
cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R² and R⁸ together with the carbon atoms to which they are attached form a 5-6 membered aromatic or heteroaromatic ring, wherein the ring may be optionally substituted by one or more substituents selected from halogen, fluoromethyl, difluoromethyl, trifluoromethyl, trifluoroethyl, trifluoropropyl, trifluoromethoxy, C₁₋₆alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl, in which case the R³ and R⁹ substituents are absent;
or R⁴ and R⁸ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R⁹ represents H or C₁₋₆alkyl;
or R⁸ and R⁹ together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹⁰ represents hydrogen, C₁₋₆alkyl, acyl, -C(O)-C(O)-C₁₋₆alkoxy, C₁₋₆alkylsulfinyl, C₁₋₆alkylsulfonyl, cycloC₃₋₇alkylsulfonyl, heterocyclylsulfonyl, arylsulfonyl, di-(C₁₋₆alkyl)aminosulfonyl, aryl, or heteroaryl;
or R² and R¹⁰ together with the atoms to which they are attached form a 3-7 membered ring which may be saturated or unsaturated, wherein the ring may optionally contain one or two additional heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R⁴ and R¹⁰ together with the atoms to which they are attached form a 3-7 membered ring which may be saturated or unsaturated, wherein the ring may optionally contain one or two additional heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹¹ represents H, C₁₋₆alkyl, acyl or heterocyclyl;
or R⁴ and R¹¹ together with the carbon atoms to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl-,
or R² and R¹¹ together with the carbon atoms to which they are attached form a 5-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
or R⁸ and R¹¹ together with the carbon atoms to which they are attached form a 4-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹² represents H or C₁₋₆alkyl;
or R¹¹ and R¹² together with the carbon atom to which they are attached form a 3-7 membered ring which may be saturated or unsaturated, wherein the ring may optionally contain one or two heteroatoms selected from sulfur, oxygen, and nitrogen, wherein the ring may be optionally substituted by one or more substituents selected from halogen, fluoromethyl, difluoromethyl, trifluoromethyl, trifluoromethoxy, C₁₋₆alkyl, aryl, heteroaryl, cycloC₃₋₇alkyl, C₁₋₆alkoxy, amino, hydroxy, cyano, C₁₋₆alkoxycarbonyl, C₁₋₆alkylamino, di-(C₁₋₆alkyl)amino, C₁₋₆alkylcarbonylamino, oxo, C₁₋₆alkylaminocarbonyl, and di-(C₁₋₆alkyl)aminocarbonyl;
R¹³ represents H, C₁₋₆alkyl, acyl or heterocyclyl;
R¹⁴ represents H or C₁₋₆alkyl;
R¹⁵ represents H, C₁₋₆alkyl, acyl or heterocyclyl;
R¹⁶ represents H or C₁₋₆alkyl;
R¹⁷ represents H, C₁₋₆alkyl, acyl, aryl, or heteroaryl;
R¹⁸ represents H, C₁₋₆alkyl, acyl or heterocyclyl;
R¹⁹ represents H or C₁₋₆alkyl;
R²⁰ represents H, C₁₋₆alkyl, acyl, aryl, or heteroaryl;
and optical isomers, prodrugs, pharmaceutically acceptable salts, hydrates, solvates, and polymorphs thereof.

2. The compound as claimed in Claim 1, wherein R¹ represents aryl or heteroaryl.

3. The compound as claimed in Claim 2, wherein R¹ represents phenyl optionally substituted by one or more substituents selected from F, C₁₋₆alkyl, and cyano; pyridyl optionally substituted by one or more amino groups; or imidazopyridyl.

4. The compound as claimed in any preceding claim, wherein R² represents H, C₁₋₆alkyl, OH, or C₁₋₆alkoxy and R³ represents H or C₁₋₆alkyl.

5. The compound as claimed in any of Claims 1 to 3, wherein R² and R³ together with the carbon atom to which they are attached form a 5-membered ring containing one or two heteroatoms selected from sulfur, oxygen, and nitrogen, wherein the ring may be optionally substituted by one or more substituents selected from C₁₋₆alkyl, oxo, phenyl, and cycloC₃₋₇alkyl.

6. The compound as claimed in any preceding claim, wherein X represents CR⁸R⁹, NR¹⁰, or SO₂.

7. The compound as claimed in Claim 6, wherein X represents NR¹⁰ and R¹⁰ represents C₁₋₆alkylcarbonyl, cycloC₃₋₁₂alkylcarbonyl, C₁₋₆alkoxycarbonyl, aryloxycarbonyl, di-(C₁₋₆alkyl)aminocarbonyl (wherein the alkyl moieties may be optionally substituted by one or more halogen atoms), -C(O)-C(O)-C₁₋₆alkoxy, C₁₋₆alkylsulfinyl, C₁₋₆alkylsulfonyl, cycloC₃₋₇alkylsulfonyl, arylsulfonyl, heterocyclylsulfonyl, or di-(C₁₋₆alkyl)aminosulfonyl; or R⁴ and R¹⁰ together with the atoms to which they are attached form a 6-membered ring optionally containing one or two additional heteroatoms selected from sulfur, oxygen, and nitrogen, wherein the ring may be optionally substituted by one or more substituents selected from oxo and C₁₋₆alkyl.

8. The compound as claimed in any of Claims 1 to 3, wherein X represents CR⁸R⁹ and R² and R⁸ together with the carbon atoms to which they are attached form a 5 or 6 membered ring optionally containing one or two heteroatoms selected from sulfur, oxygen, and nitrogen, wherein the ring may be optionally substituted by one or more substituents selected from C₁₋₆alkyl, trifluoroethyl, trifluoropropyl, C₁₋₆alkoxycarbonyl, and di-(C₁₋₆alkyl)aminocarbonyl or R² and R⁸ together with the carbon atoms to which they are attached form a 5-membered heteroaromatic ring optionally substituted by one or more C₁₋₆alkyl groups.

9. The compound as claimed in any preceding claim, wherein Y represents -CR¹¹R¹², O, NR¹⁷, or a bond.

10. The compound as claimed in any of Claims 1 to 5, wherein Y represents -CR¹¹R¹² and R¹¹ and R¹² each represent H or R⁴ and R¹¹ together with the carbon atoms to which they are attached form a 5 or 6 membered ring optionally containing one or two heteroatoms selected from sulfur, oxygen, and nitrogen, wherein the ring may be optionally substituted by one or more C₁₋₆alkoxycarbonyl groups.

11. The compound as claimed in any of Claims 1 to 5, or 8, wherein R⁴ and R⁵ each independently represent H or C₁₋₆alkyl.

12. The compound as claimed in Claim 1, which is selected from:
(*rac*)-5-Methoxy-3-(phenylethynyl)-5,6,7,8-tetrahydroquinoline, 5-Methoxy-3-(phenylethynyl)-5,6,7,8-tetrahydroquinoline,
(*rac*)-6-((5-Methoxy-5,6,7,8-tetrahydroquinolin-3-yl)ethynyl)pyridin-2-amine, (*rac*)-5-Methoxy-7,7-dimethyl-3-(phenylethynyl)-5,6,7,8-tetrahydroquinoline, (*rac*)-6-((5-Methoxy-7,7-dimethyl-5,6,7,8-tetrahydroquinolin-3-yl)ethynyl)pyridin-2-amine,
(*rac*)-3-(Imidazo[1,2-a]pyridin-2-ylethynyl)-5-methoxy-7, 7-dimethyl-5,6,7, 8-tetrahydroquinoline,
(*rac*)-6-((4-Methoxy-2,2-dimethyl-3,4-dihydro-2*H*-pyrano[2,3-b]pyridin-6-yl)ethynyl)pyridin-2-amine,
(*rac*)- 6-(Imidazo[1,2-a]pyridin-2-ylethynyl)-4-methoxy-2,2-dimethyl-3,4-dihydro-2*H-*pyrano[2,3-*b*]pyridine,
4-Methoxy-1-methyl-6-(phenylethynyl)-1,2,3,4-tetrahydro-1,8-naphthyridine, (*rac*)-3'-(Phenylethynyl)-7',8'-dihydro-6'*H*-spiro[oxazolidine-5,5'-quinolin]-2-one, (*rac*)-3-Methyl-3'-(phenylethynyl)-7',8'-dihydro-6'*H*-spiro[oxazolidine-5,5'-quinolin]-2-one,
(*rac*)-3-Phenyl-3'-(phenylethynyl)-7',8'-dihydro-6'*H*-spiro[oxazolidine-5,5'-quinolin]-2-one,
(*rac*)-*cis+*-4-Methyl-9-(phenylethynyl)-3,4,4a,5,6,10b-hexahydro-2*H-*[1,4]oxazino[2,3-*f*]quinoline,
(*rac*)-*cis*-4-Ethyl-9-(phenylethynyl)-3,4,4a,5,6,10b-hexahydro-2*H*-[1,4]oxazino[2,3-*f*]quinoline,
(*rac*)-*cis*-9-(Phenylethynyl)-4-propyl-3,4,4a,5,6,10b-hexahydro-2*H-*[1,4]oxazino[2,3-*f*]quinoline,
*cis*-9-(Phenylethynyl)-4-propyl-3,4,4a,5,6,10b-hexahydro-2*H*-[1,4]oxazino[2,3-*f*]quinoline,
*cis*-9-(Phenylethynyl)-4-propyl-3,4,4a,5,6,10b-hexahydro-2*H*-[1,4]oxazino[2,3-*f*]quinoline,
(*rac*)-*cis*-9-((2-Fluorophenyl)ethynyl)-4-propyl-3,4,4a,5,6,1Ob-hexahydro-2*H-*[1,4]oxazino[2,3-*f*]quinoline,
(*rac*)-*cis*-9-((3-Fluorophenyl)ethynyl)-4-propyl-3,4,4a,5,6,10b-hexahydro-2*H-*[1,4]oxazino[2,3-*f*]quinoline,
(*rac*)-*cis*-9-((4-Fluorophenyl)ethynyl)-4-propyl-3,4,4a,5,6,10b-hexahydro-2*H-*[1,4]oxazino[2,3-*f*]quinoline,
(*rac*)-*cis*-4-Propyl-9-(m-tolylethynyl)-3,4,4a,5,6,10b-hexahydro-2*H-*[1,4]oxazino[2,3-*f*]quinoline,
(*rac*)-*trans*-9-(Phenylethynyl)-4-propyl-3,4,4a,5,6,10b-hexahydro-2*H-*[1,4]oxazino[2,3-*f*]quinoline,
(*rac*)-*cis*-4-Isobutyl-9-(phenylethynyl)-3,4,4a,5,6,10b-hexahydro-2*H-*[1,4]oxazino[2,3-*f*]quinoline,
(*rac*)-*cis*-9-(Phenylethynyl)-4-(2,2,2-trifluoroethyl)-3,4,4a,5,6,10b-hexahydro-2*H-*[1,4]oxazino[2,3-*f*]quinoline,
(*rac*)-*cis*-9-(Phenylethynyl)-4-(3,3,3-trifluoropropyl)-3,4,4a,5,6,10b-hexahydro-2*H-*[1,4]oxazino[2,3-*f*]quinoline,
(*rac*)-*cis*-Methyl 9-(phenylethynyl)-4a,5,6,10b-tetrahydro-2*H*-[1,4]oxazino[2,3-f]quinoline-4(3*H*)-carboxylate,
(rac)-*cis*-*N,N*-Dimethyl-9-(phenylethynyl)-4a,5,6,10b-tetrahydro-2*H-*[1,4]oxazino[2,3-*f*]quinoline-4(3*H*)-carboxamide,
1-Methyl-8-(phenylethynyl)-4,5-dihydro-1*H*-pyrazolo[3,4-*f*]quinoline,
(*rac*)-*cis*-Methyl 8-(phenylethynyl)-2,3,3a,4,5,9b-hexahydro-1*H*-pyrrolo[2,3-*f*]quinoline-1-carboxylate,
Methyl 8-(phenylethynyl)-3,3a,4,5-tetrahydro-1*H*-pyrrolo[3,4-*f*]quinoline-2(9b*H*)-carboxylate,
Methyl 8-((3-fluorophenyl)ethynyl)-3,3a,4,5-tetrahydro-1*H*-pyrrolo[3,4-*f*]quinoline-2(9b*H*)-carboxylate,
(*rac*)-*cis*-Methyl 3-((3-fluorophenyl)ethynyl)-6a,7,9,9a-tetrahydro-5*H*-pyrrolo[3,4-*h*]quinoline-8(6*H*)-carboxylate,
2-Methyl-1-(3-(phenylethynyl)-7,8-dihydro-1,6-naphthyridin-6(5*H*)-yl)propan-1-one,
2,2-Dimethyl-1-(3-(phenylethynyl)-7,8-dihydro-1,6-naphthyridin-6(5*H*)-yl)propan-1-one,
1-(3-((6-Aminopyridin-2-yl)ethynyl)-7,8-dihydro-1,6-naphthyridin-6(5*H*)-yl)-2,2-dimethylpropan-1-one,
(3-Methyloxetan-3-yl)(3-(phenylethynyl)-7,8-dihydro-1,6-naphthyridin-6(5*H*)-yl)methanone,
(rac)-6-(*tert*-Butylsulfinyl)-3-(phenylethynyl)-5,6,7,8-tetrahydro-1,6-naphthyridine,
6-(Cyclopropylsulfonyl)-3-(phenylethynyl)-5,6,7,8-tetrahydro-1,6-naphthyridine,
3-(Phenylethynyl)-6-(phenylsulfonyl)-5,6,7,8-tetrahydro-1,6-naphthyridine,
Ethyl 3-(phenylethynyl)-7,8-dihydro-1,6-naphthyridine-6(5*H*)-carboxylate,
Isobutyl 3-(phenylethynyl)-7,8-dihydro-1,6-naphthyridine-6(5*H*)-carboxylate,
*tert*-Butyl 3-(phenylethynyl)-7,8-dihydro-1,6-naphthyridine-6(5*H*)-carboxylate,
Phenyl 3-(phenylethynyl)-7,8-dihydro-1,6-naphthyridine-6(5*H*)-carboxylate,
*N,N*-Dimethyl-3-(phenylethynyl)-7,8-dihydro-1,6-naphthyridine-6(5*H*)-carboxamide,
3-((3-Fluorophenyl)ethynyl)-*N,N*-dimethyl-7,8-dihydro-1,6-naphthyridine-6(5*H*)-carboxam ide,
3-((3-Cyanophenyl)ethynyl)-*N,N*-dimethyl-7,8-dihydro-1,6-naphthyridine-6(5*H*)-carboxam ide,
3-((6-Aminopyridin-2-yl)ethynyl)-*N,N*-dimethyl-7,8-dihydro-1,6-naphthyridine-6(5*H*)-carboxam ide,
3-((3-Cyanophenyl)ethynyl)-*N,N*-diethyl-7,8-dihydro-1,6-naphthyridine-6(5*H*)-carboxam ide,
3-(Phenylethynyl)-*N,N*-bis(2,2,2-trifluoroethyl)-7,8-dihydro-1,6-naphthyridine-6(5*H*)-carboxam ide,
(3-(Phenylethynyl)-7,8-dihydro-1,6-naphthyridin-6(5*H*)-yl)(piperidin-1-yl)methanone,
3-((6-(Piperidine-1-carbonyl)-5,6,7,8-tetrahydro-1,6-naphthyridin-3-yl)ethynyl)benzonitrile,
*N,N*-Dimethyl-3-(phenylethynyl)-7,8-dihydro-1,6-naphthyridine-6(5*H*)-sulfonamide,
3-(Phenylethynyl)-6-(pyrrolidin-1-ylsulfonyl)-5,6,7,8-tetrahydro-1,6-naphthyridine,
3-(Phenylethynyl)-6-(piperidin-1-ylsulfonyl)-5,6,7,8-tetrahydro-1,6-naphthyridine,
4-((3-(Phenylethynyl)-7,8-dihydro-1,6-naphthyridin-6(5*H*)-yl)sulfonyl)morpholine,
6-(Azepan-1-ylsulfonyl)-3-(phenylethynyl)-5,6,7,8-tetrahydro-1,6-naphthyridine,
(*rac*)-8-Methyl-3-(phenylethynyl)-9,10,10a,11-tetrahydro-5*H*-pyrimido[1,6-*g*][1,6]naphthyridin-7(8*H*)-one,
(*rac*)-Methyl 3-(phenylethynyl)-6,7,8,9-tetrahydro-5*H*-5,8-epiminocyclohepta[*b*]pyridine-10-carboxylate,
(*rac*)-Methyl 3-((3-fluorophenyl)ethynyl)-6,7,8,9-tetrahydro-5*H*-5,8-epiminocyclohepta[*b*]pyridine-10-carboxylate,
(*rac*)-*cis*-Methyl 3-(phenylethynyl)-7,8,9,9a-tetrahydro-5*H-*cyclopenta[*h*][1,6]naphthyridine-6(6a*H*)-carboxylate,
(*rac*)-*cis*-Methyl 3-((3-fluorophenyl)ethynyl)-7,8,9,9a-tetrahydro-5*H-*cyclopenta[*h*][1,6]naphthyridine-6(6a*H*)-carboxylate,
(*rac*)-*cis*-Methyl 3-(phenylethynyl)-6a,7,8,9,10,10a-hexahydrobenzo[*h*][1,6]naphthyridine-6(5*H*)-carboxylate,
(*rac*)-*cis*-Methyl 3-((3-fluorophenyl)ethynyl)-6a,7,8,9,10,10a-hexahydrobenzo[*h*][1,6]naphthyridine-6(5*H*)-carboxylate,
(*rac*)-3-Propyl-3'-(*m*-tolylethynyl)-7',8'-dihydro-6'*H*-spiro[oxazolidine-5,5'-quinolin]-2-one,
(*ra*c)-*cis*-Azetidin-1-yl-(9-(phenylethynyl)-5,6-dihydro-2*H*-[1,4]oxazino[2,3-*f*]quinolin-4(3*H*,4a*H*,10b*H*)-yl)methanone,
(*rac*)-3'-((3-Fluorophenyl)ethynyl)-3-propyl-7',8'-dihydro-6'*H*-spiro[oxazolidine-5,5'-quinolin]-2-one,
(*rac*)-3'-((3-Fluorophenyl)ethynyl)-3-phenyl-7',8'-dihydro-6'*H*-spiro[oxazolidine-5,5'-quinolin]-2-one,
3-(*m*-Tolylethynyl)-7,8-dihydro-5*H*-thiopyrano[4,3-*b*]pyridine 6,6-dioxide,
3-((3-Fluorophenyl)ethynyl)-7,8-dihydro-5*H*-thiopyrano[4,3-*b*]pyridine 6,6-dioxide,
3-((4-Fluorophenyl)ethynyl)-7,8-dihydro-5*H*-thiopyrano[4,3-*b*]pyridine 6,6-dioxide,
(6a*R*, 9a*R*)-Methyl 3-(phenylethynyl)-6,6a,9,9a-tetrahydro-5*H*-pyrrolo[2,3-*h*]quinoline-7(8*H*)-carboxylate,
(6a*R*,9a*R*)-Methyl 3-((3-fluorophenyl)ethynyl)-6,6a,9,9a-tetrahydro-5*H*-pyrrolo[2,3-*h*]quinoline-7(8*H*)-carboxylate,
(*rac*)-*cis*-Methyl 3-((3-fluorophenyl)ethynyl)-5a,6,8,8a-tetrahydropyrrolo[3',4':4,5]cyclopenta[1,2-*b*]pyridine-7(5*H*)-carboxylate, (*rac*)-3-Cyclopropyl-3'-(phenylethynyl)-7',8'-dihydro-6'*H*-spiro[oxazolidine-5,5'-quinolin]-2-one,
5,5-Dimethyl-3-(phenylethynyl)-7,8-dihydro-5*H*-thiopyrano[4,3-*b*]pyridine-6,6-dioxide,
(*rac*)-3-Cyclopropyl-3'-((3-fluorophenyl)ethynyl)-7',8'-dihydro-6'*H*-spiro[oxazolidine-5,5'-quinolin]-2-one,
Ethyl 2-oxo-2-(3-(phenylethynyl)-7,8-dihydro-1,6-naphthyridin-6(5*H*)-yl)acetate, 6-((4-Methylpiperazin-1-yl)sulfonyl)-3-(phenylethynyl)-5,6,7,8-tetrahydro-1,6-naphthyridine,
(*rac*)-Methyl 3-(phenylethynyl)-5a,6,8,8a-tetrahydropyrrolo[3',4':4,5]cyclopenta[1,2-*b*]pyridine-7(5H)-carboxylate,
(*rac*)-cis-Methyl 7-(phenylethynyl)-2,3,3a,4,5,9b-hexahydro-1*H*-pyrrolo[3,2-*h*]quinoline-1-carboxylate,
6-(Isopropylsulfonyl)-3-(phenylethynyl)-5,6,7,8-tetrahydro-1,6-naphthyridine, (*rac*)-3'-(Phenylethynyl)-3-propyl-7',8'-dihydro-6'*H*-spiro[oxazolidine-5,5'-quinolin]-2-one
and optical isomers, prodrugs, pharmaceutically acceptable salts, hydrates, solvates, and polymorphs thereof.

13. A pharmaceutical composition comprising a compound as claimed in any preceding claim, together with one or more pharmaceutically acceptable excipients.

14. A compound as claimed in any of Claims 1 to 12, for use in the prevention and/or treatment of a condition or disease selected from Alzheimer's disease, Creutzfeld-Jakob's syndrome/disease, bovine spongiform encephalopathy (BSE), prion related infections, diseases involving mitochondrial dysfunction, diseases involving
β-amyloid and/or tauopathy, Down's syndrome, hepatic encephalopathy, Huntington's disease, motor neuron diseases, amyotrophic lateral sclerosis (ALS), multiple system atrophy, olivoponto-cerebellar atrophy, synucleinopathies, alpha-synucleinopathies, Lewy body dementia, neurodegeneration with Brain Iron Accumulation, Parkinson-plus syndrome, Pick's disease, progressive supranuclear palsy (PSP), frontotemporal dementia and parkinsonism linked to chromosome 17 (FTDP-17), neurodegenerative diseases, post-operative cognitive deficit (POCD), systemic lupus erythematosus, systemic sclerosis, Sjogren's syndrome, Neuronal Ceroid Lipofuscinosis, neurodegenerative cerebellar ataxias, Parkinson's disease, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, eye injuries, eye diseases, eye disorders, glaucoma, retinopathy, macular degeneration, head or brain or spinal cord injuries, head or brain or spinal cord trauma, trauma, hypoglycaemia, hypoxia, perinatal hypoxia, ischaemia, ischaemia resulting from cardiac arrest or stroke or bypass operations or transplants, convulsions, epileptic convulsions, epilepsy, temporal lobe epilepsy, myoclonic epilepsy, inner ear insult, inner ear insult in tinnitus, tinnitus, sound- or drug-induced inner ear insult, sound- or drug-induced tinnitus, L-DOPA-induced dykinesias, L-DOPA-induced dykinesias in Parkinson's disease therapy, dyskinesias, dyskinesia in Huntington's disease, drug induced dyskinesias, neuroleptic-induced dyskinesias, haloperidol-induced dyskinesias, dopaminomimetic-induced dyskinesias, chorea, Huntington's chorea, athetosis, dystonia, stereotypy, ballism, tardive dyskinesias, tic disorder, torticollis spasmodicus, blepharospasm, focal and generalized dystonia, nystagmus, hereditary cerebellar ataxias, corticobasal degeneration, tremor, essential tremor, abuse, addiction, nicotine addiction, nicotine abuse, alcohol addiction, alcohol abuse, opiate addiction, opiate abuse, cocaine addiction, cocaine abuse, amphetamine addiction, amphetamine abuse, anxiety disorders, panic disorders, anxiety and panic disorders, social anxiety disorder (SAD), attention deficit hyperactivity disorder (ADHD), attention deficit syndrome (ADS), restless leg syndrome (RLS), hyperactivity in children, autism, dementia, dementia in Alzheimer's disease, dementia in Korsakoff syndrome, Korsakoff syndrome, vascular dementia, dementia related to HIV infections, HIV-1 encephalopathy, AIDS encephalopathy, AIDS dementia complex, AIDS-related dementia, major depressive disorder, major depression, depression, depression resulting from Borna virus infection, major depression resulting from Borna virus infection, bipolar manic-depressive disorder, drug tolerance, drug tolerance to opioids, movement disorders, fragile-X syndrome, irritable bowel syndrome (IBS), migraine, multiple sclerosis (MS), muscle spasms, pain, chronic pain, acute pain, inflammatory pain, neuropathic pain, diabetic neuropathic pain (DNP), pain related to rheumatic arthritis, allodynia, hyperalgesia, nociceptive pain, cancer pain, posttraumatic stress disorder (PTSD), schizophrenia, positive or negative symptoms of schizophrenia, cognitive deficits of schizophrenia, spasticity, Tourette's syndrome, urinary incontinence, vomiting, pruritic conditions, pruritis, sleep disorders, micturition disorders, neuromuscular disorder in the lower urinary tract, gastroesophageal reflux disease (GERD), gastrointestinal dysfunction, lower esophageal sphincter (LES) disease, functional gastrointestinal disorders, dyspepsia, regurgitation, respiratory tract infection, bulimia nervosa, chronic laryngitis, asthma, reflux-related asthma, lung disease, eating disorders, obesity, obesity-related disorders, obesity abuse, food addiction, binge eating disorders, agoraphobia, generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, or delirium; inhibition of tumour cell growth, migration, invasion, adhesion and toxicity in the peripheral tissues, peripheral nervous system and CNS; neoplasia, hyperplasia, dysplasia, cancer, carcinoma, sarcoma, oral cancer, squamous cell carcinoma (SCC), oral squamous cell carcinoma (SCC), lung cancer, lung adenocarcinoma, breast cancer, prostate cancer, gastric cancer, liver cancer, colon cancer, colorectal carcinoma, rhabdomyosarcoma, brain tumour, tumour of a nerve tissue, glioma, malignant glioma, astroglioma, neuroglioma, neuroblastoma, glioblastoma, medulloblastoma, cancer of skin cells, melanoma, malignant melanoma, epithelial neoplasm, lymphoma, myeloma, Hodgkin's disease, Burkitt's lymphoma, leukemia, thymoma, tumours, diabetes, hyperammonemia and liver failure and sleep disturbances.

15. A pharmaceutical composition comprising a combination of at least one compound as claimed in any of Claims 1 to 12 and at least one NMDA receptor antagonist, together with one or more pharmaceutically acceptable excipients.
